# EUROPEAN PATENT SPECIFICATION

(11) **EP 2 356 899 B1**
(45) Date of publication and mention of the grant of the patent: **13.11.2013**
(21) Application number: 09802980.4
(22) Date of filing: 29.07.2009
(51) Int. Cl.: A01H 1/00, A01H 5/00, C12N 15/09, C12N 15/82, C07K 14/415

(54) **PLANT HAVING RESISTANCE TO MULTIPLE DISEASES AND METHOD FOR PRODUCTION THEREOF**
PFLANZE MIT RESISTENZ GEGEN MEHRERE KRANKHEITEN UND VERFAHREN ZU IHRER HERSTÉLLUNG
PLANTE AYANT UNE RÉSISTANCE À DE MULTIPLES MALADIES ET PROCÉDÉ POUR SA PRODUCTION

(30) Priority: 31.07.2008 JP 2008198517
(43) Date of publication of application: 17.08.2011
(73) Proprietor: RIKEN, Wako-shi, Saitama 351-0198 (JP)
(72) Inventor: NARUSAKA Yoshihiro, Kaga-gun Okayama 709-2344 (JP); NARUSAKA Mari, Kaga-gun Okayama 709-2344 (JP); SHIRASU Ken, Yokohama-shi Kanagawa 230-0045 (JP)
(74) Representative: Cohausz & Florack
(86) International application number: PCT/JP2009/063474
(87) International publication number: WO 2010/013738

(56) References cited:
- EP-A1- 1 950 304
- WO-A1-99/06564
- WO-A2-2006/047495
- JP-A- 2008 237 184
- DATTA K ET AL: "Pyramiding transgenes for multiple resistance in rice against bacterial blight, yellow stem borer and sheath blight", THEORETICAL AND APPLIED GENETICS, SPRINGER, BERLIN, DE, vol. 106, no. 1, 1 December 2002 (2002-12-01), pages 1-8, XP002344664, ISSN: 0040-5752
- BIRKER DORIS ET AL: "A locus conferring resistance to Colletotrichum higginsianum is shared by four geographically distinct Arabidopsis accessions", PLANT JOURNAL, vol. 60, no. 4, November 2009 (2009-11), pages 602-613, XP002672133, ISSN: 0960-7412
- NARUSAKA MARI ET AL: "RRS1 and RPS4 provide a dual Resistance-gene system against fungal and bacterial pathogens", PLANT JOURNAL, vol. 60, no. 2, October 2009 (2009-10), pages 218-226, XP002672134, ISSN: 0960-7412
- MARI NARUSAKA ET AL.: 'Aburana-ka Yasairui Tanso Byokin ni Taisuru Shiroinunazuna Teikosei Idenshi no Dotei' PROCEEDINGS OF THE ANNUAL MEETING OF THE JAPANESE SOCIETY OF PLANT PHYSIOLOGISTS vol. 49, 15 March 2008, page 340, XP008141879
- ZHANG, X.C. ET AL.: 'RPS4-mediated disease resistance requires the combined presence of RPS4 transcripts with full-length and truncated open reading frames' PLANT CELL vol. 15, no. 10, 2003, pages 2333 - 2342, XP008141856
- ZHANG, X. ET AL.: 'Alternative Splicing and mRNA Levels of the Disease Resistance Gene RPS4 Are Induced during Defense Responses' PLANT PHYSIOL vol. 145, no. 4, 2007, pages 1577 - 1587, XP008141857
- NOUTOSHI, Y. ET AL.: 'A single amino acid insertion in the WRKY domain of the Arabidopsis TIR-NBS-LRR-WRKY-type disease resistance protein SLH1 (sensitive to low humidity 1) causes activation of defense responses and hypersensitive cell death' PLANT J vol. 43, no. 6, 2005, pages 873 - 888, XP008141859
- YOSHIHIRO NARUSAKA ET AL.: 'Shokubutsu -Byogen Biseibutsu Sogo Sayo ni Okeru Transcriptome Kaiseki' NIPPON SHOKUBUTSU BYORI GAKKAI SHOKUBUTSU KANSEN SEIRI DANWAKAI RONBUNSHU vol. 40, 2004, pages 83 - 90, XP008141880

### SEQUENCE LISTING

<110> Okayama Prefecture
<110> RIKEN
<120> Resistant plant to various pathogens and methods for the creation
<130> IBPF09-510WO
<150> JP 2008-198517
   <151> 2008-7-31
<160> 12
<170> PatentIn version 3.1
<210> 1
   <211> 3654
   <212> DNA
   <213> Arabidopsis thaliana Ws-0
<220>
   <221> CDS
   <222> (1)..(3651)
   <223>
<400> 1
   atg gag aca tca tct att tcc act gtg gaa gac aag cca ccg cag cat       48
   Met Glu Thr Ser Ser Ile Ser Thr Val Glu Asp Lys Pro Pro Gln His
   1               5                   10                  15
   cag gtg ttc atc aat ttc cgt ggg gca gat ttg cgc cgg aga ttc gtc       96
   Gln Val Phe Ile Asn Phe Arg Gly Ala Asp Leu Arg Arg Arg Phe Val
               20                  25                  30
   agc cat ctc gta acg gcc ttg aaa ttg aac aac atc aac gtc ttt atc      144
   Ser His Leu Val Thr Ala Leu Lys Leu Asn Asn Ile Asn Val Phe Ile
           35                  40                  45
   gac gac tat gaa gac aga ggt caa cct cta gat gta ctg ctg aag agg      192
   Asp Asp Tyr Glu Asp Arg Gly Gln Pro Leu Asp Val Leu Leu Lys Arg
       50                  55                  60
   ata gag gag tcc aaa atc gtt ttg gct atc ttc tcc ggc aac tac acc      240
   Ile Glu Glu Ser Lys Ile Val Leu Ala Ile Phe Ser Gly Asn Tyr Thr
   65                  70                  75                  80
   gag tca gtc tgg tgc gtg aga gag ctc gag aag atc aag gat tgt acg      288
   Glu Ser Val Trp Cys Val Arg Glu Leu Glu Lys Ile Lys Asp Cys Thr
                   85                  90                  95
   gat gaa gga aca ctc gtt gcg att cca atc ttc tac aag ctg gag cca      336
   Asp Glu Gly Thr Leu Val Ala Ile Pro Ile Phe Tyr Lys Leu Glu Pro
               100                 105                 110
   tcc acc gtt aga gat ttg aaa gga aag ttc ggt gat aga ttt agg agt      384
   Ser Thr Val Arg Asp Leu Lys Gly Lys Phe Gly Asp Arg Phe Arg Ser
           115                 120                 125
   atg gct aag ggt gat gag agg aaa aaa aaa tgg aag gaa gct ttt aac      432
   Met Ala Lys Gly Asp Glu Arg Lys Lys Lys Trp Lys Glu Ala Phe Asn
       130                 135                 140
   ttg att cct aac att atg ggc atc acc att gac aag aaa agt gtg gag      480
   Leu Ile Pro Asn Ile Met Gly Ile Thr Ile Asp Lys Lys Ser Val Glu
   145                 150                 155                 160
   agt gag aaa gtc aat gaa att gtg aag gcg gta aag aca gcg ttg acc      528
   Ser Glu Lys Val Asn Glu Ile Val Lys Ala Val Lys Thr Ala Leu Thr
                   165                 170                 175
   gga ata cca ccg gag gga agt cac aat gcc gtc gtg ggt gct tta ggt      576
   Gly Ile Pro Pro Glu Gly Ser His Asn Ala Val Val Gly Ala Leu Gly
               180                 185                 190
   aat agc aac gct gga act tcc tca gga gac aaa aag cat gag act ttt      624
   Asn Ser Asn Ala Gly Thr Ser Ser Gly Asp Lys Lys His Glu Thr Phe
           195                 200                 205
   gga aac gaa caa cgg tta aaa gat ttg gaa gag aag ttg gat cgt gat      672
   Gly Asn Glu Gln Arg Leu Lys Asp Leu Glu Glu Lys Leu Asp Arg Asp
       210                 215                 220
   aaa tac aag gga act cgt atc att gga gtt gtt ggg atg ccc gga att      720
   Lys Tyr Lys Gly Thr Arg Ile Ile Gly Val Val Gly Met Pro Gly Ile
   225                 230                 235                 240
   ggt aaa acc aca ctc ctg aaa gag cta tat aag acg tgg cag ggc aag      768
   Gly Lys Thr Thr Leu Leu Lys Glu Leu Tyr Lys Thr Trp Gln Gly Lys
                   245                 250                 255
   ttt tcg agg cat gcg cta atc gat caa atc cgt gta aag tcc aag cac      816
   Phe Ser Arg His Ala Leu Ile Asp Gln Ile Arg Val Lys Ser Lys His
               260                 265                 270
   ttg gag ttg gat cgc ttg cct caa atg ctc tta ggc gag ttg tcg aag      864
   Leu Glu Leu Asp Arg Leu Pro Gln Met Leu Leu Gly Glu Leu Ser Lys
           275                 280                 285
   tta aac aat cct cac gta gac aat ctc aaa gat cca tac agt caa ctg      912
   Leu Asn Asn Pro His Val Asp Asn Leu Lys Asp Pro Tyr Ser Gln Leu
       290                 295                 300
   cat gaa cgc aaa gtg ctt gtt gta ctt gat gat gtt agt aaa agg gaa      960
   His Glu Arg Lys Val Leu Val Val Leu Asp Asp Val Ser Lys Arg Glu
   305                 310                 315                 320
   caa ata gat gct ctt cgt gag ata cta gac tgg att aag gag ggt aag     1008
   Gln Ile Asp Ala Leu Arg Glu Ile Leu Asp Trp Ile Lys Glu Gly Lys
                   325                 330                 335
   gag gga agc agg gtt gtc att gca aca agc gac atg tcc tta aca aat     1056
   Glu Gly Ser Arg Val Val Ile Ala Thr Ser Asp Met Ser Leu Thr Asn
               340                 345                 350
   ggt ttg gtt gat gat act tac atg gtt caa aat ttg aac cac aga gat     1104
   Gly Leu Val Asp Asp Thr Tyr Met Val Gln Asn Leu Asn His Arg Asp
           355                 360                 365
   agc tta caa cta ttt cac tat cat gcc ttt att gat gat caa gcc aat     1152
   Ser Leu Gln Leu Phe His Tyr His Ala Phe Ile Asp Asp Gln Ala Asn
       370                 375                 380
   cct caa aag aaa gat ttc atg aag ctg tca gaa ggg ttt gta cat tac     1200
   Pro Gln Lys Lys Asp Phe Met Lys Leu Ser Glu Gly Phe Val His Tyr
   385                 390                 395                 400
   gcc aga ggc cat cca cta gcc ctc aaa gta ttg ggt gga gag ctt aat      1248
   Ala Arg Gly His Pro Leu Ala Leu Lys Val Leu Gly Gly Glu Leu Asn
                   405                 410                 415
   aag aaa agt atg gat cat tgg aat tcg aaa atg aag aaa ctt gca cag      1296
   Lys Lys Ser Met Asp His Trp Asn Ser Lys Met Lys Lys Leu Ala Gln
               420                 425                 430
   agc ccc agc cct aat att gtc agt gtc ttc caa gtg agt tat gat gaa      1344
   Ser Pro Ser Pro Asn Ile Val Ser Val Phe Gln Val Ser Tyr Asp Glu
           435                 440                 445
   ttg act aca gcg cag aaa gat gca ttt ctc gac ata gcc tgt ttc aga      1392
   Leu Thr Thr Ala Gln Lys Asp Ala Phe Leu Asp Ile Ala Cys Phe Arg
       450                 455                 460
   tca cag gac aag gat tat gta gaa agt cta ctg gct tca tct gac ctt      1440
   Ser Gln Asp Lys Asp Tyr Val Glu Ser Leu Leu Ala Ser Ser Asp Leu
   465                 470                 475                 480
   ggt tcg gct gaa gca atg agt gca gtc aaa tct ctc acg gat aag ttc      1488
   Gly Ser Ala Glu Ala Met Ser Ala Val Lys Ser Leu Thr Asp Lys Phe
                   485                 490                 495
   ctg att aat act tgt gat ggc cga gtg gag atg cat gat cta tta tat      1536
   Leu Ile Asn Thr Cys Asp Gly Arg Val Glu Met His Asp Leu Leu Tyr
               500                 505                 510
   aaa ttt tca agg gaa att gat ctg aag gca tct aat cag gat ggt agc      1584
   Lys Phe Ser Arg Glu Ile Asp Leu Lys Ala Ser Asn Gln Asp Gly Ser
           515                 520                 525
   aga caa cgg agg ctg tgg ctc cat caa cac ata atc aag gga ggc ata      1632
   Arg Gln Arg Arg Leu Trp Leu His Gln His Ile Ile Lys Gly Gly Ile
       530                 535                 540
   atc aat gta ttg caa aat aaa atg aaa gct gcc aat gtt aga ggt att      1680
   Ile Asn Val Leu Gln Asn Lys Met Lys Ala Ala Asn Val Arg Gly Ile
   545                 550                 555                 560
   ttc cta gac ttg tct gaa gtg gaa gac gaa acg agc tta gac cgc gac      1728
   Phe Leu Asp Leu Ser Glu Val Glu Asp Glu Thr Ser Leu Asp Arg Asp
                   565                 570                 575
   cac ttc ata aat atg gga aat ctc cgg tat ctc aag ttc tac aat tcc      1776
   His Phe Ile Asn Met Gly Asn Leu Arg Tyr Leu Lys Phe Tyr Asn Ser
               580                 585                 590
   cat tgt cca cag gaa tgt aaa acc aac aat aaa atc aac atc cct gat      1824
   His Cys Pro Gln Glu Cys Lys Thr Asn Asn Lys Ile Asn Ile Pro Asp
           595                 600                 605
   aaa ctt aag cta cca ttg aaa gaa gtt cga tgc ctc cac tgg ctg aaa      1872
   Lys Leu Lys Leu Pro Leu Lys Glu Val Arg Cys Leu His Trp Leu Lys
       610                 615                 620
   ttc cca ttg gag aca ctt cca aac gat ttc aac cca att aat ctt gtc      1920
   Phe Pro Leu Glu Thr Leu Pro Asn Asp Phe Asn Pro Ile Asn Leu Val
   625                 630                 635                 640
   gac ctt aag ctg ccc tac agt gaa atg gaa caa ctt tgg gag ggt gac      1968
   Asp Leu Lys Leu Pro Tyr Ser Glu Met Glu Gln Leu Trp Glu Gly Asp
                   645                 650                 655
   aag gac aca cca tgc tta agg tgg gtc gat ctc aat cac tca agt aag      2016
   Lys Asp Thr Pro Cys Leu Arg Trp Val Asp Leu Asn His Ser Ser Lys
               660                 665                 670
   ttg tgc agc ttg tca ggg tta tca aaa gct gaa aag ctt caa agg ttg      2064
   Leu Cys Ser Leu Ser Gly Leu Ser Lys Ala Glu Lys Leu Gln Arg Leu
           675                 680                 685
   aac ctt gaa ggt tgc aca aca ctg aaa gcg ttt cca cat gat atg aaa      2112
   Asn Leu Glu Gly Cys Thr Thr Leu Lys Ala Phe Pro His Asp Met Lys
       690                 695                 700
   aaa atg aaa atg ctt gct ttc ttg aat ctg aag ggg tgt aca agt ctc      2160
   Lys Met Lys Met Leu Ala Phe Leu Asn Leu Lys Gly Cys Thr Ser Leu
   705                 710                 715                 720
   gaa tct ctt cca gag atg aat ttg att tct ctg aaa aca ctt act ctc      2208
   Glu Ser Leu Pro Glu Met Asn Leu Ile Ser Leu Lys Thr Leu Thr Leu
                   725                 730                 735
   agc ggc tgc tca act ttt aag gaa ttt ccg ttg att tca gat aat ata      2256
   Ser Gly Cys Ser Thr Phe Lys Glu Phe Pro Leu Ile Ser Asp Asn Ile
               740                 745                 750
   gaa act cta tac tta gat ggc aca gca ata agt cag ctt cct atg aac      2304
   Glu Thr Leu Tyr Leu Asp Gly Thr Ala Ile Ser Gln Leu Pro Met Asn
           755                 760                 765
   atg gag aag ctt cag aga ctt gtt gta ttg aat atg aaa gac tgc aaa      2352
   Met Glu Lys Leu Gln Arg Leu Val Val Leu Asn Met Lys Asp Cys Lys
       770                 775                 780
   atg cta gag gaa att cca ggc cgt gtt ggt gag ctg aaa gct ctt caa      2400
   Met Leu Glu Glu Ile Pro Gly Arg Val Gly Glu Leu Lys Ala Leu Gln
   785                 790                 795                 800
   gaa ctg ata ctc tct gat tgt tta aat ctc aag att ttc cca gaa atc      2448
   Glu Leu Ile Leu Ser Asp Cys Leu Asn Leu Lys Ile Phe Pro Glu Ile
                   805                 810                 815
   gac att agc ttt tta aac att tta ctt ttg gat ggg aca gcc att gaa      2496
   Asp Ile Ser Phe Leu Asn Ile Leu Leu Leu Asp Gly Thr Ala Ile Glu
               820                 825                 830
   gtg atg cca caa tta ccc tca gtg cag tat ttg tgc tta agc aga aat      2544
   Val Met Pro Gln Leu Pro Ser Val Gln Tyr Leu Cys Leu Ser Arg Asn
           835                 840                 845
   gct aag atc agc tgc ctt cct gtt gga atc agt cag ctt tct caa ctt      2592
   Ala Lys Ile Ser Cys Leu Pro Val Gly Ile Ser Gln Leu Ser Gln Leu
       850                 855                 860
   aaa tgg ctg gac ctg aag tat tgt acg agt ctt aca tca gtt cca gag      2640
   Lys Trp Leu Asp Leu Lys Tyr Cys Thr Ser Leu Thr Ser Val Pro Glu
   865                 870                 875                 880
   ttt cca cca aat ctt cag tgc tta gat gca cac ggc tgt agt tcg ctg      2688
   Phe Pro Pro Asn Leu Gln Cys Leu Asp Ala His Gly Cys Ser Ser Leu
                   885                     890                 895
   aag aca gtt tca aag cct ttg gcc cgt atc atg cca act gaa cag aat      2736
   Lys Thr Val Ser Lys Pro Leu Ala Arg Ile Met Pro Thr Glu Gln Asn
               900                     905                 910
   cat tcc aca ttc att ttc acc aac tgt gag aac ctg gaa caa gct gca      2784
   His Ser Thr Phe Ile Phe Thr Asn Cys Glu Asn Leu Glu Gln Ala Ala
           915                     920                 925
   aag gag gaa atc aca tcg tat gct caa agg aaa tgc cag ctg tta tca      2832
   Lys Glu Glu Ile Thr Ser Tyr Ala Gln Arg Lys Cys Gln Leu Leu Ser
       930                     935                 940
   tat gct cgg aaa cgc tac aat ggg ggt ctt gtt tca gaa tct ttg ttc      2880
   Tyr Ala Arg Lys Arg Tyr Asn Gly Gly Leu Val Ser Glu Ser Leu Phe
   945                 950                     955                 960
   agc act tgc ttt cct gga tgt gaa gtg cct tct tgg ttt tgt cat gaa      2928
   Ser Thr Cys Phe Pro Gly Cys Glu Val Pro Ser Trp Phe Cys His Glu
                   965                     970                 975
   aca gtt gga tct gag tta gaa gta aaa ctc ctc cca cat tgg cat gac      2976
   Thr Val Gly Ser Glu Leu Glu Val Lys Leu Leu Pro His Trp His Asp
               980                     985                 990
   aag aag ctt gcc ggg att gct cta  tgt gct gta gtc tca  tgt ctt gac    3024
   Lys Lys Leu Ala Gly Ile Ala Leu  Cys Ala Val Val Ser  Cys Leu Asp
           995                     1000                1005
   ccc caa  gat caa gtc agc cgc  ttg tca gtg acc tgc  acc ttt aaa       3069
   Pro Gln  Asp Gln Val Ser Arg  Leu Ser Val Thr Cys  Thr Phe Lys
       1010                 1015                 1020
   gta aaa  gat gaa gac aag tct  tgg gtc cca tat act  tgt cca gta       3114
   Val Lys  Asp Glu Asp Lys Ser  Trp Val Pro Tyr Thr  Cys Pro Val
       1025                 1030                 1035
   gga agt  tgg acc aga cat gga  ggc ggg aaa gat aag  att gag cta       3159
   Gly Ser  Trp Thr Arg His Gly  Gly Gly Lys Asp Lys  Ile Glu Leu
       1040                 1045                 1050
   gac cat  gtc ttc att gga tac  acc agt tgc cca cat  act ata aaa       3204
   Asp His  Val Phe Ile Gly Tyr  Thr Ser Cys Pro His  Thr Ile Lys
       1055                 1060                 1065
   tgt cat  gaa gaa ggt aac tca  gat gaa tgc aat cct  acc gaa gcc       3249
   Cys His  Glu Glu Gly Asn Ser  Asp Glu Cys Asn Pro  Thr Glu Ala
       1070                 1075                 1080
   tct ctt  aaa ttt act gtg aca  gga ggt aca agt gag  aat gga aaa       3294
   Ser Leu  Lys Phe Thr Val Thr  Gly Gly Thr Ser Glu  Asn Gly Lys
       1085                 1090                 1095
   tac aag  gtg ttg aag tgt ggt  tta agt ttg gtg tat  gct aag gat       3339
   Tyr Lys  Val Leu Lys Cys Gly  Leu Ser Leu Val Tyr  Ala Lys Asp
       1100                 1105                 1110
   aaa gac  aaa aat agt gct ctt  gaa aca aag tac gat  atg ctt att       3384
   Lys Asp  Lys Asn Ser Ala Leu  Glu Thr Lys Tyr Asp  Met Leu Ile
       1115                 1120                 1125
   gga aag  agc ttt caa gaa act  tca gaa ggg gtt gat  gga agg gta       3429
   Gly Lys  Ser Phe Gln Glu Thr  Ser Glu Gly Val Asp  Gly Arg Val
       1130                 1135                 1140
   aag aaa  acg aaa gga aag tac  gtt atg cct gtt gaa  aag aac ttt       3474
   Lys Lys  Thr Lys Gly Lys Tyr  Val Met Pro Val Glu  Lys Asn Phe
       1145                 1150                 1155
   caa gaa  act aca gaa ggg gtt  gat gga aga gta aag  aaa aag aaa       3519
   Gln Glu  Thr Thr Glu Gly Val  Asp Gly Arg Val Lys  Lys Lys Lys
       1160                 1165                 1170
   aaa aca  aga atg gat aat gga  cgg cca aag aag aag  cag aga tca       3564
   Lys Thr  Arg Met Asp Asn Gly  Arg Pro Lys Lys Lys  Gln Arg Ser
       1175                 1180                 1185
   gga aga  gat gat aat caa act  agg atg caa gtg gaa  ctc caa gaa       3609
   Gly Arg  Asp Asp Asn Gln Thr  Arg Met Gln Val Glu  Leu Gln Glu
       1190                 1195                 1200
   gga aat  ata aat tca gtg atc  atg cac acg gtt aag  aat ttc tga       3654
   Gly Asn  Ile Asn Ser Val Ile  Met His Thr Val Lys  Asn Phe
       1205                 1210                 1215
<210> 2
   <211> 1217
   <212> PRT
   <213> Arabidopsis thaliana Ws-0
<400> 2
   Met Glu Thr Ser Ser Ile Ser Thr Val Glu Asp Lys Pro Pro Gln His
   1               5                   10                  15
   Gln Val Phe Ile Asn Phe Arg Gly Ala Asp Leu Arg Arg Arg Phe Val
               20                  25                  30
   Ser His Leu Val Thr Ala Leu Lys Leu Asn Asn Ile Asn Val Phe Ile
           35                  40                  45
   Asp Asp Tyr Glu Asp Arg Gly Gln Pro Leu Asp Val Leu Leu Lys Arg
       50                  55                  60
   Ile Glu Glu Ser Lys Ile Val Leu Ala Ile Phe Ser Gly Asn Tyr Thr
   65                  70                  75                  80
   Glu Ser Val Trp Cys Val Arg Glu Leu Glu Lys Ile Lys Asp Cys Thr
                   85                  90                  95
   Asp Glu Gly Thr Leu Val Ala Ile Pro Ile Phe Tyr Lys Leu Glu Pro
               100                 105                 110
   Ser Thr Val Arg Asp Leu Lys Gly Lys Phe Gly Asp Arg Phe Arg Ser
           115                 120                 125
   Met Ala Lys Gly Asp Glu Arg Lys Lys Lys Trp Lys Glu Ala Phe Asn
       130                 135                 140
   Leu Ile Pro Asn Ile Met Gly Ile Thr Ile Asp Lys Lys Ser Val Glu
   145                 150                 155                 160
   Ser Glu Lys Val Asn Glu Ile Val Lys Ala Val Lys Thr Ala Leu Thr
                   165                 170                 175
   Gly Ile Pro Pro Glu Gly Ser His Asn Ala Val Val Gly Ala Leu Gly
               180                 185                 190
   Asn Ser Asn Ala Gly Thr Ser Ser Gly Asp Lys Lys His Glu Thr Phe
           195                 200                 205
   Gly Asn Glu Gln Arg Leu Lys Asp Leu Glu Glu Lys Leu Asp Arg Asp
       210                 215                 220
   Lys Tyr Lys Gly Thr Arg Ile Ile Gly Val Val Gly Met Pro Gly Ile
   225                 230                 235                 240
   Gly Lys Thr Thr Leu Leu Lys Glu Leu Tyr Lys Thr Trp Gln Gly Lys
                   245                 250                 255
   Phe Ser Arg His Ala Leu Ile Asp Gln Ile Arg Val Lys Ser Lys His
               260                 265                 270
   Leu Glu Leu Asp Arg Leu Pro Gln Met Leu Leu Gly Glu Leu Ser Lys
           275                 280                 285
   Leu Asn Asn Pro His Val Asp Asn Leu Lys Asp Pro Tyr Ser Gln Leu
       290                 295                 300
   His Glu Arg Lys Val Leu Val Val Leu Asp Asp Val Ser Lys Arg Glu
   305                 310                 315                 320
   Gln Ile Asp Ala Leu Arg Glu Ile Leu Asp Trp Ile Lys Glu Gly Lys
                   325                 330                 335
   Glu Gly Ser Arg Val Val Ile Ala Thr Ser Asp Met Ser Leu Thr Asn
               340                 345                 350
   Gly Leu Val Asp Asp Thr Tyr Met Val Gln Asn Leu Asn His Arg Asp
   355 360 365
   Ser Leu Gln Leu Phe His Tyr His Ala Phe Ile Asp Asp Gln Ala Asn
   370 375 380
   Pro Gln Lys Lys Asp Phe Met Lys Leu Ser Glu Gly Phe Val His Tyr
   385 390 395 400
   Ala Arg Gly His Pro Leu Ala Leu Lys Val Leu Gly Gly Glu Leu Asn
   405 410 415
   Lys Lys Ser Met Asp His Trp Asn Ser Lys Met Lys Lys Leu Ala Gln
   420 425 430
   Ser Pro Ser Pro Asn Ile Val Ser Val Phe Gln Val Ser Tyr Asp Glu
   435 440 445
   Leu Thr Thr Ala Gln Lys Asp Ala Phe Leu Asp Ile Ala Cys Phe Arg
   450 455 460
   Ser Gln Asp Lys Asp Tyr Val Glu Ser Leu Leu Ala Ser Ser Asp Leu
   465 470 475 480
   Gly Ser Ala Glu Ala Met Ser Ala Val Lys Ser Leu Thr Asp Lys Phe
   485 490 495
   Leu Ile Asn Thr Cys Asp Gly Arg Val Glu Met His Asp Leu Leu Tyr
   500 505 510
   Lys Phe Ser Arg Glu Ile Asp Leu Lys Ala Ser Asn Gln Asp Gly Ser
   515 520 525
   Arg Gln Arg Arg Leu Trp Leu His Gln His Ile Ile Lys Gly Gly Ile
   530 535 540
   Ile Asn Val Leu Gln Asn Lys Met Lys Ala Ala Asn Val Arg Gly Ile
   545 550 555 560
   Phe Leu Asp Leu Ser Glu Val Glu Asp Glu Thr Ser Leu Asp Arg Asp
   565 570 575
   His Phe Ile Asn Met Gly Asn Leu Arg Tyr Leu Lys Phe Tyr Asn Ser
   580 585 590
   His Cys Pro Gln Glu Cys Lys Thr Asn Asn Lys Ile Asn Ile Pro Asp
   595 600 605
   Lys Leu Lys Leu Pro Leu Lys Glu Val Arg Cys Leu His Trp Leu Lys
   610 615 620
   Phe Pro Leu Glu Thr Leu Pro Asn Asp Phe Asn Pro Ile Asn Leu Val
   625 630 635 640
   Asp Leu Lys Leu Pro Tyr Ser Glu Met Glu Gln Leu Trp Glu Gly Asp
   645 650 655
   Lys Asp Thr Pro Cys Leu Arg Trp Val Asp Leu Asn His Ser Ser Lys
   660 665 670
   Leu Cys Ser Leu Ser Gly Leu Ser Lys Ala Glu Lys Leu Gln Arg Leu
   675 680 685
   Asn Leu Glu Gly Cys Thr Thr Leu Lys Ala Phe Pro His Asp Met Lys
   690 695 700
   Lys Met Lys Met Leu Ala Phe Leu Asn Leu Lys Gly Cys Thr Ser Leu
   705 710 715 720
   Glu Ser Leu Pro Glu Met Asn Leu Ile Ser Leu Lys Thr Leu Thr Leu
   725 730 735
   Ser Gly Cys Ser Thr Phe Lys Glu Phe Pro Leu Ile Ser Asp Asn Ile
   740 745 750
   Glu Thr Leu Tyr Leu Asp Gly Thr Ala Ile Ser Gln Leu Pro Met Asn
   755 760 765
   Met Glu Lys Leu Gln Arg Leu Val Val Leu Asn Met Lys Asp Cys Lys
   770 775 780
   Met Leu Glu Glu Ile Pro Gly Arg Val Gly Glu Leu Lys Ala Leu Gln
   785 790 795 800
   Glu Leu Ile Leu Ser Asp Cys Leu Asn Leu Lys Ile Phe Pro Glu Ile
   805 810 815
   Asp Ile Ser Phe Leu Asn Ile Leu Leu Leu Asp Gly Thr Ala Ile Glu
   820 825 830
   Val Met Pro Gln Leu Pro Ser Val Gln Tyr Leu Cys Leu Ser Arg Asn
   835 840 845
   Ala Lys Ile Ser Cys Leu Pro Val Gly Ile Ser Gln Leu Ser Gln Leu
   850 855 860
   Lys Trp Leu Asp Leu Lys Tyr Cys Thr Ser Leu Thr Ser Val Pro Glu
   865 870 875 880
   Phe Pro Pro Asn Leu Gln Cys Leu Asp Ala His Gly Cys Ser Ser Leu
   885 890 895
   Lys Thr Val Ser Lys Pro Leu Ala Arg Ile Met Pro Thr Glu Gln Asn
   900 905 910
   His Ser Thr Phe Ile Phe Thr Asn Cys Glu Asn Leu Glu Gln Ala Ala
   915 920 925
   Lys Glu Glu Ile Thr Ser Tyr Ala Gln Arg Lys Cys Gln Leu Leu Ser
   930 935 940
   Tyr Ala Arg Lys Arg Tyr Asn Gly Gly Leu Val Ser Glu Ser Leu Phe
   945 950 955 960
   Ser Thr Cys Phe Pro Gly Cys Glu Val Pro Ser Trp Phe Cys His Glu
   965 970 975
   Thr Val Gly Ser Glu Leu Glu Val Lys Leu Leu Pro His Trp His Asp
   980 985 990
   Lys Lys Leu Ala Gly Ile Ala Leu Cys Ala Val Val Ser Cys Leu Asp
   995 1000 1005
   Pro Gln Asp Gln Val Ser Arg Leu Ser Val Thr Cys Thr Phe Lys
   1010 1015 1020
   Val Lys Asp Glu Asp Lys Ser Trp Val Pro Tyr Thr Cys Pro Val
   1025 1030 1035
   Gly Ser Trp Thr Arg His Gly Gly Gly Lys Asp Lys Ile Glu Leu
   1040 1045 1050
   Asp His Val Phe Ile Gly Tyr Thr Ser Cys Pro His Thr Ile Lys
   1055 1060 1065
   Cys His Glu Glu Gly Asn Ser Asp Glu Cys Asn Pro Thr Glu Ala
   1070 1075 1080
   Ser Leu Lys Phe Thr Val Thr Gly Gly Thr Ser Glu Asn Gly Lys
       1085                1090                1095
   Tyr Lys Val Leu Lys Cys Gly Leu Ser Leu Val Tyr Ala Lys Asp
       1100                1105                1110
   Lys Asp Lys Asn Ser Ala Leu Glu Thr Lys Tyr Asp Met Leu Ile
       1115                1120                1125
   Gly Lys Ser Phe Gln Glu Thr Ser Glu Gly Val Asp Gly Arg Val
       1130                1135                1140
   Lys Lys Thr Lys Gly Lys Tyr Val Met Pro Val Glu Lys Asn Phe
       1145                1150                1155
   Gln Glu Thr Thr Glu Gly Val Asp Gly Arg Val Lys Lys Lys Lys
       1160                1165                1170
   Lys Thr Arg Met Asp Asn Gly Arg Pro Lys Lys Lys Gln Arg Ser
       1175                1180                1185
   Gly Arg Asp Asp Asn Gln Thr Arg Met Gln Val Glu Leu Gln Glu
       1190                1195                1200
   Gly Asn Ile Asn Ser Val Ile Met His Thr Val Lys Asn Phe
       1205                1210                1215
<210> 3
   <211> 4122
   <212> DNA
   <213> Arabidopsis thaliana Ws-0
<220>
   <221> CDS
   <222> (1)..(4119)
   <223>
<400> 3
   atg acc aat tgt gaa aag gat gag gaa ttc gtg tgc atc agc tgc gta      48
   Met Thr Asn Cys Glu Lys Asp Glu Glu Phe Val Cys Ile Ser Cys Val
   1               5                   10                  15
   gaa gag gta cgg tac tct ttc gtg agc cac ctc tcc gaa gct ctc cgc      96
   Glu Glu Val Arg Tyr Ser Phe Val Ser His Leu Ser Glu Ala Leu Arg
               20                  25                  30
   cga aaa ggc ata aat aat gtg gtc gta ggt gta gat agc gat gat ctg      144
   Arg Lys Gly Ile Asn Asn Val Val Val Gly Val Asp Ser Asp Asp Leu
           35                  40                  45
   ctt ttc aag gag tct cag gca aag atc gag aaa gct ggg gtt tct gtg      192
   Leu Phe Lys Glu Ser Gln Ala Lys Ile Glu Lys Ala Gly Val Ser Val
       50                  55                  60
   atg gtt tta ccc gga aac tgt gat cct tcc gat gta tgg ctt gac aag      240
   Met Val Leu Pro Gly Asn Cys Asp Pro Ser Asp Val Trp Leu Asp Lys
   65                  70                  75                  80
   ttc gcc aag gtt ctc gag tgc caa agg aac aac aag gac cag gcg gtg      288
   Phe Ala Lys Val Leu Glu Cys Gln Arg Asn Asn Lys Asp Gln Ala Val
                   85                  90                  95
   gtt cca gta ttg tac ggt gac agt cta tta cgg gac caa tgg ctt agc      336
   Val Pro Val Leu Tyr Gly Asp Ser Leu Leu Arg Asp Gln Trp Leu Ser
               100                 105                 110
   gag ctg gat ttc aaa ggc tta tca cga att cac caa tcc agg aag gaa      384
   Glu Leu Asp Phe Lys Gly Leu Ser Arg Ile His Gln Ser Arg Lys Glu
           115                 120                 125
   tgt agt gac tct ata ctt gta gaa gag att gtg aga gat gtg tac gag      432
   Cys Ser Asp Ser Ile Leu Val Glu Glu Ile Val Arg Asp Val Tyr Glu
       130                 135                 140
   acg cac ttt tat gtt gga cga att gga atc tat tcg aag ctg ctg gag      480
   Thr His Phe Tyr Val Gly Arg Ile Gly Ile Tyr Ser Lys Leu Leu Glu
   145                 150                 155                 160
   att gaa aac atg gtt aac aag caa ccg ata ggc atc cgt tgt gtt gga      528
   Ile Glu Asn Met Val Asn Lys Gln Pro Ile Gly Ile Arg Cys Val Gly
                   165                 170                 175
   att tgg ggt atg cct ggc ata gga aag aca aca ctt gct aaa gca gtc      576
   Ile Trp Gly Met Pro Gly Ile Gly Lys Thr Thr Leu Ala Lys Ala Val
               180                 185                 190
   ttt gac caa atg tct agc gcc ttt gat gct tct tgt ttt atc gaa gac      624
   Phe Asp Gln Met Ser Ser Ala Phe Asp Ala Ser Cys Phe Ile Glu Asp
           195                 200                 205
   tat gac aaa tca att cat gag aag ggt ctt tat tgt ttg ctg gag gaa      672
   Tyr Asp Lys Ser Ile His Glu Lys Gly Leu Tyr Cys Leu Leu Glu Glu
       210                 215                 220
   caa ctt ttg ccg ggc aat gat gca acc att atg aaa ctg agc tcg ctc      720
   Gln Leu Leu Pro Gly Asn Asp Ala Thr Ile Met Lys Leu Ser Ser Leu
   225                 230                 235                 240
   aga gac aga ttg aac agt aag aga gtt ctt gtt gtt ctc gat gac gtg      768
   Arg Asp Arg Leu Asn Ser Lys Arg Val Leu Val Val Leu Asp Asp Val
                   245                 250                 255
   cgc aat gct ctg gtt ggg gag tcc ttt ctc gag ggg ttt gac tgg cta      816
   Arg Asn Ala Leu Val Gly Glu Ser Phe Leu Glu Gly Phe Asp Trp Leu
               260                 265                 270
   gga ccc gga agc ctg atc atc ata acc tct aga gat aaa caa gtg ttt      864
   Gly Pro Gly Ser Leu Ile Ile Ile Thr Ser Arg Asp Lys Gln Val Phe
           275                 280                 285
   tgc ctt tgc gga atc aat caa ata tat gag gtc cag ggt tta aat gag      912
   Cys Leu Cys Gly Ile Asn Gln Ile Tyr Glu Val Gln Gly Leu Asn Glu
       290                 295                 300
   aaa gag gct cgt caa ctt ttc ttg ctg tct gcg tct ata aag gag gat      960
   Lys Glu Ala Arg Gln Leu Phe Leu Leu Ser Ala Ser Ile Lys Glu Asp
   305                 310                 315                 320
   atg gga gag cag aat ctc cag gag ttg tca gtg aga gta ata aat tat     1008
   Met Gly Glu Gln Asn Leu Gln Glu Leu Ser Val Arg Val Ile Asn Tyr
                   325                 330                 335
   gct aat gga aac ccg tta gct atc aat gtt tat gga aga gag ctg aaa     1056
   Ala Asn Gly Asn Pro Leu Ala Ile Asn Val Tyr Gly Arg Glu Leu Lys
               340                 345                 350
   ggt aag aaa aaa ctc tca gaa atg gag act gca ttc ctc aaa ctc aag     1104
   Gly Lys Lys Lys Leu Ser Glu Met Glu Thr Ala Phe Leu Lys Leu Lys
           355                 360                 365
   cga cgt cct cca ttt aag att gtc gat gca ttt aaa agc acc tat gac     1152
   Arg Arg Pro Pro Phe Lys Ile Val Asp Ala Phe Lys Ser Thr Tyr Asp
       370                 375                 380
   aca ctc agt gac aac gaa aag aac att ttt ttg gac ata gct tgt ttc     1200
   Thr Leu Ser Asp Asn Glu Lys Asn Ile Phe Leu Asp Ile Ala Cys Phe
   385                 390                 395                 400
   ttc cag gga gaa aat gtc aac tac gtg ata caa ctg ctt gag ggt tgt     1248
   Phe Gln Gly Glu Asn Val Asn Tyr Val Ile Gln Leu Leu Glu Gly Cys
                   405                 410                 415
   ggt ttc ttt cca cat gtt gaa att gat gtc ctt gtt gac aag tgt ctg     1296
   Gly Phe Phe Pro His Val Glu Ile Asp Val Leu Val Asp Lys Cys Leu
               420                 425                 430
   gta act att tca gaa aac cga gtt tgg ttg cat aag ctg acc cag gat     1344
   Val Thr Ile Ser Glu Asn Arg Val Trp Leu His Lys Leu Thr Gln Asp
           435                 440                 445
   atc ggc cga gaa atc ata aat gga gaa aca gta cag atc gag agg cgc     1392
   Ile Gly Arg Glu Ile Ile Asn Gly Glu Thr Val Gln Ile Glu Arg Arg
       450                 455                 460
   aga aga ctg tgg gaa cct tgg agc atc aaa tat tta tta gaa tat aat     1440
   Arg Arg Leu Trp Glu Pro Trp Ser Ile Lys Tyr Leu Leu Glu Tyr Asn
   465                 470                 475                 480
   gaa cac aaa gca aat gga gaa cct aaa aca acc ttc aaa cgt gct cag     1488
   Glu His Lys Ala Asn Gly Glu Pro Lys Thr Thr Phe Lys Arg Ala Gln
                   485                 490                 495
   ggc tct gaa gag atc gaa ggc ctg ttt cta gac aca tca aac tta aga     1536
   Gly Ser Glu Glu Ile Glu Gly Leu Phe Leu Asp Thr Ser Asn Leu Arg
               500                 505                 510
   ttt gat ctg cag ccc tct gcc ttt aag aat atg ttg aac ctt aga ttg     1584
   Phe Asp Leu Gln Pro Ser Ala Phe Lys Asn Met Leu Asn Leu Arg Leu
           515                 520                 525
   ctc aaa att tat tgt tcc aat cct gaa gtc cat cct gta atc aat ttc     1632
   Leu Lys Ile Tyr Cys Ser Asn Pro Glu Val His Pro Val Ile Asn Phe
       530                 535                 540
   cca aca ggc tct ctg cat tct ctt cct aat gag cta aga ctc ctc cat      1680
   Pro Thr Gly Ser Leu His Ser Leu Pro Asn Glu Leu Arg Leu Leu His
   545                 550                 555                 560
   tgg gag aac tat cct ctg aaa tct ttg cct cag aat ttt gat cct agg      1728
   Trp Glu Asn Tyr Pro Leu Lys Ser Leu Pro Gln Asn Phe Asp Pro Arg
                   565                 570                 575
   cac ctt gtc gaa atc aac atg ccg tat agt caa ctt cag aaa ctt tgg      1776
   His Leu Val Glu Ile Asn Met Pro Tyr Ser Gln Leu Gln Lys Leu Trp
               580                 585                 590
   ggt gga acc aag aac ctg gag atg ttg agg acg atc agg ctt tgc cat      1824
   Gly Gly Thr Lys Asn Leu Glu Met Leu Arg Thr Ile Arg Leu Cys His
           595                 600                 605
   tcc cac cat cta gtt gat atc gat gat ctc tta aaa gct gaa aat ctt      1872
   Ser His His Leu Val Asp Ile Asp Asp Leu Leu Lys Ala Glu Asn Leu
       610                 615                 620
   gag gta att gat ctc caa ggt tgt acg aga ctg cag aat ttc cca gcc      1920
   Glu Val Ile Asp Leu Gln Gly Cys Thr Arg Leu Gln Asn Phe Pro Ala
   625                 630                 635                 640
   gca ggt cga ttg cta cgt cta cga gat gta aat ctc tca ggt tgc ata      1968
   Ala Gly Arg Leu Leu Arg Leu Arg Asp Val Asn Leu Ser Gly Cys Ile
                   645                 650                 655
   aag att aaa agt gtc cta gaa att cca cca aat att gag aaa cta cat      2016
   Lys Ile Lys Ser Val Leu Glu Ile Pro Pro Asn Ile Glu Lys Leu His
               660                 665                 670
   cta cag gga act ggc ata tta gca tta cca gtt tcc act gtt aag cca      2064
   Leu Gln Gly Thr Gly Ile Leu Ala Leu Pro Val Ser Thr Val Lys Pro
           675                 680                 685
   aac cat aga gag ctt gtg aat ttt cta aca gaa att ccg ggt ctt tca      2112
   Asn His Arg Glu Leu Val Asn Phe Leu Thr Glu Ile Pro Gly Leu Ser
       690                 695                 700
   gag gcc tcg aaa ctt gag cgt tta aca agt ctg ctg gaa tct aac tca      2160
   Glu Ala Ser Lys Leu Glu Arg Leu Thr Ser Leu Leu Glu Ser Asn Ser
   705                 710                 715                 720
   tct tgt caa gat ctt ggg aag ctt att tgc ttg gag ctg aaa gat tgc      2208
   Ser Cys Gln Asp Leu Gly Lys Leu Ile Cys Leu Glu Leu Lys Asp Cys
                   725                 730                 735
   tct tgt ttg cag agt ctg cca aac atg gct aat tta gat ctt aat gtt      2256
   Ser Cys Leu Gln Ser Leu Pro Asn Met Ala Asn Leu Asp Leu Asn Val
               740                 745                 750
   ctt gat ctc tcg ggt tgc tca agt ctt aat tct att cag ggt ttc cct      2304
   Leu Asp Leu Ser Gly Cys Ser Ser Leu Asn Ser Ile Gln Gly Phe Pro
           755                 760                 765
   cgt ttt ctg aaa cag tta tat ctt ggt ggc act gca ata aga gaa gtg      2352
   Arg Phe Leu Lys Gln Leu Tyr Leu Gly Gly Thr Ala Ile Arg Glu Val
       770                 775                 780
   cca caa ctt cct caa agt cta gaa atc ttg aat gca cat gga tct tgt      2400
   Pro Gln Leu Pro Gln Ser Leu Glu Ile Leu Asn Ala His Gly Ser Cys
   785                 790                 795                 800
   ttg cga agt ctg cca aac atg gct aat tta gaa ttt ctc aaa gtt ctt      2448
   Leu Arg Ser Leu Pro Asn Met Ala Asn Leu Glu Phe Leu Lys Val Leu
                   805                 810                 815
   gat ctc tct ggt tgc tca gag ctc gag act att cag ggt ttt cct cgg      2496
   Asp Leu Ser Gly Cys Ser Glu Leu Glu Thr Ile Gln Gly Phe Pro Arg
               820                 825                 830
   aac cta aaa gag tta tat ttt gct ggc act acg tta aga gaa gtg ccc      2544
   Asn Leu Lys Glu Leu Tyr Phe Ala Gly Thr Thr Leu Arg Glu Val Pro
           835                 840                 845
   caa ctt cct tta agc cta gag gtc ttg aat gca cat ggt tct gac tcg      2592
   Gln Leu Pro Leu Ser Leu Glu Val Leu Asn Ala His Gly Ser Asp Ser
       850                 855                 860
   gag aag ctt cct atg cat tac aag ttc aac aat ttt ttc gat cta tct      2640
   Glu Lys Leu Pro Met His Tyr Lys Phe Asn Asn Phe Phe Asp Leu Ser
   865                 870                 875                 880
   caa caa gtg gtc aac gat ttt ttc ttg aaa acg ctg act tat gta aaa      2688
   Gln Gln Val Val Asn Asp Phe Phe Leu Lys Thr Leu Thr Tyr Val Lys
                   885                 890                 895
   cac ata cca aga ggg tat acg cag gaa ctc atc aac aaa gct ccg act      2736
   His Ile Pro Arg Gly Tyr Thr Gln Glu Leu Ile Asn Lys Ala Pro Thr
               900                 905                 910
   ttc agc ttc agt gcg ccc tca cat aca aat caa aac gcc aca ttt gat      2784
   Phe Ser Phe Ser Ala Pro Ser His Thr Asn Gln Asn Ala Thr Phe Asp
           915                 920                 925
   ctg caa cca gga tct tct gta atg aca cga cta aat cat tca tgg agg      2832
   Leu Gln Pro Gly Ser Ser Val Met Thr Arg Leu Asn His Ser Trp Arg
       930                 935                 940
   aac acg ctt gtg gga ttt ggt atg ctg gtg gaa gtt gca ttt ccc gag      2880
   Asn Thr Leu Val Gly Phe Gly Met Leu Val Glu Val Ala Phe Pro Glu
   945                 950                 955                 960
   gac tac tgt gat gct aca gat ttt ggc ata agt tgt gtt tgc aga tgg      2928
   Asp Tyr Cys Asp Ala Thr Asp Phe Gly Ile Ser Cys Val Cys Arg Trp
                   965                 970                 975
   agc aac aaa gaa ggc cgc tct tgt agg ata gaa aga aat ttt cat tgt      2976
   Ser Asn Lys Glu Gly Arg Ser Cys Arg Ile Glu Arg Asn Phe His Cys
               980                 985                 990
   tgg gca cca ggg aaa gtt gtt cca  aaa gtt cga aag gat  cat acg ttt    3024
   Trp Ala Pro Gly Lys Val Val Pro  Lys Val Arg Lys Asp  His Thr Phe
           995                 1000                1005
   gtc ttt  agt gat gtc aac atg  cgc cca agt acc ggt  gaa gga aat       3069
   Val Phe  Ser Asp Val Asn Met  Arg Pro Ser Thr Gly  Glu Gly Asn
       1010                1015                1020
   gac cct  gat atc tgg gct gga  tta gtt gta ttt gag  ttc ttt cct      3114
   Asp Pro  Asp Ile Trp Ala Gly  Leu Val Val Phe Glu  Phe Phe Pro
       1025                 1030                 1035
   atc aat  cag cag aca aag tgt  cta aat gat agg ttc  aca gtg aca      3159
   Ile Asn  Gln Gln Thr Lys Cys  Leu Asn Asp Arg Phe  Thr Val Thr
       1040                 1045                 1050
   aga tgt  gga gtc cgt gta ata  aat gtt gca act ggc  aat aca agt      3204
   Arg Cys  Gly Val Arg Val Ile  Asn Val Ala Thr Gly  Asn Thr Ser
       1055                 1060                 1065
   ctt gag  aac ata tca cta gtt  ttg tct ttg gat cca  gtg gag gtt      3249
   Leu Glu  Asn Ile Ser Leu Val  Leu Ser Leu Asp Pro  Val Glu Val
       1070                 1075                 1080
   tct ggt  tat gaa gta ttg aga  gtc agc tat gat gat  tta cag gag      3294
   Ser Gly  Tyr Glu Val Leu Arg  Val Ser Tyr Asp Asp  Leu Gln Glu
       1085                 1090                 1095
   atg gat  aaa gtt cta ttt ctt  tac ata gcg tct ttg  ttc aat gac      3339
   Met Asp  Lys Val Leu Phe Leu  Tyr Ile Ala Ser Leu  Phe Asn Asp
       1100                 1105                 1110
   gag gat  gtt gat ttt gtg gca  cca ctt att gcc ggt  att gac ttg      3384
   Glu Asp  Val Asp Phe Val Ala  Pro Leu Ile Ala Gly  Ile Asp Leu
       1115                 1120                 1125
   gat gtt  agc tct ggg ctc aag  gtc tta gcc gat gtg  tct ctc ata      3429
   Asp Val  Ser Ser Gly Leu Lys  Val Leu Ala Asp Val  Ser Leu Ile
       1130                 1135                 1140
   agt gta  tca tcc aat ggg gaa  ata gtg atg cat agt  ttg caa aga      3474
   Ser Val  Ser Ser Asn Gly Glu  Ile Val Met His Ser  Leu Gln Arg
       1145                 1150                 1155
   caa atg  ggc aaa gaa atc ctc  cat gga caa tcc atg  ctg ctg tct      3519
   Gln Met  Gly Lys Glu Ile Leu  His Gly Gln Ser Met  Leu Leu Ser
       1160                 1165                 1170
   gat tgt  gag agt tcc atg acc  gag aat ttg tct gac  gta cca aaa      3564
   Asp Cys  Glu Ser Ser Met Thr  Glu Asn Leu Ser Asp  Val Pro Lys
       1175                 1180                 1185
   aag gag  aag aaa cat cgc gaa  agt aag gta aag aaa  gtg gtt tcc      3609
   Lys Glu  Lys Lys His Arg Glu  Ser Lys Val Lys Lys  Val Val Ser
       1190                 1195                 1200
   ata ccg  gct ata gac gag gga  gat cta tgg act tgg  cga aag tac      3654
   Ile Pro  Ala Ile Asp Glu Gly  Asp Leu Trp Thr Trp  Arg Lys Tyr
       1205                 1210                 1215
   ggt caa  aaa gac atc tta ggt  tct cgt ttt cca agg  ggt tac tac      3699
   Gly Gln  Lys Asp Ile Leu Gly  Ser Arg Phe Pro Arg  Gly Tyr Tyr
       1220                 1225                 1230
   aga tgc  gct tac aag ttc acg  cat ggt tgt aaa gct  aca aaa caa      3744
   Arg Cys  Ala Tyr Lys Phe Thr  His Gly Cys Lys Ala  Thr Lys Gln
       1235                 1240                 1245
   gtc caa  cgg agc gag acc gat  tca aac atg tta gct  att act tac      3789
   Val Gln Arg Ser Glu Thr Asp Ser Asn Met Leu Ala Ile Thr Tyr
       1250                1255                1260
   cta tct gag cat aac cat cca cgg ccc act aaa cgc aag gct ctc      3834
   Leu Ser Glu His Asn His Pro Arg Pro Thr Lys Arg Lys Ala Leu
       1265                1270                1275
   gct gac tcc act cgt tcc act tcc tcc tcc atc tgc tca gcc ata      3879
   Ala Asp Ser Thr Arg Ser Thr Ser Ser Ser Ile Cys Ser Ala Ile
       1280                1285                1290
   act acc tct gcc tca tct aga gtc ttc caa aac aaa gac gaa cca      3924
   Thr Thr Ser Ala Ser Ser Arg Val Phe Gln Asn Lys Asp Glu Pro
       1295                1300                1305
   aat aaa ccc cac ttg cct tcc tcc tcc act cct cct gga aac gcg      3969
   Asn Lys Pro His Leu Pro Ser Ser Ser Thr Pro Pro Gly Asn Ala
       1310                1315                1320
   gct gtc ttg ttt aaa atg acg gac atg gag gag ttt cag gac aat      4014
   Ala Val Leu Phe Lys Met Thr Asp Met Glu Glu Phe Gln Asp Asn
       1325                1330                1335
   atg gag gtg gat aat gac gtc gta gat aca cgt aca ctg gca ttg      4059
   Met Glu Val Asp Asn Asp Val Val Asp Thr Arg Thr Leu Ala Leu
       1340                1345                1350
   ttt cca gag ttt caa cat cag ccg gag gaa gaa tac cct tgg tca      4104
   Phe Pro Glu Phe Gln His Gln Pro Glu Glu Glu Tyr Pro Trp Ser
       1355                1360                1365
   aca ttc ttc gat tat tag                                          4122
   Thr Phe Phe Asp Tyr
       1370
<210> 4
   <211> 1373
   <212> PRT
   <213> Arabidopsis thaliana Ws-0
<400> 4
   Met Thr Asn Cys Glu Lys Asp Glu Glu Phe Val Cys Ile Ser Cys Val
   1               5                   10                  15
   Glu Glu Val Arg Tyr Ser Phe Val Ser His Leu Ser Glu Ala Leu Arg
               20                  25                  30
   Arg Lys Gly Ile Asn Asn Val Val Val Gly Val Asp Ser Asp Asp Leu
           35                  40                  45
   Leu Phe Lys Glu Ser Gln Ala Lys Ile Glu Lys Ala Gly Val Ser Val
       50                  55                  60
   Met Val Leu Pro Gly Asn Cys Asp Pro Ser Asp Val Trp Leu Asp Lys
   65                  70                  75                  80
   Phe Ala Lys Val Leu Glu Cys Gln Arg Asn Asn Lys Asp Gln Ala Val
   85 90 95
   Val Pro Val Leu Tyr Gly Asp Ser Leu Leu Arg Asp Gln Trp Leu Ser
   100 105 110
   Glu Leu Asp Phe Lys Gly Leu Ser Arg Ile His Gln Ser Arg Lys Glu
   115 120 125
   Cys Ser Asp Ser Ile Leu Val Glu Glu Ile Val Arg Asp Val Tyr Glu
   130 135 140
   Thr His Phe Tyr Val Gly Arg Ile Gly Ile Tyr Ser Lys Leu Leu Glu
   145 150 155 160
   Ile Glu Asn Met Val Asn Lys Gln Pro Ile Gly Ile Arg Cys Val Gly
   165 170 175
   Ile Trp Gly Met Pro Gly Ile Gly Lys Thr Thr Leu Ala Lys Ala Val
   180 185 190
   Phe Asp Gln Met Ser Ser Ala Phe Asp Ala Ser Cys Phe Ile Glu Asp
   195 200 205
   Tyr Asp Lys Ser Ile His Glu Lys Gly Leu Tyr Cys Leu Leu Glu Glu
   210 215 220
   Gln Leu Leu Pro Gly Asn Asp Ala Thr Ile Met Lys Leu Ser Ser Leu
   225 230 235 240
   Arg Asp Arg Leu Asn Ser Lys Arg Val Leu Val Val Leu Asp Asp Val
   245 250 255
   Arg Asn Ala Leu Val Gly Glu Ser Phe Leu Glu Gly Phe Asp Trp Leu
   260 265 270
   Gly Pro Gly Ser Leu Ile Ile Ile Thr Ser Arg Asp Lys Gln Val Phe
   275 280 285
   Cys Leu Cys Gly Ile Asn Gln Ile Tyr Glu Val Gln Gly Leu Asn Glu
   290 295 300
   Lys Glu Ala Arg Gln Leu Phe Leu Leu Ser Ala Ser Ile Lys Glu Asp
   305 310 315 320
   Met Gly Glu Gln Asn Leu Gln Glu Leu Ser Val Arg Val Ile Asn Tyr
                   325                 330                 335
   Ala Asn Gly Asn Pro Leu Ala Ile Asn Val Tyr Gly Arg Glu Leu Lys
               340                 345                 350
   Gly Lys Lys Lys Leu Ser Glu Met Glu Thr Ala Phe Leu Lys Leu Lys
           355                 360                 365
   Arg Arg Pro Pro Phe Lys Ile Val Asp Ala Phe Lys Ser Thr Tyr Asp
       370                 375                 380
   Thr Leu Ser Asp Asn Glu Lys Asn Ile Phe Leu Asp Ile Ala Cys Phe
   385                 390                 395                 400
   Phe Gln Gly Glu Asn Val Asn Tyr Val Ile Gln Leu Leu Glu Gly Cys
                   405                 410                 415
   Gly Phe Phe Pro His Val Glu Ile Asp Val Leu Val Asp Lys Cys Leu
               420                 425                 430
   Val Thr Ile Ser Glu Asn Arg Val Trp Leu His Lys Leu Thr Gln Asp
           435                 440                 445
   Ile Gly Arg Glu Ile Ile Asn Gly Glu Thr Val Gln Ile Glu Arg Arg
       450                 455                 460
   Arg Arg Leu Trp Glu Pro Trp Ser Ile Lys Tyr Leu Leu Glu Tyr Asn
   465                 470                 475                 480
   Glu His Lys Ala Asn Gly Glu Pro Lys Thr Thr Phe Lys Arg Ala Gln
                   485                 490                 495
   Gly Ser Glu Glu Ile Glu Gly Leu Phe Leu Asp Thr Ser Asn Leu Arg
               500                 505                 510
   Phe Asp Leu Gln Pro Ser Ala Phe Lys Asn Met Leu Asn Leu Arg Leu
           515                 520                 525
   Leu Lys Ile Tyr Cys Ser Asn Pro Glu Val His Pro Val Ile Asn Phe
       530                 535                 540
   Pro Thr Gly Ser Leu His Ser Leu Pro Asn Glu Leu Arg Leu Leu His
   545                 550                 555                 560
   Trp Glu Asn Tyr Pro Leu Lys Ser Leu Pro Gln Asn Phe Asp Pro Arg
   565 570 575
   His Leu Val Glu Ile Asn Met Pro Tyr Ser Gln Leu Gln Lys Leu Trp
   580 585 590
   Gly Gly Thr Lys Asn Leu Glu Met Leu Arg Thr Ile Arg Leu Cys His
   595 600 605
   Ser His His Leu Val Asp Ile Asp Asp Leu Leu Lys Ala Glu Asn Leu
   610 615 620
   Glu Val Ile Asp Leu Gln Gly Cys Thr Arg Leu Gln Asn Phe Pro Ala
   625 630 635 640
   Ala Gly Arg Leu Leu Arg Leu Arg Asp Val Asn Leu Ser Gly Cys Ile
   645 650 655
   Lys Ile Lys Ser Val Leu Glu Ile Pro Pro Asn Ile Glu Lys Leu His
   660 665 670
   Leu Gln Gly Thr Gly Ile Leu Ala Leu Pro Val Ser Thr Val Lys Pro
   675 680 685
   Asn His Arg Glu Leu Val Asn Phe Leu Thr Glu Ile Pro Gly Leu Ser
   690 695 700
   Glu Ala Ser Lys Leu Glu Arg Leu Thr Ser Leu Leu Glu Ser Asn Ser
   705 710 715 720
   Ser Cys Gln Asp Leu Gly Lys Leu Ile Cys Leu Glu Leu Lys Asp Cys
   725 730 735
   Ser Cys Leu Gln Ser Leu Pro Asn Met Ala Asn Leu Asp Leu Asn Val
   740 745 750
   Leu Asp Leu Ser Gly Cys Ser Ser Leu Asn Ser Ile Gln Gly Phe Pro
   755 760 765
   Arg Phe Leu Lys Gln Leu Tyr Leu Gly Gly Thr Ala Ile Arg Glu Val
   770 775 780
   Pro Gln Leu Pro Gln Ser Leu Glu Ile Leu Asn Ala His Gly Ser Cys
   785 790 795 800
   Leu Arg Ser Leu Pro Asn Met Ala Asn Leu Glu Phe Leu Lys Val Leu
   805 810 815
   Asp Leu Ser Gly Cys Ser Glu Leu Glu Thr Ile Gln Gly Phe Pro Arg
   820 825 830
   Asn Leu Lys Glu Leu Tyr Phe Ala Gly Thr Thr Leu Arg Glu Val Pro
   835 840 845
   Gln Leu Pro Leu Ser Leu Glu Val Leu Asn Ala His Gly Ser Asp Ser
   850 855 860
   Glu Lys Leu Pro Met His Tyr Lys Phe Asn Asn Phe Phe Asp Leu Ser
   865 870 875 880
   Gln Gln Val Val Asn Asp Phe Phe Leu Lys Thr Leu Thr Tyr Val Lys
   885 890 895
   His Ile Pro Arg Gly Tyr Thr Gln Glu Leu Ile Asn Lys Ala Pro Thr
   900 905 910
   Phe Ser Phe Ser Ala Pro Ser His Thr Asn Gln Asn Ala Thr Phe Asp
   915 920 925
   Leu Gln Pro Gly Ser Ser Val Met Thr Arg Leu Asn His Ser Trp Arg
   930 935 940
   Asn Thr Leu Val Gly Phe Gly Met Leu Val Glu Val Ala Phe Pro Glu
   945 950 955 960
   Asp Tyr Cys Asp Ala Thr Asp Phe Gly Ile Ser Cys Val Cys Arg Trp
   965 970 975
   Ser Asn Lys Glu Gly Arg Ser Cys Arg Ile Glu Arg Asn Phe His Cys
   980 985 990
   Trp Ala Pro Gly Lys Val Val Pro Lys Val Arg Lys Asp His Thr Phe
   995 1000 1005
   Val Phe Ser Asp Val Asn Met Arg Pro Ser Thr Gly Glu Gly Asn
   1010 1015 1020
   Asp Pro Asp Ile Trp Ala Gly Leu Val Val Phe Glu Phe Phe Pro
   1025 1030 1035
   Ile Asn Gln Gln Thr Lys Cys Leu Asn Asp Arg Phe Thr Val Thr
   1040 1045 1050
   Arg Cys Gly Val Arg Val Ile Asn Val Ala Thr Gly Asn Thr Ser
       1055                1060                1065
   Leu Glu Asn Ile Ser Leu Val Leu Ser Leu Asp Pro Val Glu Val
       1070                1075                1080
   Ser Gly Tyr Glu Val Leu Arg Val Ser Tyr Asp Asp Leu Gln Glu
       1085                1090                1095
   Met Asp Lys Val Leu Phe Leu Tyr Ile Ala Ser Leu Phe Asn Asp
       1100                1105                1110
   Glu Asp Val Asp Phe Val Ala Pro Leu Ile Ala Gly Ile Asp Leu
       1115                1120                1125
   Asp Val Ser Ser Gly Leu Lys Val Leu Ala Asp Val Ser Leu Ile
       1130                1135                1140
   Ser Val Ser Ser Asn Gly Glu Ile Val Met His Ser Leu Gln Arg
       1145                1150                1155
   Gln Met Gly Lys Glu Ile Leu His Gly Gln Ser Met Leu Leu Ser
       1160                1165                1170
   Asp Cys Glu Ser Ser Met Thr Glu Asn Leu Ser Asp Val Pro Lys
       1175                1180                1185
   Lys Glu Lys Lys His Arg Glu Ser Lys Val Lys Lys Val Val Ser
       1190                1195                1200
   Ile Pro Ala Ile Asp Glu Gly Asp Leu Trp Thr Trp Arg Lys Tyr
       1205                1210                1215
   Gly Gln Lys Asp Ile Leu Gly Ser Arg Phe Pro Arg Gly Tyr Tyr
       1220                1225                1230
   Arg Cys Ala Tyr Lys Phe Thr His Gly Cys Lys Ala Thr Lys Gln
       1235                1240                1245
   Val Gln Arg Ser Glu Thr Asp Ser Asn Met Leu Ala Ile Thr Tyr
       1250                1255                1260
   Leu Ser Glu His Asn His Pro Arg Pro Thr Lys Arg Lys Ala Leu
       1265                1270                1275
   Ala Asp Ser Thr Arg Ser Thr Ser Ser Ser Ile Cys Ser Ala Ile
   1280 1285 1290
   Thr Thr Ser Ala Ser Ser Arg Val Phe Gln Asn Lys Asp Glu Pro
   1295 1300 1305
   Asn Lys Pro His Leu Pro Ser Ser Ser Thr Pro Pro Gly Asn Ala
   1310 1315 1320
   Ala Val Leu Phe Lys Met Thr Asp Met Glu Glu Phe Gln Asp Asn
   1325 1330 1335
   Met Glu Val Asp Asn Asp Val Val Asp Thr Arg Thr Leu Ala Leu
   1340 1345 1350
   Phe Pro Glu Phe Gln His Gln Pro Glu Glu Glu Tyr Pro Trp Ser
   1355 1360 1365
   Thr Phe Phe Asp Tyr
   1370
<210> 5
   <211> 3654
   <212> DNA
   <213> Arabidopsis thaliana No-0
<220>
   <221> CDS
   <222> (1)..(3651)
   <223>
<400> 5
   atg gag aca tcg tct att tcc act gtg gaa gac aag cca ccg cag cat        48
   Met Glu Thr Ser Ser Ile Ser Thr Val Glu Asp Lys Pro Pro Gln His
   1               5                   10                  15
   cag gtg ttc atc aat ttc cgt ggg gca gat ttg cgc cgg aga ttc gtc        96
   Gln Val Phe Ile Asn Phe Arg Gly Ala Asp Leu Arg Arg Arg Phe Val
               20                  25                  30
   agc cat ctc gtc acg gcc ttg aaa ttg aac aac atc aac gtc ttt atc       144
   Ser His Leu Val Thr Ala Leu Lys Leu Asn Asn Ile Asn Val Phe Ile
           35                  40                  45
   gac gac tat gaa gac aga ggt caa cct cta gat gta ctg ctg aag agg       192
   Asp Asp Tyr Glu Asp Arg Gly Gln Pro Leu Asp Val Leu Leu Lys Arg
       50                  55                  60
   ata gag gag tcc aaa atc gtt ttg gct atc ttc tcc ggc aac tac acc       240
   Ile Glu Glu Ser Lys Ile Val Leu Ala Ile Phe Ser Gly Asn Tyr Thr
   65                  70                  75                  80
   gag tca gtc tgg tgc gtg aga gag ctg gag aag atc aaa gat tgt acg       288
   Glu Ser Val Trp Cys Val Arg Glu Leu Glu Lys Ile Lys Asp Cys Thr
                   85                  90                  95
   gat gaa gga aca ctc gtt gcg att cca atc ttc tac aag ctg gag cca      336
   Asp Glu Gly Thr Leu Val Ala Ile Pro Ile Phe Tyr Lys Leu Glu Pro
               100                 105                 110
   tcc acc gtt aga gat ttg aaa gga aag ttc ggt gat aga ttt agg agt      384
   Ser Thr Val Arg Asp Leu Lys Gly Lys Phe Gly Asp Arg Phe Arg Ser
           115                 120                 125
   atg gct aag ggt gat gag agg aaa aaa aaa tgg aag gaa gct ttt aac      432
   Met Ala Lys Gly Asp Glu Arg Lys Lys Lys Trp Lys Glu Ala Phe Asn
       130                 135                 140
   ttg att cct aac att atg ggc atc acc att gac aag aaa agt gtg gag      480
   Leu Ile Pro Asn Ile Met Gly Ile Thr Ile Asp Lys Lys Ser Val Glu
   145                 150                 155                 160
   agt gag aaa gtc aat gaa att gtg aag gcc gtg aag aca gcg ttg acc      528
   Ser Glu Lys Val Asn Glu Ile Val Lys Ala Val Lys Thr Ala Leu Thr
                   165                 170                 175
   gga ata cca ccg gag gga agt cac aat gcc gtc gtg ggt gct tta ggt      576
   Gly Ile Pro Pro Glu Gly Ser His Asn Ala Val Val Gly Ala Leu Gly
               180                 185                 190
   aat agc aac gct gga act tcc tca gga gac aaa aag cat gag act ttt      624
   Asn Ser Asn Ala Gly Thr Ser Ser Gly Asp Lys Lys His Glu Thr Phe
           195                 200                 205
   gga aac gaa caa cgg tta aaa gat ttg gaa gag aag ttg gat cgt gat      672
   Gly Asn Glu Gln Arg Leu Lys Asp Leu Glu Glu Lys Leu Asp Arg Asp
       210                 215                 220
   aaa tac aag gga act cgt atc att gga gtt gtc ggg atg ccc gga att      720
   Lys Tyr Lys Gly Thr Arg Ile Ile Gly Val Val Gly Met Pro Gly Ile
   225                 230                 235                 240
   ggt aaa acc aca ctc ctg aaa gag cta tat aag acg tgg cag ggc aag      768
   Gly Lys Thr Thr Leu Leu Lys Glu Leu Tyr Lys Thr Trp Gln Gly Lys
                   245                 250                 255
   ttt tcg agg cat gcg cta atc gat caa atc cgt gta aag tcc aag cac      816
   Phe Ser Arg His Ala Leu Ile Asp Gln Ile Arg Val Lys Ser Lys His
               260                 265                 270
   ttg gag ttg gat cgc ttg cct caa atg gtc tta ggc gag tta tcg aag      864
   Leu Glu Leu Asp Arg Leu Pro Gln Met Val Leu Gly Glu Leu Ser Lys
           275                 280                 285
   tta aac aat cct cac gta gac aat ctc aaa gat cca tac agt caa ctg      912
   Leu Asn Asn Pro His Val Asp Asn Leu Lys Asp Pro Tyr Ser Gln Leu
       290                 295                 300
   cat gaa cgc aaa gtg ctt gtt gta ctt gat gat gtt agt aaa agg gaa      960
   His Glu Arg Lys Val Leu Val Val Leu Asp Asp Val Ser Lys Arg Glu
   305                 310                 315                 320
   caa ata gat gct ctt cgt gag ata cta gac tgg att aag gag ggt aag     1008
   Gln Ile Asp Ala Leu Arg Glu Ile Leu Asp Trp Ile Lys Glu Gly Lys
                   325                 330                 335
   gag gga agc agg gtt gtc att gca aca agc gac atg tcc tta aca aat      1056
   Glu Gly Ser Arg Val Val Ile Ala Thr Ser Asp Met Ser Leu Thr Asn
               340                 345                 350
   ggt ttg gtt gat gat act tac atg gtt caa aat ttg aac cac aga gat      1104
   Gly Leu Val Asp Asp Thr Tyr Met Val Gln Asn Leu Asn His Arg Asp
           355                 360                 365
   agc tta caa cta ttt cac tat cat gcc ttt att gat gat caa gcc aat      1152
   Ser Leu Gln Leu Phe His Tyr His Ala Phe Ile Asp Asp Gln Ala Asn
       370                 375                 380
   cct caa aag aaa gat ttc atg aag ctg tca gaa ggg ttt gta cat tac      1200
   Pro Gln Lys Lys Asp Phe Met Lys Leu Ser Glu Gly Phe Val His Tyr
   385                 390                 395                 400
   gcc aga ggc cat cca cta gcc ctc aaa gta ttg ggt gga gag ctt aat      1248
   Ala Arg Gly His Pro Leu Ala Leu Lys Val Leu Gly Gly Glu Leu Asn
                   405                 410                 415
   aag aaa agt atg gat cat tgg aat tcg aaa atg aag aaa ctt gca cag      1296
   Lys Lys Ser Met Asp His Trp Asn Ser Lys Met Lys Lys Leu Ala Gln
               420                 425                 430
   agc ccc agc cct aat att gtc agt gtc ttc caa gtg agt tat gat gaa      1344
   Ser Pro Ser Pro Asn Ile Val Ser Val Phe Gln Val Ser Tyr Asp Glu
           435                 440                 445
   ttg act aca gcg cag aaa gat gca ttt ctc gac ata gcc tgt ttc aga      1392
   Leu Thr Thr Ala Gln Lys Asp Ala Phe Leu Asp Ile Ala Cys Phe Arg
       450                 455                 460
   tca cag gac aag gat tat gta gaa agt cta ctg gct tca tct gac ctt      1440
   Ser Gln Asp Lys Asp Tyr Val Glu Ser Leu Leu Ala Ser Ser Asp Leu
   465                 470                 475                 480
   ggt tcg gct gaa gca atg agt gca gtc aaa tct ctc acg gat aag ttc      1488
   Gly Ser Ala Glu Ala Met Ser Ala Val Lys Ser Leu Thr Asp Lys Phe
                   485                 490                 495
   ctg att aat act tgt gat ggc cga gtg gag atg cat gat cta tta tat      1536
   Leu Ile Asn Thr Cys Asp Gly Arg Val Glu Met His Asp Leu Leu Tyr
               500                 505                 510
   aaa ttt tca agg gaa gtt gat ctg aag gca tct aat cag gat ggt agc      1584
   Lys Phe Ser Arg Glu Val Asp Leu Lys Ala Ser Asn Gln Asp Gly Ser
           515                 520                 525
   aga caa cgg agg ctg tgg ctc cat caa cac ata atc aag gga ggc ata      1632
   Arg Gln Arg Arg Leu Trp Leu His Gln His Ile Ile Lys Gly Gly Ile
       530                 535                 540
   atc aat gta ttg caa aat aaa atg aaa gct gcc aat gtt aga ggt att      1680
   Ile Asn Val Leu Gln Asn Lys Met Lys Ala Ala Asn Val Arg Gly Ile
   545                 550                 555                 560
   ttc cta gac ttg tct gaa gtg gaa gac gaa acg agc tta gac cgc gac      1728
   Phe Leu Asp Leu Ser Glu Val Glu Asp Glu Thr Ser Leu Asp Arg Asp
                   565                 570                 575
   cac ttc ata aat atg gga aat ctc cgg tat ctc aag ttc tac aat tcc      1776
   His Phe Ile Asn Met Gly Asn Leu Arg Tyr Leu Lys Phe Tyr Asn Ser
               580                 585                 590
   cat tgt cca cag gaa tgt aaa acc aac aat aaa atc aac atc cct gat      1824
   His Cys Pro Gln Glu Cys Lys Thr Asn Asn Lys Ile Asn Ile Pro Asp
           595                 600                 605
   aaa ctt aag cta cca ttg aaa gaa gtt cga tgc ctc cac tgg ctg aaa      1872
   Lys Leu Lys Leu Pro Leu Lys Glu Val Arg Cys Leu His Trp Leu Lys
       610                 615                 620
   ttc cca ttg gag aca ctt cca aac gat ttc aac cca att aat ctt gtc      1920
   Phe Pro Leu Glu Thr Leu Pro Asn Asp Phe Asn Pro Ile Asn Leu Val
   625                 630                 635                 640
   gac ctt aag ctg ccc tac agt gaa atg gaa caa ctt tgg gag ggt gac      1968
   Asp Leu Lys Leu Pro Tyr Ser Glu Met Glu Gln Leu Trp Glu Gly Asp
                   645                 650                 655
   aag gac aca cca tgc tta agg tgg gtc gat ctc aat cac tca agt aag      2016
   Lys Asp Thr Pro Cys Leu Arg Trp Val Asp Leu Asn His Ser Ser Lys
               660                 665                 670
   ttg tgc agc ttg tca ggg tta tca aaa gct gaa aag ctt caa agg ttg      2064
   Leu Cys Ser Leu Ser Gly Leu Ser Lys Ala Glu Lys Leu Gln Arg Leu
           675                 680                 685
   aac ctt gaa ggt tgc aca aca ctg aaa gcg ttt cca cat gat atg aaa      2112
   Asn Leu Glu Gly Cys Thr Thr Leu Lys Ala Phe Pro His Asp Met Lys
       690                 695                 700
   aaa atg aaa atg ctt gct ttc ttg aat ctg aag gga tgt aca agt ctc      2160
   Lys Met Lys Met Leu Ala Phe Leu Asn Leu Lys Gly Cys Thr Ser Leu
   705                 710                 715                 720
   gaa tct ctt cca gag atg aat ttg att tct ctg aaa aca ctt act ctc      2208
   Glu Ser Leu Pro Glu Met Asn Leu Ile Ser Leu Lys Thr Leu Thr Leu
                   725                 730                 735
   agc ggc tgc tca act ttt aag gaa ttt ccg ttg att tca gat aat ata      2256
   Ser Gly Cys Ser Thr Phe Lys Glu Phe Pro Leu Ile Ser Asp Asn Ile
               740                 745                 750
   gaa act cta tac tta gat ggc aca gca ata agt cag ctt cct atg aac      2304
   Glu Thr Leu Tyr Leu Asp Gly Thr Ala Ile Ser Gln Leu Pro Met Asn
           755                 760                 765
   atg gag aag ctt cag aga ctt gtt gta ttg aat atg aaa gac tgc aaa      2352
   Met Glu Lys Leu Gln Arg Leu Val Val Leu Asn Met Lys Asp Cys Lys
       770                 775                 780
   atg cta gag gaa att cca ggc cgt gtt ggt gag ctg aaa gct ctt caa      2400
   Met Leu Glu Glu Ile Pro Gly Arg Val Gly Glu Leu Lys Ala Leu Gln
   785                 790                 795                 800
   gaa ctg ata ctc tct gat tgt tta aat ctc aag att ttc cca gaa atc      2448
   Glu Leu Ile Leu Ser Asp Cys Leu Asn Leu Lys Ile Phe Pro Glu Ile
                   805                 810                 815
   aac att agc ttt tta aac att tta ctt ttg gat ggg aca gcc att gaa      2496
   Asn Ile Ser Phe Leu Asn Ile Leu Leu Leu Asp Gly Thr Ala Ile Glu
                   820                 825                 830
   gtg atg cca caa tta ccc tca gtg cag tat ttg tgc tta agc aga aat      2544
   Val Met Pro Gln Leu Pro Ser Val Gln Tyr Leu Cys Leu Ser Arg Asn
               835                 840                 845
   gct aag atc agc tgc ctt cct gtt gga atc agt cag ctt tct caa ctt      2592
   Ala Lys Ile Ser Cys Leu Pro Val Gly Ile Ser Gln Leu Ser Gln Leu
       850                 855                 860
   aaa tgg ctg gac ctg aag tat tgt acg agt ctt aca tca gtt cca gag      2640
   Lys Trp Leu Asp Leu Lys Tyr Cys Thr Ser Leu Thr Ser Val Pro Glu
   865                 870                 875                 880
   ttt cca cca aat ctt cag tgc tta gat gca cac ggc tgt agt tcg ctg      2688
   Phe Pro Pro Asn Leu Gln Cys Leu Asp Ala His Gly Cys Ser Ser Leu
                   885                 890                 895
   aag aca gtt tca aag cct ttg gcc cgt atc atg cca act gaa cag aat      2736
   Lys Thr Val Ser Lys Pro Leu Ala Arg Ile Met Pro Thr Glu Gln Asn
                   900                 905                 910
   cat tcc aca ttc att ttc acc aac tgt gag aac ctg gaa caa gct gca      2784
   His Ser Thr Phe Ile Phe Thr Asn Cys Glu Asn Leu Glu Gln Ala Ala
               915                 920                 925
   aag gag gaa atc aca tcg tat gct caa agg aaa tgc cag ctg tta tca      2832
   Lys Glu Glu Ile Thr Ser Tyr Ala Gln Arg Lys Cys Gln Leu Leu Ser
       930                 935                 940
   tat gct cgg aaa cgc tac aat ggg ggt ctt gtt tca gaa tcc ttg ttc      2880
   Tyr Ala Arg Lys Arg Tyr Asn Gly Gly Leu Val Ser Glu Ser Leu Phe
   945                 950                 955                 960
   agc act tgc ttt cct gga tgt gaa gtg cct tct tgg ttt tgt cat gaa      2928
   Ser Thr Cys Phe Pro Gly Cys Glu Val Pro Ser Trp Phe Cys His Glu
                   965                 970                 975
   aca gtt gga tct gag tta gaa gta aaa ctc ctc cca cat tgg cat gac      2976
   Thr Val Gly Ser Glu Leu Glu Val Lys Leu Leu Pro His Trp His Asp
                   980                 985                 990
   aag aag ctt gcc ggg att gct cta  tgt gct gta gtc tca  tgt ctt gac    3024
   Lys Lys Leu Ala Gly Ile Ala Leu  Cys Ala Val Val Ser  Cys Leu Asp
               995                 1000                1005
   ccc caa  gat caa gtc agc cgc  ttg tca gtg acc tgc  acc ttt aaa      3069
   Pro Gln  Asp Gln Val Ser Arg  Leu Ser Val Thr Cys  Thr Phe Lys
       1010                 1015                 1020
   gta aaa  gat gaa gac aag tct  tgg gtc cca tat act  tgt cca gta      3114
   Val Lys  Asp Glu Asp Lys Ser  Trp Val Pro Tyr Thr  Cys Pro Val
       1025                 1030                 1035
   gga agt  tgg acc aga cat gga  ggc ggg aaa gat aaa  att gag cta      3159
   Gly Ser  Trp Thr Arg His Gly  Gly Gly Lys Asp Lys  Ile Glu Leu
       1040                 1045                 1050
   gac cat  gtc ttc att gga tac  acc agt tgc cca cat  act ata aaa      3204
   Asp His Val Phe Ile Gly Tyr Thr Ser Cys Pro His Thr Ile Lys
       1055                1060                1065
   tgt cat gaa gaa ggt aac tca gat gaa tgc aat cct acc gaa gcc      3249
   Cys His Glu Glu Gly Asn Ser Asp Glu Cys Asn Pro Thr Glu Ala
       1070                1075                1080
   tct ctt aaa ttt act gtg aca gga ggt aca agt gag aat gga aaa      3294
   Ser Leu Lys Phe Thr Val Thr Gly Gly Thr Ser Glu Asn Gly Lys
       1085                1090                1095
   tac aag gtg ttg aag tgt ggt tta agt ttg gtg tat gct aag gat      3339
   Tyr Lys Val Leu Lys Cys Gly Leu Ser Leu Val Tyr Ala Lys Asp
       1100                1105                1110
   aaa gac aaa aat agt gct ctt gaa aca aag tac gat atg ctt att      3384
   Lys Asp Lys Asn Ser Ala Leu Glu Thr Lys Tyr Asp Met Leu Ile
       1115                1120                1125
   gga aag agc ttt caa gaa act tca gaa ggg gtt gat gga agg gta      3429
   Gly Lys Ser Phe Gln Glu Thr Ser Glu Gly Val Asp Gly Arg Val
       1130                1135                1140
   aag aaa acg aaa gga aag tac gtt atg cct gtt gaa aag aac ttt      3474
   Lys Lys Thr Lys Gly Lys Tyr Val Met Pro Val Glu Lys Asn Phe
       1145                1150                1155
   caa gaa act aca gaa ggg gtt gat gga aga gta aag aaa aag aaa      3519
   Gln Glu Thr Thr Glu Gly Val Asp Gly Arg Val Lys Lys Lys Lys
       1160                1165                1170
   aaa aca aga atg gat aat gga cgg cca aag aag aag cag aga tca      3564
   Lys Thr Arg Met Asp Asn Gly Arg Pro Lys Lys Lys Gln Arg Ser
       1175                1180                1185
   gga aga gat gat aat caa act agg atg caa gtg gaa ctc caa gaa      3609
   Gly Arg Asp Asp Asn Gln Thr Arg Met Gln Val Glu Leu Gln Glu
       1190                1195                1200
   gga aat ata aat tca gtg atc atg cac acg gtt aag aat ttc tga      3654
   Gly Asn Ile Asn Ser Val Ile Met His Thr Val Lys Asn Phe
       1205                1210                1215
<210> 6
   <211> 1217
   <212> PRT
   <213> Arabidopsis thaliana No-0
<400> 6
   Met Glu Thr Ser Ser Ile Ser Thr Val Glu Asp Lys Pro Pro Gln His
   1               5                   10                  15
   Gln Val Phe Ile Asn Phe Arg Gly Ala Asp Leu Arg Arg Arg Phe Val
               20                  25                  30
   Ser His Leu Val Thr Ala Leu Lys Leu Asn Asn Ile Asn Val Phe Ile
           35                  40                  45
   Asp Asp Tyr Glu Asp Arg Gly Gln Pro Leu Asp Val Leu Leu Lys Arg
       50                  55                  60
   Ile Glu Glu Ser Lys Ile Val Leu Ala Ile Phe Ser Gly Asn Tyr Thr
   65                  70                  75                  80
   Glu Ser Val Trp Cys Val Arg Glu Leu Glu Lys Ile Lys Asp Cys Thr
                   85                  90                  95
   Asp Glu Gly Thr Leu Val Ala Ile Pro Ile Phe Tyr Lys Leu Glu Pro
               100                 105                 110
   Ser Thr Val Arg Asp Leu Lys Gly Lys Phe Gly Asp Arg Phe Arg Ser
           115                 120                 125
   Met Ala Lys Gly Asp Glu Arg Lys Lys Lys Trp Lys Glu Ala Phe Asn
       130                 135                 140
   Leu Ile Pro Asn Ile Met Gly Ile Thr Ile Asp Lys Lys Ser Val Glu
   145                 150                 155                 160
   Ser Glu Lys Val Asn Glu Ile Val Lys Ala Val Lys Thr Ala Leu Thr
                   165                 170                 175
   Gly Ile Pro Pro Glu Gly Ser His Asn Ala Val Val Gly Ala Leu Gly
               180                 185                 190
   Asn Ser Asn Ala Gly Thr Ser Ser Gly Asp Lys Lys His Glu Thr Phe
           195                 200                 205
   Gly Asn Glu Gln Arg Leu Lys Asp Leu Glu Glu Lys Leu Asp Arg Asp
       210                 215                 220
   Lys Tyr Lys Gly Thr Arg Ile Ile Gly Val Val Gly Met Pro Gly Ile
   225                 230                 235                 240
   Gly Lys Thr Thr Leu Leu Lys Glu Leu Tyr Lys Thr Trp Gln Gly Lys
                   245                 250                 255
   Phe Ser Arg His Ala Leu Ile Asp Gln Ile Arg Val Lys Ser Lys His
               260                 265                 270
   Leu Glu Leu Asp Arg Leu Pro Gln Met Val Leu Gly Glu Leu Ser Lys
           275                 280                 285
   Leu Asn Asn Pro His Val Asp Asn Leu Lys Asp Pro Tyr Ser Gln Leu
       290                 295                 300
   His Glu Arg Lys Val Leu Val Val Leu Asp Asp Val Ser Lys Arg Glu
   305                 310                 315                 320
   Gln Ile Asp Ala Leu Arg Glu Ile Leu Asp Trp Ile Lys Glu Gly Lys
                   325                 330                 335
   Glu Gly Ser Arg Val Val Ile Ala Thr Ser Asp Met Ser Leu Thr Asn
               340                 345                 350
   Gly Leu Val Asp Asp Thr Tyr Met Val Gln Asn Leu Asn His Arg Asp
           355                 360                 365
   Ser Leu Gln Leu Phe His Tyr His Ala Phe Ile Asp Asp Gln Ala Asn
       370                 375                 380
   Pro Gln Lys Lys Asp Phe Met Lys Leu Ser Glu Gly Phe Val His Tyr
   385                 390                 395                 400
   Ala Arg Gly His Pro Leu Ala Leu Lys Val Leu Gly Gly Glu Leu Asn
                   405                 410                 415
   Lys Lys Ser Met Asp His Trp Asn Ser Lys Met Lys Lys Leu Ala Gln
               420                 425                 430
   Ser Pro Ser Pro Asn Ile Val Ser Val Phe Gln Val Ser Tyr Asp Glu
           435                 440                 445
   Leu Thr Thr Ala Gln Lys Asp Ala Phe Leu Asp Ile Ala Cys Phe Arg
       450                 455                 460
   Ser Gln Asp Lys Asp Tyr Val Glu Ser Leu Leu Ala Ser Ser Asp Leu
   465                 470                 475                 480
   Gly Ser Ala Glu Ala Met Ser Ala Val Lys Ser Leu Thr Asp Lys Phe
                   485                 490                 495
   Leu Ile Asn Thr Cys Asp Gly Arg Val Glu Met His Asp Leu Leu Tyr
               500                 505                 510
   Lys Phe Ser Arg Glu Val Asp Leu Lys Ala Ser Asn Gln Asp Gly Ser
           515                 520                 525
   Arg Gln Arg Arg Leu Trp Leu His Gln His Ile Ile Lys Gly Gly Ile
       530                 535                 540
   Ile Asn Val Leu Gln Asn Lys Met Lys Ala Ala Asn Val Arg Gly Ile
   545                 550                 555                 560
   Phe Leu Asp Leu Ser Glu Val Glu Asp Glu Thr Ser Leu Asp Arg Asp
                   565                 570                 575
   His Phe Ile Asn Met Gly Asn Leu Arg Tyr Leu Lys Phe Tyr Asn Ser
               580                 585                 590
   His Cys Pro Gln Glu Cys Lys Thr Asn Asn Lys Ile Asn Ile Pro Asp
           595                 600                 605
   Lys Leu Lys Leu Pro Leu Lys Glu Val Arg Cys Leu His Trp Leu Lys
       610                 615                 620
   Phe Pro Leu Glu Thr Leu Pro Asn Asp Phe Asn Pro Ile Asn Leu Val
   625                 630                 635                 640
   Asp Leu Lys Leu Pro Tyr Ser Glu Met Glu Gln Leu Trp Glu Gly Asp
                   645                 650                 655
   Lys Asp Thr Pro Cys Leu Arg Trp Val Asp Leu Asn His Ser Ser Lys
               660                 665                 670
   Leu Cys Ser Leu Ser Gly Leu Ser Lys Ala Glu Lys Leu Gln Arg Leu
           675                 680                 685
   Asn Leu Glu Gly Cys Thr Thr Leu Lys Ala Phe Pro His Asp Met Lys
       690                 695                 700
   Lys Met Lys Met Leu Ala Phe Leu Asn Leu Lys Gly Cys Thr Ser Leu
   705                 710                 715                 720
   Glu Ser Leu Pro Glu Met Asn Leu Ile Ser Leu Lys Thr Leu Thr Leu
                   725                 730                 735
   Ser Gly Cys Ser Thr Phe Lys Glu Phe Pro Leu Ile Ser Asp Asn Ile
               740                 745                 750
   Glu Thr Leu Tyr Leu Asp Gly Thr Ala Ile Ser Gln Leu Pro Met Asn
           755                 760                 765
   Met Glu Lys Leu Gln Arg Leu Val Val Leu Asn Met Lys Asp Cys Lys
   770 775 780
   Met Leu Glu Glu Ile Pro Gly Arg Val Gly Glu Leu Lys Ala Leu Gln
   785 790 795 800
   Glu Leu Ile Leu Ser Asp Cys Leu Asn Leu Lys Ile Phe Pro Glu Ile
   805 810 815
   Asn Ile Ser Phe Leu Asn Ile Leu Leu Leu Asp Gly Thr Ala Ile Glu
   820 825 830
   Val Met Pro Gln Leu Pro Ser Val Gln Tyr Leu Cys Leu Ser Arg Asn
   835 840 845
   Ala Lys Ile Ser Cys Leu Pro Val Gly Ile Ser Gln Leu Ser Gln Leu
   850 855 860
   Lys Trp Leu Asp Leu Lys Tyr Cys Thr Ser Leu Thr Ser Val Pro Glu
   865 870 875 880
   Phe Pro Pro Asn Leu Gln Cys Leu Asp Ala His Gly Cys Ser Ser Leu
   885 890 895
   Lys Thr Val Ser Lys Pro Leu Ala Arg Ile Met Pro Thr Glu Gln Asn
   900 905 910
   His Ser Thr Phe Ile Phe Thr Asn Cys Glu Asn Leu Glu Gln Ala Ala
   915 920 925
   Lys Glu Glu Ile Thr Ser Tyr Ala Gln Arg Lys Cys Gln Leu Leu Ser
   930 935 940
   Tyr Ala Arg Lys Arg Tyr Asn Gly Gly Leu Val Ser Glu Ser Leu Phe
   945 950 955 960
   Ser Thr Cys Phe Pro Gly Cys Glu Val Pro Ser Trp Phe Cys His Glu
   965 970 975
   Thr Val Gly Ser Glu Leu Glu Val Lys Leu Leu Pro His Trp His Asp
   980 985 990
   Lys Lys Leu Ala Gly Ile Ala Leu Cys Ala Val Val Ser Cys Leu Asp
   995 1000 1005
   Pro Gln Asp Gln Val Ser Arg Leu Ser Val Thr Cys Thr Phe Lys
   1010 1015 1020
   Val Lys Asp Glu Asp Lys Ser Trp Val Pro Tyr Thr Cys Pro Val
       1025                1030                1035
   Gly Ser Trp Thr Arg His Gly Gly Gly Lys Asp Lys Ile Glu Leu
       1040                1045                1050
   Asp His Val Phe Ile Gly Tyr Thr Ser Cys Pro His Thr Ile Lys
       1055                1060                1065
   Cys His Glu Glu Gly Asn Ser Asp Glu Cys Asn Pro Thr Glu Ala
       1070                1075                1080
   Ser Leu Lys Phe Thr Val Thr Gly Gly Thr Ser Glu Asn Gly Lys
       1085                1090                1095
   Tyr Lys Val Leu Lys Cys Gly Leu Ser Leu Val Tyr Ala Lys Asp
       1100                1105                1110
   Lys Asp Lys Asn Ser Ala Leu Glu Thr Lys Tyr Asp Met Leu Ile
       1115                1120                1125
   Gly Lys Ser Phe Gln Glu Thr Ser Glu Gly Val Asp Gly Arg Val
       1130                1135                1140
   Lys Lys Thr Lys Gly Lys Tyr Val Met Pro Val Glu Lys Asn Phe
       1145                1150                1155
   Gln Glu Thr Thr Glu Gly Val Asp Gly Arg Val Lys Lys Lys Lys
       1160                1165                1170
   Lys Thr Arg Met Asp Asn Gly Arg Pro Lys Lys Lys Gln Arg Ser
       1175                1180                1185
   Gly Arg Asp Asp Asn Gln Thr Arg Met Gln Val Glu Leu Gln Glu
       1190                1195                1200
   Gly Asn Ile Asn Ser Val Ile Met His Thr Val Lys Asn Phe
       1205                1210                1215
<210> 7
   <211> 4137
   <212> DNA
   <213> Arabidopsis thaliana No-0
<220>
   <221> CDS
   <222> (1)..(4134)
   <223>
<400> 7
   atg acc aat tgt gaa aag gat gag gaa ttc gtg tgc atc agc tgc gta       48
   Met Thr Asn Cys Glu Lys Asp Glu Glu Phe Val Cys Ile Ser Cys Val
   1               5                   10                  15
   gaa gag gta cgg tac tct ttc gtg agc cac ctc tct gaa gct ctc cgt       96
   Glu Glu Val Arg Tyr Ser Phe Val Ser His Leu Ser Glu Ala Leu Arg
               20                  25                  30
   cga aaa ggc ata aat aat gtg gtc gta gat gta gat atc gat gat ctg      144
   Arg Lys Gly Ile Asn Asn Val Val Val Asp Val Asp Ile Asp Asp Leu
           35                  40                  45
   ctt ttc aag gag tct cag gca aag atc gag aaa gct ggg gtt tct gtg      192
   Leu Phe Lys Glu Ser Gln Ala Lys Ile Glu Lys Ala Gly Val Ser Val
       50                  55                  60
   atg gtt tta ccc gga aac tgt gac cct tcc gag gta tgg ctt gac aag      240
   Met Val Leu Pro Gly Asn Cys Asp Pro Ser Glu Val Trp Leu Asp Lys
   65                  70                  75                  80
   ttc gcc aag gtt ctc gag tgc caa agg aac aac aag gac cag gcg gtg      288
   Phe Ala Lys Val Leu Glu Cys Gln Arg Asn Asn Lys Asp Gln Ala Val
                   85                  90                  95
   gtt tca gtg ttg tac ggt gac agt cta tta cgg gac caa tgg ctt agc      336
   Val Ser Val Leu Tyr Gly Asp Ser Leu Leu Arg Asp Gln Trp Leu Ser
               100                 105                 110
   gag ctg gat ttc aga ggc tta tca cga att cac caa tcc agg aag gaa      384
   Glu Leu Asp Phe Arg Gly Leu Ser Arg Ile His Gln Ser Arg Lys Glu
           115                 120                 125
   tgt agt gac tct ata ctt gta gaa gag att gtg aga gat gtg tac gag      432
   Cys Ser Asp Ser Ile Leu Val Glu Glu Ile Val Arg Asp Val Tyr Glu
       130                 135                 140
   acg cac ttt tat gtt gga cga att gga atc tat tcg aag ctg ctg gag      480
   Thr His Phe Tyr Val Gly Arg Ile Gly Ile Tyr Ser Lys Leu Leu Glu
   145                 150                 155                 160
   att gaa aac atg gtt aac aag caa ccg ata ggc atc cgt tgt gtt gga      528
   Ile Glu Asn Met Val Asn Lys Gln Pro Ile Gly Ile Arg Cys Val Gly
                   165                 170                 175
   att tgg ggt atg cct ggc ata gga aag aca aca ctt gct aaa gca gtc      576
   Ile Trp Gly Met Pro Gly Ile Gly Lys Thr Thr Leu Ala Lys Ala Val
               180                 185                 190
   ttt gac caa atg tct agc gcc ttt gat gct tct tgt ttt atc gaa gac      624
   Phe Asp Gln Met Ser Ser Ala Phe Asp Ala Ser Cys Phe Ile Glu Asp
           195                 200                 205
   tat gac aaa tca att cat gag aag ggt ctt tat tgt ttg ctg gag gaa      672
   Tyr Asp Lys Ser Ile His Glu Lys Gly Leu Tyr Cys Leu Leu Glu Glu
       210                 215                 220
   caa ctt ttg ccg ggt aat gat gca acc att atg aaa ctg agc tcg ctc      720
   Gln Leu Leu Pro Gly Asn Asp Ala Thr Ile Met Lys Leu Ser Ser Leu
   225                 230                 235                 240
   aga gac aga ttg aac agt aag aga gtt ctt gtt gtt ctc gat gac gtg      768
   Arg Asp Arg Leu Asn Ser Lys Arg Val Leu Val Val Leu Asp Asp Val
                   245                 250                 255
   tgc aat gct ctg gtt gca gag tct ttt ctc gag ggg ttt gac tgg cta      816
   Cys Asn Ala Leu Val Ala Glu Ser Phe Leu Glu Gly Phe Asp Trp Leu
               260                 265                 270
   gga ccc gga agc ctg atc atc ata acc tct aga gat aaa caa gtg ttt      864
   Gly Pro Gly Ser Leu Ile Ile Ile Thr Ser Arg Asp Lys Gln Val Phe
           275                 280                 285
   cgc ctt tgc gga atc aat caa ata tat gag gtc cag ggt tta aat gag      912
   Arg Leu Cys Gly Ile Asn Gln Ile Tyr Glu Val Gln Gly Leu Asn Glu
       290                 295                 300
   aaa gag gct cgt caa ctt ttc ttg ctg tct gcg tct ata atg gag gat      960
   Lys Glu Ala Arg Gln Leu Phe Leu Leu Ser Ala Ser Ile Met Glu Asp
   305                 310                 315                 320
   atg gga gag cag aat ctc cat gag ttg tca gtg aga gta ata agt tac     1008
   Met Gly Glu Gln Asn Leu His Glu Leu Ser Val Arg Val Ile Ser Tyr
                   325                 330                 335
   gct aat gga aac ccg tta gct atc agt gtt tat gga aga gag ctg aaa     1056
   Ala Asn Gly Asn Pro Leu Ala Ile Ser Val Tyr Gly Arg Glu Leu Lys
               340                 345                 350
   ggt aag aaa aaa ctc tca gaa atg gag act gca ttc ctc aaa ctc aag     1104
   Gly Lys Lys Lys Leu Ser Glu Met Glu Thr Ala Phe Leu Lys Leu Lys
           355                 360                 365
   cga cgt cct cca ttt aag att gtc gat gca ttt aaa agc agc tac gac     1152
   Arg Arg Pro Pro Phe Lys Ile Val Asp Ala Phe Lys Ser Ser Tyr Asp
       370                 375                 380
   aca ctc agt gac aac gaa aag aac att ttt ttg gac ata gct tgt ttt     1200
   Thr Leu Ser Asp Asn Glu Lys Asn Ile Phe Leu Asp Ile Ala Cys Phe
   385                 390                 395                 400
   ttc cag gga gaa aat gtc aac tac gtg ata caa ctg ctt gag ggt tgt     1248
   Phe Gln Gly Glu Asn Val Asn Tyr Val Ile Gln Leu Leu Glu Gly Cys
                   405                 410                 415
   ggt ttc ttt cca cat gtt gaa att gat gtc ctt gtt gac aag tgt ctg     1296
   Gly Phe Phe Pro His Val Glu Ile Asp Val Leu Val Asp Lys Cys Leu
               420                 425                 430
   gtg act att tca gaa aac cga gtt tgg ttg cat aag ctg acc cag gat     1344
   Val Thr Ile Ser Glu Asn Arg Val Trp Leu His Lys Leu Thr Gln Asp
           435                 440                 445
   atc ggc cga gaa atc ata aat gga gaa aca gta cag atc gag agg cgc     1392
   Ile Gly Arg Glu Ile Ile Asn Gly Glu Thr Val Gln Ile Glu Arg Arg
       450                 455                 460
   aga aga ctg tgg gaa cct tgg agc atc aaa tat tta tta gaa tat aat     1440
   Arg Arg Leu Trp Glu Pro Trp Ser Ile Lys Tyr Leu Leu Glu Tyr Asn
   465 470 475 480
   gaa cac aaa gca aat gga gaa cct aaa aca acc ttc aaa cgt gct cag 1488
   Glu His Lys Ala Asn Gly Glu Pro Lys Thr Thr Phe Lys Arg Ala Gln
   485 490 495
   ggc tct gaa gag atc gaa ggc ctg ttt cta gac aca tca aac tta aga 1536
   Gly Ser Glu Glu Ile Glu Gly Leu Phe Leu Asp Thr Ser Asn Leu Arg
   500 505 510
   ttt gat ctg cag ccc tct gcc ttt aag aat atg ttg aac ctt aga ttg 1584
   Phe Asp Leu Gln Pro Ser Ala Phe Lys Asn Met Leu Asn Leu Arg Leu
   515 520 525
   ctc aaa att tat tgt tcc aat cct gaa gtc cat cct gta atc aat ttc 1632
   Leu Lys Ile Tyr Cys Ser Asn Pro Glu Val His Pro Val Ile Asn Phe
   530 535 540
   cca aca ggc tct ctg cat tct ctt cct aat gag cta aga ctc ctc cat 1680
   Pro Thr Gly Ser Leu His Ser Leu Pro Asn Glu Leu Arg Leu Leu His
   545 550 555 560
   tgg gag aac tat cct ctg aaa tct ttg cct cag aat ttt gat cct agg 1728
   Trp Glu Asn Tyr Pro Leu Lys Ser Leu Pro Gln Asn Phe Asp Pro Arg
   565 570 575
   cac ctt gtc gaa atc aac atg ccg tat agt caa ctt cag aaa ctt tgg 1776
   His Leu Val Glu Ile Asn Met Pro Tyr Ser Gln Leu Gln Lys Leu Trp
   580 585 590
   ggt gga acc aag aac ctg gag atg ttg agg acg atc agg ctt tgc cat 1824
   Gly Gly Thr Lys Asn Leu Glu Met Leu Arg Thr Ile Arg Leu Cys His
   595 600 605
   tcc cag cat cta gtt gat atc gat gat ctc tta aaa gct gaa aat ctt 1872
   Ser Gln His Leu Val Asp Ile Asp Asp Leu Leu Lys Ala Glu Asn Leu
   610 615 620
   gag gta att gat ctc caa ggt tgt acg aga ctg cag aat ttc cca gcc 1920
   Glu Val Ile Asp Leu Gln Gly Cys Thr Arg Leu Gln Asn Phe Pro Ala
   625 630 635 640
   gca ggt cga ttg cta cgt cta cga gtt gta aat ctc tca ggt tgc ata 1968
   Ala Gly Arg Leu Leu Arg Leu Arg Val Val Asn Leu Ser Gly Cys Ile
   645 650 655
   aag att aaa agt gtc cta gaa att cca cca aat att gag aaa cta cat 2016
   Lys Ile Lys Ser Val Leu Glu Ile Pro Pro Asn Ile Glu Lys Leu His
   660 665 670
   cta cag gga act ggc ata tta gca tta cca gtt tcc act gtt aag cca 2064
   Leu Gln Gly Thr Gly Ile Leu Ala Leu Pro Val Ser Thr Val Lys Pro
   675 680 685
   aac cat aga gag ctt gtg aat ttt cta aca gaa att ccg ggt ctt tca 2112
   Asn His Arg Glu Leu Val Asn Phe Leu Thr Glu Ile Pro Gly Leu Ser
   690 695 700
   gag gcc tcg aaa ctt gag cgt tta aca agt ctg ctg gaa tct aac tca 2160
   Glu Ala Ser Lys Leu Glu Arg Leu Thr Ser Leu Leu Glu Ser Asn Ser
   705 710 715 720
   tct tgt caa gat ctt ggg aag ctt att tgc ttg gag ctg aaa gat tgc      2208
   Ser Cys Gln Asp Leu Gly Lys Leu Ile Cys Leu Glu Leu Lys Asp Cys
                   725                 730                 735
   tct tgt ttg cag agt ctg cca aac atg gct aat tta gat ctt aat gtt      2256
   Ser Cys Leu Gln Ser Leu Pro Asn Met Ala Asn Leu Asp Leu Asn Val
               740                 745                 750
   ctt gat ctc tcg ggt tgc tca agt ctt aat tct att cag ggt ttc cct      2304
   Leu Asp Leu Ser Gly Cys Ser Ser Leu Asn Ser Ile Gln Gly Phe Pro
           755                 760                 765
   cgt ttt ctg aaa cag tta tat ctt ggt ggc act gca ata aga gaa gtg      2352
   Arg Phe Leu Lys Gln Leu Tyr Leu Gly Gly Thr Ala Ile Arg Glu Val
       770                 775                 780
   cca caa ctt cct caa agt cta gaa atc ttg aat gca cat gga tct tgt      2400
   Pro Gln Leu Pro Gln Ser Leu Glu Ile Leu Asn Ala His Gly Ser Cys
   785                 790                 795                 800
   ttg cga agt ctg cca aac atg gct aat tta gaa ttt ctc aaa gtt ctt      2448
   Leu Arg Ser Leu Pro Asn Met Ala Asn Leu Glu Phe Leu Lys Val Leu
                   805                 810                 815
   gat ctc tct ggt tgc tca gag ctc gag act att cag ggt ttt cct cgg      2496
   Asp Leu Ser Gly Cys Ser Glu Leu Glu Thr Ile Gln Gly Phe Pro Arg
               820                 825                 830
   aac cta aaa gag tta tat ttt gct ggc act acg tta aga gaa gtg ccc      2544
   Asn Leu Lys Glu Leu Tyr Phe Ala Gly Thr Thr Leu Arg Glu Val Pro
           835                 840                 845
   caa ctt cct tta agc cta gag gtc ttg aat gca cat ggt tct gac tcg      2592
   Gln Leu Pro Leu Ser Leu Glu Val Leu Asn Ala His Gly Ser Asp Ser
       850                 855                 860
   gag aag ctt cct atg cat tac aag ttc aac aat ttt ttc gat cta tct      2640
   Glu Lys Leu Pro Met His Tyr Lys Phe Asn Asn Phe Phe Asp Leu Ser
   865                 870                 875                 880
   caa caa gtg gtc aac gat ttt ttc ttg aaa gcg ctg act tat gta aaa      2688
   Gln Gln Val Val Asn Asp Phe Phe Leu Lys Ala Leu Thr Tyr Val Lys
                   885                 890                 895
   cac ata cca aga ggg tat acg cag gaa ctc atc aac aaa gct ccg act      2736
   His Ile Pro Arg Gly Tyr Thr Gln Glu Leu Ile Asn Lys Ala Pro Thr
               900                 905                 910
   ttc agc ttc agt gcg ccc tca cat aca aat caa aac gcc aca ttt gat      2784
   Phe Ser Phe Ser Ala Pro Ser His Thr Asn Gln Asn Ala Thr Phe Asp
           915                 920                 925
   ctg caa cca gga tct tct gta atg aca cga cta aat cat tca tgg agg      2832
   Leu Gln Pro Gly Ser Ser Val Met Thr Arg Leu Asn His Ser Trp Arg
       930                 935                 940
   aac acg ctt gtg gga ttt ggt atg ctg gtg gaa gtt gca ttt ccc gag      2880
   Asn Thr Leu Val Gly Phe Gly Met Leu Val Glu Val Ala Phe Pro Glu
   945                 950                 955                 960
   gac tac tgt gat gct aca gat gtt ggc ata agt tgt gtt tgc aga tgg     2928
   Asp Tyr Cys Asp Ala Thr Asp Val Gly Ile Ser Cys Val Cys Arg Trp
                   965                 970                 975
   agc aac aaa gaa ggc cgc tct tgt agg ata gaa aga aat ttt cat tgt     2976
   Ser Asn Lys Glu Gly Arg Ser Cys Arg Ile Glu Arg Asn Phe His Cys
               980                 985                 990
   tgg gca cca ggg aaa gtt gtt cca  aaa gtt cga aag gat  cat acg ttt   3024
   Trp Ala Pro Gly Lys Val Val Pro  Lys Val Arg Lys Asp  His Thr Phe
           995                 1000                 1005
   gtc ttt  agt gat gtc aac atg  cgc cca agt acc ggt  gaa gga aat     3069
   Val Phe  Ser Asp Val Asn Met  Arg Pro Ser Thr Gly  Glu Gly Asn
       1010                 1015                 1020
   gac cct  gat atc tgg gct gga  tta gtt gta ttt gag  ttc ttt cct     3114
   Asp Pro  Asp Ile Trp Ala Gly  Leu Val Val Phe Glu  Phe Phe Pro
       1025                 1030                 1035
   atc aat  cag cag aca aag tgt  cta aat gat agg ttc  aca gtg aca     3159
   Ile Asn  Gln Gln Thr Lys Cys  Leu Asn Asp Arg Phe  Thr Val Thr
       1040                 1045                 1050
   aga tgt  gga gtc cgt gta ata  aat gtt gca act ggc  aat aca agt     3204
   Arg Cys  Gly Val Arg Val Ile  Asn Val Ala Thr Gly  Asn Thr Ser
       1055                 1060                 1065
   ctt gag  aac ata tca cta gtt  ttg tct ttg gat cca  gtg gag gtt     3249
   Leu Glu  Asn Ile Ser Leu Val  Leu Ser Leu Asp Pro  Val Glu Val
       1070                 1075                 1080
   tct ggt  tat gaa gta ttg aga  gtc agc tat gat gat  tta cag gag     3294
   Ser Gly  Tyr Glu Val Leu Arg  Val Ser Tyr Asp Asp  Leu Gln Glu
       1085                 1090                 1095
   atg gat  aaa gtt cta ttt ctt  tac ata gcg tct ttg  ttc aat gac     3339
   Met Asp  Lys Val Leu Phe Leu  Tyr Ile Ala Ser Leu  Phe Asn Asp
       1100                 1105                 1110
   gag gat  gtt gat ttt gtg gca  cca ctt att gcc ggt  att gac ttg     3384
   Glu Asp  Val Asp Phe Val Ala  Pro Leu Ile Ala Gly  Ile Asp Leu
       1115                 1120                 1125
   gat gtt  agc tct ggg ctc aag  gtc tta gcc gat gtg  tct ctc ata     3429
   Asp Val  Ser Ser Gly Leu Lys  Val Leu Ala Asp Val  Ser Leu Ile
       1130                 1135                 1140
   agt gta  tca tcc aat ggg gaa  ata gtg atg cat agt  ttg caa aga     3474
   Ser Val  Ser Ser Asn Gly Glu  Ile Val Met His Ser  Leu Gln Arg
       1145                 1150                 1155
   caa atg  ggc aaa gaa atc ctc  cat gga caa tcc atg  ctg ctg tct     3519
   Gln Met  Gly Lys Glu Ile Leu  His Gly Gln Ser Met  Leu Leu Ser
       1160                 1165                 1170
   gat tgt  gag agt tcc atg acc  gag aat ttg tct gac  gta cca aaa     3564
   Asp Cys  Glu Ser Ser Met Thr  Glu Asn Leu Ser Asp  Val Pro Lys
       1175                 1180                 1185
   aag gag  aag aaa cat cgc gaa  agt aag gta aag aaa  gtg gtt tcc     3609
   Lys Glu  Lys Lys His Arg Glu  Ser Lys Val Lys Lys  Val Val Ser
       1190                 1195                 1200
   ata ccg  gct ata gac gag gga  gat cta tgg act tgg  cga aag tac      3654
   Ile Pro  Ala Ile Asp Glu Gly  Asp Leu Trp Thr Trp  Arg Lys Tyr
       1205                 1210                 1215
   ggt caa  aaa gac atc tta ggt  tct cgt ttt cca agg  ggt tac tac      3699
   Gly Gln  Lys Asp Ile Leu Gly  Ser Arg Phe Pro Arg  Gly Tyr Tyr
       1220                 1225                 1230
   agg tgc  gct tac aag ttc acg  cat ggt tgt aaa gct  aca aaa caa      3744
   Arg Cys  Ala Tyr Lys Phe Thr  His Gly Cys Lys Ala  Thr Lys Gln
       1235                 1240                 1245
   gtc caa  cgg agc gag acc gat  tca aac atg tta gct  att act tac      3789
   Val Gln  Arg Ser Glu Thr Asp  Ser Asn Met Leu Ala  Ile Thr Tyr
       1250                 1255                 1260
   cta tct  gag cat aac cat cca  cgg ccc act aaa cgc  aag gct ctc      3834
   Leu Ser  Glu His Asn His Pro  Arg Pro Thr Lys Arg  Lys Ala Leu
       1265                 1270                 1275
   gct gac  tcc act cgt tcc act  tcc tcc tcc atc tgc  tca gcc ata      3879
   Ala Asp  Ser Thr Arg Ser Thr  Ser Ser Ser Ile Cys  Ser Ala Ile
       1280                 1285                 1290
   act acc  tct gcc tca tct aga  gtc ttc caa aac aaa  gac gaa cca      3924
   Thr Thr  Ser Ala Ser Ser Arg  Val Phe Gln Asn Lys  Asp Glu Pro
       1295                 1300                 1305
   aat caa  ccc cac ttg cct tcc  tcc tcc act cct cct  aga aac gcg      3969
   Asn Gln  Pro His Leu Pro Ser  Ser Ser Thr Pro Pro  Arg Asn Ala
       1310                 1315                 1320
   gct gtc  ttg ttt aaa atg acg  gac atg gag gag ttt  cag gac aat      4014
   Ala Val  Leu Phe Lys Met Thr  Asp Met Glu Glu Phe  Gln Asp Asn
       1325                 1330                 1335
   atg gag  gtg gat aat gac gtc  gta gat aca cgt aca  ctg gca ttg      4059
   Met Glu  Val Asp Asn Asp Val  Val Asp Thr Arg Thr  Leu Ala Leu
       1340                 1345                 1350
   ttt cca  gag ttt caa cat cag  ccg gag gaa gaa gac  cca tgg tca      4104
   Phe Pro  Glu Phe Gln His Gln  Pro Glu Glu Glu Asp  Pro Trp Ser
       1355                 1360                 1365
   aca ttc  ttc gat gat tat aat  ttt tac ttt tga                      4137
   Thr Phe  Phe Asp Asp Tyr Asn  Phe Tyr Phe
       1370                 1375
<210> 8
   <211> 1378
   <212> PRT
   <213> Arabidopsis thaliana No-0
<400> 8
   Met Thr Asn Cys Glu Lys Asp Glu Glu Phe Val Cys Ile Ser Cys Val
   1               5                   10                  15
   Glu Glu Val Arg Tyr Ser Phe Val Ser His Leu Ser Glu Ala Leu Arg
               20                  25                  30
   Arg Lys Gly Ile Asn Asn Val Val Val Asp Val Asp Ile Asp Asp Leu
           35                  40                  45
   Leu Phe Lys Glu Ser Gln Ala Lys Ile Glu Lys Ala Gly Val Ser Val
       50                  55                  60
   Met Val Leu Pro Gly Asn Cys Asp Pro Ser Glu Val Trp Leu Asp Lys
   65                  70                  75                  80
   Phe Ala Lys Val Leu Glu Cys Gln Arg Asn Asn Lys Asp Gln Ala Val
                   85                  90                  95
   Val Ser Val Leu Tyr Gly Asp Ser Leu Leu Arg Asp Gln Trp Leu Ser
               100                 105                 110
   Glu Leu Asp Phe Arg Gly Leu Ser Arg Ile His Gln Ser Arg Lys Glu
           115                 120                 125
   Cys Ser Asp Ser Ile Leu Val Glu Glu Ile Val Arg Asp Val Tyr Glu
       130                 135                 140
   Thr His Phe Tyr Val Gly Arg Ile Gly Ile Tyr Ser Lys Leu Leu Glu
   145                 150                 155                 160
   Ile Glu Asn Met Val Asn Lys Gln Pro Ile Gly Ile Arg Cys Val Gly
                   165                 170                 175
   Ile Trp Gly Met Pro Gly Ile Gly Lys Thr Thr Leu Ala Lys Ala Val
               180                 185                 190
   Phe Asp Gln Met Ser Ser Ala Phe Asp Ala Ser Cys Phe Ile Glu Asp
           195                 200                 205
   Tyr Asp Lys Ser Ile His Glu Lys Gly Leu Tyr Cys Leu Leu Glu Glu
       210                 215                 220
   Gln Leu Leu Pro Gly Asn Asp Ala Thr Ile Met Lys Leu Ser Ser Leu
   225                 230                 235                 240
   Arg Asp Arg Leu Asn Ser Lys Arg Val Leu Val Val Leu Asp Asp Val
                   245                 250                 255
   Cys Asn Ala Leu Val Ala Glu Ser Phe Leu Glu Gly Phe Asp Trp Leu
               260                 265                 270
   Gly Pro Gly Ser Leu Ile Ile Ile Thr Ser Arg Asp Lys Gln Val Phe
           275                 280                 285
   Arg Leu Cys Gly Ile Asn Gln Ile Tyr Glu Val Gln Gly Leu Asn Glu
       290                 295                 300
   Lys Glu Ala Arg Gln Leu Phe Leu Leu Ser Ala Ser Ile Met Glu Asp
   305                 310                 315                 320
   Met Gly Glu Gln Asn Leu His Glu Leu Ser Val Arg Val Ile Ser Tyr
                   325                 330                 335
   Ala Asn Gly Asn Pro Leu Ala Ile Ser Val Tyr Gly Arg Glu Leu Lys
               340                 345                 350
   Gly Lys Lys Lys Leu Ser Glu Met Glu Thr Ala Phe Leu Lys Leu Lys
           355                 360                 365
   Arg Arg Pro Pro Phe Lys Ile Val Asp Ala Phe Lys Ser Ser Tyr Asp
       370                 375                 380
   Thr Leu Ser Asp Asn Glu Lys Asn Ile Phe Leu Asp Ile Ala Cys Phe
   385                 390                 395                 400
   Phe Gln Gly Glu Asn Val Asn Tyr Val Ile Gln Leu Leu Glu Gly Cys
                   405                 410                 415
   Gly Phe Phe Pro His Val Glu Ile Asp Val Leu Val Asp Lys Cys Leu
               420                 425                 430
   Val Thr Ile Ser Glu Asn Arg Val Trp Leu His Lys Leu Thr Gln Asp
           435                 440                 445
   Ile Gly Arg Glu Ile Ile Asn Gly Glu Thr Val Gln Ile Glu Arg Arg
       450                 455                 460
   Arg Arg Leu Trp Glu Pro Trp Ser Ile Lys Tyr Leu Leu Glu Tyr Asn
   465                 470                 475                 480
   Glu His Lys Ala Asn Gly Glu Pro Lys Thr Thr Phe Lys Arg Ala Gln
                   485                 490                 495
   Gly Ser Glu Glu Ile Glu Gly Leu Phe Leu Asp Thr Ser Asn Leu Arg
               500                 505                 510
   Phe Asp Leu Gln Pro Ser Ala Phe Lys Asn Met Leu Asn Leu Arg Leu
           515                 520                 525
   Leu Lys Ile Tyr Cys Ser Asn Pro Glu Val His Pro Val Ile Asn Phe
       530                 535                 540
   Pro Thr Gly Ser Leu His Ser Leu Pro Asn Glu Leu Arg Leu Leu His
   545                 550                 555                 560
   Trp Glu Asn Tyr Pro Leu Lys Ser Leu Pro Gln Asn Phe Asp Pro Arg
                   565                 570                 575
   His Leu Val Glu Ile Asn Met Pro Tyr Ser Gln Leu Gln Lys Leu Trp
               580                 585                 590
   Gly Gly Thr Lys Asn Leu Glu Met Leu Arg Thr Ile Arg Leu Cys His
           595                 600                 605
   Ser Gln His Leu Val Asp Ile Asp Asp Leu Leu Lys Ala Glu Asn Leu
       610                 615                 620
   Glu Val Ile Asp Leu Gln Gly Cys Thr Arg Leu Gln Asn Phe Pro Ala
   625                 630                 635                 640
   Ala Gly Arg Leu Leu Arg Leu Arg Val Val Asn Leu Ser Gly Cys Ile
                   645                 650                 655
   Lys Ile Lys Ser Val Leu Glu Ile Pro Pro Asn Ile Glu Lys Leu His
               660                 665                 670
   Leu Gln Gly Thr Gly Ile Leu Ala Leu Pro Val Ser Thr Val Lys Pro
           675                 680                 685
   Asn His Arg Glu Leu Val Asn Phe Leu Thr Glu Ile Pro Gly Leu Ser
       690                 695                 700
   Glu Ala Ser Lys Leu Glu Arg Leu Thr Ser Leu Leu Glu Ser Asn Ser
   705                 710                 715                 720
   Ser Cys Gln Asp Leu Gly Lys Leu Ile Cys Leu Glu Leu Lys Asp Cys
                   725                 730                 735
   Ser Cys Leu Gln Ser Leu Pro Asn Met Ala Asn Leu Asp Leu Asn Val
                   740                 745                 750
   Leu Asp Leu Ser Gly Cys Ser Ser Leu Asn Ser Ile Gln Gly Phe Pro
           755                 760                 765
   Arg Phe Leu Lys Gln Leu Tyr Leu Gly Gly Thr Ala Ile Arg Glu Val
       770                 775                 780
   Pro Gln Leu Pro Gln Ser Leu Glu Ile Leu Asn Ala His Gly Ser Cys
   785                 790                 795                 800
   Leu Arg Ser Leu Pro Asn Met Ala Asn Leu Glu Phe Leu Lys Val Leu
                   805                 810                 815
   Asp Leu Ser Gly Cys Ser Glu Leu Glu Thr Ile Gln Gly Phe Pro Arg
               820                 825                 830
   Asn Leu Lys Glu Leu Tyr Phe Ala Gly Thr Thr Leu Arg Glu Val Pro
           835                 840                 845
   Gln Leu Pro Leu Ser Leu Glu Val Leu Asn Ala His Gly Ser Asp Ser
       850                 855                 860
   Glu Lys Leu Pro Met His Tyr Lys Phe Asn Asn Phe Phe Asp Leu Ser
   865                 870                 875                 880
   Gln Gln Val Val Asn Asp Phe Phe Leu Lys Ala Leu Thr Tyr Val Lys
                   885                 890                 895
   His Ile Pro Arg Gly Tyr Thr Gln Glu Leu Ile Asn Lys Ala Pro Thr
               900                 905                 910
   Phe Ser Phe Ser Ala Pro Ser His Thr Asn Gln Asn Ala Thr Phe Asp
           915                 920                 925
   Leu Gln Pro Gly Ser Ser Val Met Thr Arg Leu Asn His Ser Trp Arg
       930                 935                 940
   Asn Thr Leu Val Gly Phe Gly Met Leu Val Glu Val Ala Phe Pro Glu
   945                 950                 955                 960
   Asp Tyr Cys Asp Ala Thr Asp Val Gly Ile Ser Cys Val Cys Arg Trp
                   965                 970                 975
   Ser Asn Lys Glu Gly Arg Ser Cys Arg Ile Glu Arg Asn Phe His Cys
               980                 985                 990
   Trp Ala Pro Gly Lys Val Val Pro Lys Val Arg Lys Asp His Thr Phe
   995 1000 1005
   Val Phe Ser Asp Val Asn Met Arg Pro Ser Thr Gly Glu Gly Asn
   1010 1015 1020
   Asp Pro Asp Ile Trp Ala Gly Leu Val Val Phe Glu Phe Phe Pro
   1025 1030 1035
   Ile Asn Gln Gln Thr Lys Cys Leu Asn Asp Arg Phe Thr Val Thr
   1040 1045 1050
   Arg Cys Gly Val Arg Val Ile Asn Val Ala Thr Gly Asn Thr Ser
   1055 1060 1065
   Leu Glu Asn Ile Ser Leu Val Leu Ser Leu Asp Pro Val Glu Val
   1070 1075 1080
   Ser Gly Tyr Glu Val Leu Arg Val Ser Tyr Asp Asp Leu Gln Glu
   1085 1090 1095
   Met Asp Lys Val Leu Phe Leu Tyr Ile Ala Ser Leu Phe Asn Asp
   1100 1105 1110
   Glu Asp Val Asp Phe Val Ala Pro Leu Ile Ala Gly Ile Asp Leu
   1115 1120 1125
   Asp Val Ser Ser Gly Leu Lys Val Leu Ala Asp Val Ser Leu Ile
   1130 1135 1140
   Ser Val Ser Ser Asn Gly Glu Ile Val Met His Ser Leu Gln Arg
   1145 1150 1155
   Gln Met Gly Lys Glu Ile Leu His Gly Gln Ser Met Leu Leu Ser
   1160 1165 1170
   Asp Cys Glu Ser Ser Met Thr Glu Asn Leu Ser Asp Val Pro Lys
   1175 1180 1185
   Lys Glu Lys Lys His Arg Glu Ser Lys Val Lys Lys Val Val Ser
   1190 1195 1200
   Ile Pro Ala Ile Asp Glu Gly Asp Leu Trp Thr Trp Arg Lys Tyr
   1205 1210 1215
   Gly Gln  Lys Asp Ile Leu Gly  Ser Arg Phe Pro Arg  Gly Tyr Tyr
       1220                 1225                 1230
   Arg Cys  Ala Tyr Lys Phe Thr  His Gly Cys Lys Ala  Thr Lys Gln
       1235                 1240                 1245
   Val Gln  Arg Ser Glu Thr Asp  Ser Asn Met Leu Ala  Ile Thr Tyr
       1250                 1255                 1260
   Leu Ser  Glu His Asn His Pro  Arg Pro Thr Lys Arg  Lys Ala Leu
       1265                 1270                 1275
   Ala Asp  Ser Thr Arg Ser Thr  Ser Ser Ser Ile Cys  Ser Ala Ile
       1280                 1285                 1290
   Thr Thr  Ser Ala Ser Ser Arg  Val Phe Gln Asn Lys  Asp Glu Pro
       1295                 1300                 1305
   Asn Gln  Pro His Leu Pro Ser  Ser Ser Thr Pro Pro  Arg Asn Ala
       1310                 1315                 1320
   Ala Val  Leu Phe Lys Met Thr  Asp Met Glu Glu Phe  Gln Asp Asn
       1325                 1330                 1335
   Met Glu  Val Asp Asn Asp Val  Val Asp Thr Arg Thr  Leu Ala Leu
       1340                 1345                 1350
   Phe Pro  Glu Phe Gln His Gln  Pro Glu Glu Glu Asp  Pro Trp Ser
       1355                 1360                 1365
   Thr Phe  Phe Asp Asp Tyr Asn  Phe Tyr Phe
       1370                 1375
<210> 9
   <211> 3654
   <212> DNA
   <213> Arabidopsis thaliana Nd-1
<220>
   <221> CDS
   <222> (1)..(3651)
   <223>
<400> 9
   atg gag aca tcg tct att tcc act gtg gaa gac aag cca ccg cag cat        48
   Met Glu Thr Ser Ser Ile Ser Thr Val Glu Asp Lys Pro Pro Gln His
   1               5                   10                  15
   cag gtg ttc atc aat ttc cgt ggg gca gat ttg cgc cgg aga ttc gtc        96
   Gln Val Phe Ile Asn Phe Arg Gly Ala Asp Leu Arg Arg Arg Phe Val
               20                  25                  30
   agc cat ctc gtc acg gcc ttg aaa ttg aac aac atc aac gtc ttt atc      144
   Ser His Leu Val Thr Ala Leu Lys Leu Asn Asn Ile Asn Val Phe Ile
           35                  40                  45
   gac gac tat gaa gac aga ggt caa cct cta gat gta ctg ctg aag agg      192
   Asp Asp Tyr Glu Asp Arg Gly Gln Pro Leu Asp Val Leu Leu Lys Arg
       50                  55                  60
   ata gag gag tcc aaa atc gtt ttg gct atc ttc tcc ggc aac tac acc      240
   Ile Glu Glu Ser Lys Ile Val Leu Ala Ile Phe Ser Gly Asn Tyr Thr
   65                  70                  75                  80
   gag tca gtc tgg tgc gtg aga gag ctg gag aag atc aaa gat tgt acg      288
   Glu Ser Val Trp Cys Val Arg Glu Leu Glu Lys Ile Lys Asp Cys Thr
                   85                  90                  95
   gat gaa gga aca ctc gtt gcg att cca atc ttc tac aag ctg gag cca      336
   Asp Glu Gly Thr Leu Val Ala Ile Pro Ile Phe Tyr Lys Leu Glu Pro
               100                 105                 110
   tcc acc gtt aga gat ttg aaa gga aag ttc ggt gat aga ttt agg agt      384
   Ser Thr Val Arg Asp Leu Lys Gly Lys Phe Gly Asp Arg Phe Arg Ser
           115                 120                 125
   atg gct aag ggt gat gag agg aaa aaa aaa tgg aag gaa gct ttt aac      432
   Met Ala Lys Gly Asp Glu Arg Lys Lys Lys Trp Lys Glu Ala Phe Asn
       130                 135                 140
   ttg att cct aac att atg ggc atc acc att gac aag aaa agt gtg gag      480
   Leu Ile Pro Asn Ile Met Gly Ile Thr Ile Asp Lys Lys Ser Val Glu
   145                 150                 155                 160
   agt gag aaa gtc aat gaa att gtg aag gcc gtg aag aca gcg ttg acc      528
   Ser Glu Lys Val Asn Glu Ile Val Lys Ala Val Lys Thr Ala Leu Thr
                   165                 170                 175
   gga ata cca ccg gag gga agt cac aat gcc gtc gtg ggt gct tta ggt      576
   Gly Ile Pro Pro Glu Gly Ser His Asn Ala Val Val Gly Ala Leu Gly
               180                 185                 190
   aat agc aac gct gga act tcc tca gga gac aaa aag cat gag act ttt      624
   Asn Ser Asn Ala Gly Thr Ser Ser Gly Asp Lys Lys His Glu Thr Phe
           195                 200                 205
   gga aac gaa caa cgg tta aaa gat ttg gaa gag aag ttg gat cgt gat      672
   Gly Asn Glu Gln Arg Leu Lys Asp Leu Glu Glu Lys Leu Asp Arg Asp
       210                 215                 220
   aaa tac aag gga act cgt atc att gga gtt gtc ggg atg ccc gga att      720
   Lys Tyr Lys Gly Thr Arg Ile Ile Gly Val Val Gly Met Pro Gly Ile
   225                 230                 235                 240
   ggt aaa acc aca ctc ctg aaa gag cta tat aag acg tgg cag ggc aag      768
   Gly Lys Thr Thr Leu Leu Lys Glu Leu Tyr Lys Thr Trp Gln Gly Lys
                   245                 250                 255
   ttt tcg agg cat gcg cta atc gat caa atc cgt gta aag tcc aag cac      816
   Phe Ser Arg His Ala Leu Ile Asp Gln Ile Arg Val Lys Ser Lys His
               260                 265                 270
   ttg gag ttg gat cgc ttg cct caa atg gtc tta ggc gag tta tcg aag       864
   Leu Glu Leu Asp Arg Leu Pro Gln Met Val Leu Gly Glu Leu Ser Lys
           275                 280                 285
   tta aac aat cct cac gta gac aat ctc aaa gat cca tac agt caa ctg       912
   Leu Asn Asn Pro His Val Asp Asn Leu Lys Asp Pro Tyr Ser Gln Leu
       290                 295                 300
   cat gaa cgc aaa gtg ctt gtt gta ctt gat gat gtt agt aaa agg gaa       960
   His Glu Arg Lys Val Leu Val Val Leu Asp Asp Val Ser Lys Arg Glu
   305                 310                 315                 320
   caa ata gat gct ctt cgt gag ata cta gac tgg att aag gag ggt aag      1008
   Gln Ile Asp Ala Leu Arg Glu Ile Leu Asp Trp Ile Lys Glu Gly Lys
                   325                 330                 335
   gag gga agc agg gtt gtc att gca aca agc gac atg tcc tta aca aat      1056
   Glu Gly Ser Arg Val Val Ile Ala Thr Ser Asp Met Ser Leu Thr Asn
               340                 345                 350
   ggt ttg gtt gat gat act tac atg gtt caa aat ttg aac cac aga gat      1104
   Gly Leu Val Asp Asp Thr Tyr Met Val Gln Asn Leu Asn His Arg Asp
           355                 360                 365
   agc tta caa cta ttt cac tat cat gcc ttt att gat gat caa gcc aat      1152
   Ser Leu Gln Leu Phe His Tyr His Ala Phe Ile Asp Asp Gln Ala Asn
       370                 375                 380
   cct caa aag aaa gat ttc atg aag ctg tca gaa ggg ttt gta cat tac      1200
   Pro Gln Lys Lys Asp Phe Met Lys Leu Ser Glu Gly Phe Val His Tyr
   385                 390                 395                 400
   gcc aga ggc cat cca cta gcc ctc aaa gta ttg ggt gga gag ctt aat      1248
   Ala Arg Gly His Pro Leu Ala Leu Lys Val Leu Gly Gly Glu Leu Asn
                   405                 410                 415
   aag aaa agt atg gat cat tgg aat tcg aaa atg aag aaa ctt gca cag      1296
   Lys Lys Ser Met Asp His Trp Asn Ser Lys Met Lys Lys Leu Ala Gln
               420                 425                 430
   agc ccc agc cct aat att gtc agt gtc ttc caa gtg agt tat gat gaa      1344
   Ser Pro Ser Pro Asn Ile Val Ser Val Phe Gln Val Ser Tyr Asp Glu
           435                 440                 445
   ttg act aca gcg cag aaa gat gca ttt ctc gac ata gcc tgt ttc aga      1392
   Leu Thr Thr Ala Gln Lys Asp Ala Phe Leu Asp Ile Ala Cys Phe Arg
       450                 455                 460
   tca cag gac aag gat tat gta gaa agt cta ctg gct tca tct gac ctt      1440
   Ser Gln Asp Lys Asp Tyr Val Glu Ser Leu Leu Ala Ser Ser Asp Leu
   465                 470                 475                 480
   ggt tcg gct gaa gca atg agt gca gtc aaa tct ctc acg gat aag ttc      1488
   Gly Ser Ala Glu Ala Met Ser Ala Val Lys Ser Leu Thr Asp Lys Phe
                   485                 490                 495
   ctg att aat act tgt gat ggc cga gtg gag atg cat gat cta tta tat      1536
   Leu Ile Asn Thr Cys Asp Gly Arg Val Glu Met His Asp Leu Leu Tyr
               500                 505                 510
   aaa ttt tca agg gaa gtt gat ctg aag gca tct aat cag gat ggt agc      1584
   Lys Phe Ser Arg Glu Val Asp Leu Lys Ala Ser Asn Gln Asp Gly Ser
           515                 520                 525
   aga caa cgg agg ctg tgg ctc cat caa cac ata atc aag gga ggc ata      1632
   Arg Gln Arg Arg Leu Trp Leu His Gln His Ile Ile Lys Gly Gly Ile
       530                 535                 540
   atc aat gta ttg caa aat aaa atg aaa gct gcc aat gtt aga ggt att      1680
   Ile Asn Val Leu Gln Asn Lys Met Lys Ala Ala Asn Val Arg Gly Ile
   545                 550                 555                 560
   ttc cta gac ttg tct gaa gtg gaa gac gaa acg agc tta gac cgc gac      1728
   Phe Leu Asp Leu Ser Glu Val Glu Asp Glu Thr Ser Leu Asp Arg Asp
                   565                 570                 575
   cac ttc ata aat atg gga aat ctc cgg tat ctc aag ttc tac aat tcc      1776
   His Phe Ile Asn Met Gly Asn Leu Arg Tyr Leu Lys Phe Tyr Asn Ser
               580                 585                 590
   cat tgt cca cag gaa tgt aaa acc aac aat aaa atc aac atc cct gat      1824
   His Cys Pro Gln Glu Cys Lys Thr Asn Asn Lys Ile Asn Ile Pro Asp
           595                 600                 605
   aaa ctt aag cta cca ttg aaa gaa gtt cga tgc ctc cac tgg ctg aaa      1872
   Lys Leu Lys Leu Pro Leu Lys Glu Val Arg Cys Leu His Trp Leu Lys
       610                 615                 620
   ttc cca ttg gag aca ctt cca aac gat ttc aac cca att aat ctt gtc      1920
   Phe Pro Leu Glu Thr Leu Pro Asn Asp Phe Asn Pro Ile Asn Leu Val
   625                 630                 635                 640
   gac ctt aag ctg ccc tac agt gaa atg gaa caa ctt tgg gag ggt gac      1968
   Asp Leu Lys Leu Pro Tyr Ser Glu Met Glu Gln Leu Trp Glu Gly Asp
                   645                 650                 655
   aag gac aca cca tgc tta agg tgg gtc gat ctc aat cac tca agt aag      2016
   Lys Asp Thr Pro Cys Leu Arg Trp Val Asp Leu Asn His Ser Ser Lys
               660                 665                 670
   ttg tgc agc ttg tca ggg tta tca aaa gct gaa aag ctt caa agg ttg      2064
   Leu Cys Ser Leu Ser Gly Leu Ser Lys Ala Glu Lys Leu Gln Arg Leu
           675                 680                 685
   aac ctt gaa ggt tgc aca aca ctg aaa gcg ttt cca cat gat atg aaa      2112
   Asn Leu Glu Gly Cys Thr Thr Leu Lys Ala Phe Pro His Asp Met Lys
       690                 695                 700
   aaa atg aaa atg ctt gct ttc ttg aat ctg aag gga tgt aca agt ctc      2160
   Lys Met Lys Met Leu Ala Phe Leu Asn Leu Lys Gly Cys Thr Ser Leu
   705                 710                 715                 720
   gaa tct ctt cca gag atg aat ttg att tct ctg aaa aca ctt act ctc      2208
   Glu Ser Leu Pro Glu Met Asn Leu Ile Ser Leu Lys Thr Leu Thr Leu
                   725                 730                 735
   agc ggc tgc tca act ttt aag gaa ttt ccg ttg att tca gat aat ata      2256
   Ser Gly Cys Ser Thr Phe Lys Glu Phe Pro Leu Ile Ser Asp Asn Ile
               740                 745                 750
   gaa act cta tac tta gat ggc aca gca ata agt cag ctt cct atg aac     2304
   Glu Thr Leu Tyr Leu Asp Gly Thr Ala Ile Ser Gln Leu Pro Met Asn
           755                 760                 765
   atg gag aag ctt cag aga ctt gtt gta ttg aat atg aaa gac tgc aaa     2352
   Met Glu Lys Leu Gln Arg Leu Val Val Leu Asn Met Lys Asp Cys Lys
       770                 775                 780
   atg cta gag gaa att cca ggc cgt gtt ggt gag ctg aaa gct ctt caa     2400
   Met Leu Glu Glu Ile Pro Gly Arg Val Gly Glu Leu Lys Ala Leu Gln
   785                 790                 795                 800
   gaa ctg ata ctc tct gat tgt tta aat ctc aag att ttc cca gaa atc     2448
   Glu Leu Ile Leu Ser Asp Cys Leu Asn Leu Lys Ile Phe Pro Glu Ile
                   805                 810                 815
   aac att agc ttt tta aac att tta ctt ttg gat ggg aca gcc att gaa     2496
   Asn Ile Ser Phe Leu Asn Ile Leu Leu Leu Asp Gly Thr Ala Ile Glu
               820                 825                 830
   gtg atg cca caa tta ccc tca gtg cag tat ttg tgc tta agc aga aat     2544
   Val Met Pro Gln Leu Pro Ser Val Gln Tyr Leu Cys Leu Ser Arg Asn
           835                 840                 845
   gct aag atc agc tgc ctt cct gtt gga atc agt cag ctt tct caa ctt     2592
   Ala Lys Ile Ser Cys Leu Pro Val Gly Ile Ser Gln Leu Ser Gln Leu
       850                 855                 860
   aaa tgg ctg gac ctg aag tat tgt acg agt ctt aca tca gtt cca gag     2640
   Lys Trp Leu Asp Leu Lys Tyr Cys Thr Ser Leu Thr Ser Val Pro Glu
   865                 870                 875                 880
   ttt cca cca aat ctt cag tgc tta gat gca cac ggc tgt agt tcg ctg     2688
   Phe Pro Pro Asn Leu Gln Cys Leu Asp Ala His Gly Cys Ser Ser Leu
                   885                 890                 895
   aag aca gtt tca aag cct ttg gcc cgt atc atg cca act gaa cag aat     2736
   Lys Thr Val Ser Lys Pro Leu Ala Arg Ile Met Pro Thr Glu Gln Asn
               900                 905                 910
   cat tcc aca ttc att ttc acc aac tgt gag aac ctg gaa caa gct gca     2784
   His Ser Thr Phe Ile Phe Thr Asn Cys Glu Asn Leu Glu Gln Ala Ala
           915                 920                 925
   aag gag gaa atc aca tcg tat gct caa agg aaa tgc cag ctg tta tca     2832
   Lys Glu Glu Ile Thr Ser Tyr Ala Gln Arg Lys Cys Gln Leu Leu Ser
       930                 935                 940
   tat gct cgg aaa cgc tac aat ggg ggt ctt gtt tca gaa tcc ttg ttc     2880
   Tyr Ala Arg Lys Arg Tyr Asn Gly Gly Leu Val Ser Glu Ser Leu Phe
   945                 950                 955                 960
   agc act tgc ttt cct gga tgt gaa gtg cct tct tgg ttt tgt cat gaa     2928
   Ser Thr Cys Phe Pro Gly Cys Glu Val Pro Ser Trp Phe Cys His Glu
                   965                 970                 975
   aca gtt gga tct gag tta gaa gta aaa ctc ctc cca cat tgg cat gac     2976
   Thr Val Gly Ser Glu Leu Glu Val Lys Leu Leu Pro His Trp His Asp
               980                 985                 990
   aag aag ctt gcc ggg att gct cta  tgt gct gta gtc tca  tgt ctt gac   3024
   Lys Lys Leu Ala Gly Ile Ala Leu  Cys Ala Val Val Ser  Cys Leu Asp
             995                 1000                1005
   ccc caa  gat caa gtc agc cgc  ttg tca gtg acc tgc  acc ttt aaa      3069
   Pro Gln  Asp Gln Val Ser Arg  Leu Ser Val Thr Cys  Thr Phe Lys
       1010                 1015                 1020
   gta aaa  gat gaa gac aag tct  tgg gtc cca tat act  tgt cca gta      3114
   Val Lys  Asp Glu Asp Lys Ser  Trp Val Pro Tyr Thr  Cys Pro Val
       1025                 1030                 1035
   gga agt  tgg acc aga cat gga  ggc ggg aaa gat aaa  att gag cta      3159
   Gly Ser  Trp Thr Arg His Gly  Gly Gly Lys Asp Lys  Ile Glu Leu
       1040                 1045                 1050
   gac cat  gtc ttc att gga tac  acc agt tgc cca cat  act ata aaa      3204
   Asp His  Val Phe Ile Gly Tyr  Thr Ser Cys Pro His  Thr Ile Lys
       1055                 1060                 1065
   tgt cat  gaa gaa ggt aac tca  gat gaa tgc aat cct  acc gaa gcc      3249
   Cys His  Glu Glu Gly Asn Ser  Asp Glu Cys Asn Pro  Thr Glu Ala
       1070                 1075                 1080
   tct ctt  aaa ttt act gtg aca  gga ggt aca agt gag  aat gga aaa      3294
   Ser Leu  Lys Phe Thr Val Thr  Gly Gly Thr Ser Glu  Asn Gly Lys
       1085                 1090                 1095
   tac aag  gtg ttg aag tgt ggt  tta agt ttg gtg tat  gct aag gat      3339
   Tyr Lys  Val Leu Lys Cys Gly  Leu Ser Leu Val Tyr  Ala Lys Asp
       1100                 1105                 1110
   aaa gac  aaa aat agt gct ctt  gaa aca aag tac gat  atg ctt att      3384
   Lys Asp  Lys Asn Ser Ala Leu  Glu Thr Lys Tyr Asp  Met Leu Ile
       1115                 1120                 1125
   gga aag  agc ttt caa gaa act  tca gaa ggg gtt gat  gga agg gta      3429
   Gly Lys  Ser Phe Gln Glu Thr  Ser Glu Gly Val Asp  Gly Arg Val
       1130                 1135                 1140
   aag aaa  acg aaa gga aag tac  gtt atg cct gtt gaa  aag aac ttt      3474
   Lys Lys  Thr Lys Gly Lys Tyr  Val Met Pro Val Glu  Lys Asn Phe
       1145                 1150                 1155
   caa gaa  act aca gaa ggg gtt  gat gga aga gta aag  aaa aag aaa      3519
   Gln Glu  Thr Thr Glu Gly Val  Asp Gly Arg Val Lys  Lys Lys Lys
       1160                 1165                 1170
   aaa aca  aga atg gat aat gga  cgg cca aag aag aag  cag aga tca      3564
   Lys Thr  Arg Met Asp Asn Gly  Arg Pro Lys Lys Lys  Gln Arg Ser
       1175                 1180                 1185
   gga aga  gat gat aat caa act  agg atg caa gtg gaa  ctc caa gaa      3609
   Gly Arg  Asp Asp Asn Gln Thr  Arg Met Gln Val Glu  Leu Gln Glu
       1190                 1195                 1200
   gga aat  ata aat tca gtg atc  atg cac acg gtt aag  aat ttc tga      3654
   Gly Asn  Ile Asn Ser Val Ile  Met His Thr Val Lys  Asn Phe
       1205                 1210                 1215
<210> 10
   <211> 1217
   <212> PRT
   <213> Arabidopsis thaliana Nd-1
<400> 10
   Met Glu Thr Ser Ser Ile Ser Thr Val Glu Asp Lys Pro Pro Gln His
   1               5                   10                  15
   Gln Val Phe Ile Asn Phe Arg Gly Ala Asp Leu Arg Arg Arg Phe Val
               20                  25                  30
   Ser His Leu Val Thr Ala Leu Lys Leu Asn Asn Ile Asn Val Phe Ile
           35                  40                  45
   Asp Asp Tyr Glu Asp Arg Gly Gln Pro Leu Asp Val Leu Leu Lys Arg
       50                  55                  60
   Ile Glu Glu Ser Lys Ile Val Leu Ala Ile Phe Ser Gly Asn Tyr Thr
   65                  70                  75                  80
   Glu Ser Val Trp Cys Val Arg Glu Leu Glu Lys Ile Lys Asp Cys Thr
                   85                  90                  95
   Asp Glu Gly Thr Leu Val Ala Ile Pro Ile Phe Tyr Lys Leu Glu Pro
               100                 105                 110
   Ser Thr Val Arg Asp Leu Lys Gly Lys Phe Gly Asp Arg Phe Arg Ser
           115                 120                 125
   Met Ala Lys Gly Asp Glu Arg Lys Lys Lys Trp Lys Glu Ala Phe Asn
       130                 135                 140
   Leu Ile Pro Asn Ile Met Gly Ile Thr Ile Asp Lys Lys Ser Val Glu
   145                 150                 155                 160
   Ser Glu Lys Val Asn Glu Ile Val Lys Ala Val Lys Thr Ala Leu Thr
                   165                 170                 175
   Gly Ile Pro Pro Glu Gly Ser His Asn Ala Val Val Gly Ala Leu Gly
               180                 185                 190
   Asn Ser Asn Ala Gly Thr Ser Ser Gly Asp Lys Lys His Glu Thr Phe
           195                 200                 205
   Gly Asn Glu Gln Arg Leu Lys Asp Leu Glu Glu Lys Leu Asp Arg Asp
       210                 215                 220
   Lys Tyr Lys Gly Thr Arg Ile Ile Gly Val Val Gly Met Pro Gly Ile
   225                 230                 235                 240
   Gly Lys Thr Thr Leu Leu Lys Glu Leu Tyr Lys Thr Trp Gln Gly Lys
                   245                 250                 255
   Phe Ser Arg His Ala Leu Ile Asp Gln Ile Arg Val Lys Ser Lys His
               260                 265                 270
   Leu Glu Leu Asp Arg Leu Pro Gln Met Val Leu Gly Glu Leu Ser Lys
           275                 280                 285
   Leu Asn Asn Pro His Val Asp Asn Leu Lys Asp Pro Tyr Ser Gln Leu
       290                 295                 300
   His Glu Arg Lys Val Leu Val Val Leu Asp Asp Val Ser Lys Arg Glu
   305                 310                 315                 320
   Gln Ile Asp Ala Leu Arg Glu Ile Leu Asp Trp Ile Lys Glu Gly Lys
                   325                 330                 335
   Glu Gly Ser Arg Val Val Ile Ala Thr Ser Asp Met Ser Leu Thr Asn
               340                 345                 350
   Gly Leu Val Asp Asp Thr Tyr Met Val Gln Asn Leu Asn His Arg Asp
           355                 360                 365
   Ser Leu Gln Leu Phe His Tyr His Ala Phe Ile Asp Asp Gln Ala Asn
       370                 375                 380
   Pro Gln Lys Lys Asp Phe Met Lys Leu Ser Glu Gly Phe Val His Tyr
   385                 390                 395                 400
   Ala Arg Gly His Pro Leu Ala Leu Lys Val Leu Gly Gly Glu Leu Asn
                   405                 410                 415
   Lys Lys Ser Met Asp His Trp Asn Ser Lys Met Lys Lys Leu Ala Gln
               420                 425                 430
   Ser Pro Ser Pro Asn Ile Val Ser Val Phe Gln Val Ser Tyr Asp Glu
           435                 440                 445
   Leu Thr Thr Ala Gln Lys Asp Ala Phe Leu Asp Ile Ala Cys Phe Arg
       450                 455                 460
   Ser Gln Asp Lys Asp Tyr Val Glu Ser Leu Leu Ala Ser Ser Asp Leu
   465 470 475 480
   Gly Ser Ala Glu Ala Met Ser Ala Val Lys Ser Leu Thr Asp Lys Phe
   485 490 495
   Leu Ile Asn Thr Cys Asp Gly Arg Val Glu Met His Asp Leu Leu Tyr
   500 505 510
   Lys Phe Ser Arg Glu Val Asp Leu Lys Ala Ser Asn Gln Asp Gly Ser
   515 520 525
   Arg Gln Arg Arg Leu Trp Leu His Gln His Ile Ile Lys Gly Gly Ile
   530 535 540
   Ile Asn Val Leu Gln Asn Lys Met Lys Ala Ala Asn Val Arg Gly Ile
   545 550 555 560
   Phe Leu Asp Leu Ser Glu Val Glu Asp Glu Thr Ser Leu Asp Arg Asp
   565 570 575
   His Phe Ile Asn Met Gly Asn Leu Arg Tyr Leu Lys Phe Tyr Asn Ser
   580 585 590
   His Cys Pro Gln Glu Cys Lys Thr Asn Asn Lys Ile Asn Ile Pro Asp
   595 600 605
   Lys Leu Lys Leu Pro Leu Lys Glu Val Arg Cys Leu His Trp Leu Lys
   610 615 620
   Phe Pro Leu Glu Thr Leu Pro Asn Asp Phe Asn Pro Ile Asn Leu Val
   625 630 635 640
   Asp Leu Lys Leu Pro Tyr Ser Glu Met Glu Gln Leu Trp Glu Gly Asp
   645 650 655
   Lys Asp Thr Pro Cys Leu Arg Trp Val Asp Leu Asn His Ser Ser Lys
   660 665 670
   Leu Cys Ser Leu Ser Gly Leu Ser Lys Ala Glu Lys Leu Gln Arg Leu
   675 680 685
   Asn Leu Glu Gly Cys Thr Thr Leu Lys Ala Phe Pro His Asp Met Lys
   690 695 700
   Lys Met Lys Met Leu Ala Phe Leu Asn Leu Lys Gly Cys Thr Ser Leu
   705 710 715 720
   Glu Ser Leu Pro Glu Met Asn Leu Ile Ser Leu Lys Thr Leu Thr Leu
   725 730 735
   Ser Gly Cys Ser Thr Phe Lys Glu Phe Pro Leu Ile Ser Asp Asn Ile
   740 745 750
   Glu Thr Leu Tyr Leu Asp Gly Thr Ala Ile Ser Gln Leu Pro Met Asn
   755 760 765
   Met Glu Lys Leu Gln Arg Leu Val Val Leu Asn Met Lys Asp Cys Lys
   770 775 780
   Met Leu Glu Glu Ile Pro Gly Arg Val Gly Glu Leu Lys Ala Leu Gln
   785 790 795 800
   Glu Leu Ile Leu Ser Asp Cys Leu Asn Leu Lys Ile Phe Pro Glu Ile
   805 810 815
   Asn Ile Ser Phe Leu Asn Ile Leu Leu Leu Asp Gly Thr Ala Ile Glu
   820 825 830
   Val Met Pro Gln Leu Pro Ser Val Gln Tyr Leu Cys Leu Ser Arg Asn
   835 840 845
   Ala Lys Ile Ser Cys Leu Pro Val Gly Ile Ser Gln Leu Ser Gln Leu
   850 855 860
   Lys Trp Leu Asp Leu Lys Tyr Cys Thr Ser Leu Thr Ser Val Pro Glu
   865 870 875 880
   Phe Pro Pro Asn Leu Gln Cys Leu Asp Ala His Gly Cys Ser Ser Leu
   885 890 895
   Lys Thr Val Ser Lys Pro Leu Ala Arg Ile Met Pro Thr Glu Gln Asn
   900 905 910
   His Ser Thr Phe Ile Phe Thr Asn Cys Glu Asn Leu Glu Gln Ala Ala
   915 920 925
   Lys Glu Glu Ile Thr Ser Tyr Ala Gln Arg Lys Cys Gln Leu Leu Ser
   930 935 940
   Tyr Ala Arg Lys Arg Tyr Asn Gly Gly Leu Val Ser Glu Ser Leu Phe
   945 950 955 960
   Ser Thr Cys Phe Pro Gly Cys Glu Val Pro Ser Trp Phe Cys His Glu
                   965                 970                 975
   Thr Val Gly Ser Glu Leu Glu Val Lys Leu Leu Pro His Trp His Asp
               980                 985                 990
   Lys Lys Leu Ala Gly Ile Ala Leu  Cys Ala Val Val Ser  Cys Leu Asp
           995                 1000                 1005
   Pro Gln  Asp Gln Val Ser Arg  Leu Ser Val Thr Cys  Thr Phe Lys
       1010                 1015                 1020
   Val Lys  Asp Glu Asp Lys Ser  Trp Val Pro Tyr Thr  Cys Pro Val
       1025                 1030                 1035
   Gly Ser  Trp Thr Arg His Gly  Gly Gly Lys Asp Lys  Ile Glu Leu
       1040                 1045                 1050
   Asp His  Val Phe Ile Gly Tyr  Thr Ser Cys Pro His  Thr Ile Lys
       1055                 1060                 1065
   Cys His  Glu Glu Gly Asn Ser  Asp Glu Cys Asn Pro  Thr Glu Ala
       1070                 1075                 1080
   Ser Leu  Lys Phe Thr Val Thr  Gly Gly Thr Ser Glu  Asn Gly Lys
       1085                 1090                 1095
   Tyr Lys  Val Leu Lys Cys Gly  Leu Ser Leu Val Tyr  Ala Lys Asp
       1100                 1105                 1110
   Lys Asp  Lys Asn Ser Ala Leu  Glu Thr Lys Tyr Asp  Met Leu Ile
       1115                 1120                 1125
   Gly Lys  Ser Phe Gln Glu Thr  Ser Glu Gly Val Asp  Gly Arg Val
       1130                 1135                 1140
   Lys Lys  Thr Lys Gly Lys Tyr  Val Met Pro Val Glu  Lys Asn Phe
       1145                 1150                 1155
   Gln Glu  Thr Thr Glu Gly Val  Asp Gly Arg Val Lys  Lys Lys Lys
       1160                 1165                 1170
   Lys Thr  Arg Met Asp Asn Gly  Arg Pro Lys Lys Lys  Gln Arg Ser
       1175                 1180                 1185
   Gly Arg Asp Asp Asn Gln Thr Arg Met Gln Val Glu Leu Gln Glu
   1190 1195 1200
   Gly Asn Ile Asn Ser Val Ile Met His Thr Val Lys Asn Phe
   1205 1210 1215
<210> 11
   <211> 4137
   <212> DNA
   <213> Arabidopsis thaliana Nd-1
<220>
   <221> CDS
   <222> (1)..(4134)
   <223>
<400> 11
   atg acc aat tgt gaa aag gat gag gaa ttc gtg tgc atc agc tgc gta        48
   Met Thr Asn Cys Glu Lys Asp Glu Glu Phe Val Cys Ile Ser Cys Val
   1               5                   10                  15
   gaa gag gta cgg tac tct ttc gtg agc cac ctc tct gaa gct ctc cgt        96
   Glu Glu Val Arg Tyr Ser Phe Val Ser His Leu Ser Glu Ala Leu Arg
               20                  25                  30
   cga aaa ggc ata aat aat gtg gtc gta gat gta gat atc gat gat ctg       144
   Arg Lys Gly Ile Asn Asn Val Val Val Asp Val Asp Ile Asp Asp Leu
           35                  40                  45
   ctt ttc aag gag tct cag gca aag atc gag aaa gct ggg gtt tct gtg       192
   Leu Phe Lys Glu Ser Gln Ala Lys Ile Glu Lys Ala Gly Val Ser Val
       50                  55                  60
   atg gtt tta ccc gga aac tgt gac cct tcc gag gta tgg ctt gac aag       240
   Met Val Leu Pro Gly Asn Cys Asp Pro Ser Glu Val Trp Leu Asp Lys
   65                  70                  75                  80
   ttc gcc aag gtt ctc gag tgc caa agg aac aac aag gac cag gcg gtg       288
   Phe Ala Lys Val Leu Glu Cys Gln Arg Asn Asn Lys Asp Gln Ala Val
                   85                  90                  95
   gtt tca gtg ttg tac ggt gac agt cta tta cgg gac caa tgg ctt agc       336
   Val Ser Val Leu Tyr Gly Asp Ser Leu Leu Arg Asp Gln Trp Leu Ser
               100                 105                 110
   gag ctg gat ttc aga ggc tta tca cga att cac caa tcc agg aag gaa       384
   Glu Leu Asp Phe Arg Gly Leu Ser Arg Ile His Gln Ser Arg Lys Glu
           115                 120                 125
   tgt agt gac tct ata ctt gta gaa gag att gtg aga gat gtg tac gag       432
   Cys Ser Asp Ser Ile Leu Val Glu Glu Ile Val Arg Asp Val Tyr Glu
       130                 135                 140
   acg cac ttt tat gtt gga cga att gga atc tat tcg aag ctg ctg gag       480
   Thr His Phe Tyr Val Gly Arg Ile Gly Ile Tyr Ser Lys Leu Leu Glu
   145                 150                 155                 160
   att gaa aac atg gtt aac aag caa ccg ata ggc atc cgt tgt gtt gga       528
   Ile Glu Asn Met Val Asn Lys Gln Pro Ile Gly Ile Arg Cys Val Gly
                   165                 170                 175
   att tgg ggt atg cct ggc ata gga aag aca aca ctt gct aaa gca gtc      576
   Ile Trp Gly Met Pro Gly Ile Gly Lys Thr Thr Leu Ala Lys Ala Val
               180                 185                 190
   ttt gac caa atg tct agc gcc ttt gat gct tct tgt ttt atc gaa gac      624
   Phe Asp Gln Met Ser Ser Ala Phe Asp Ala Ser Cys Phe Ile Glu Asp
           195                 200                 205
   tat gac aaa tca att cat gag aag ggt ctt tat tgt ttg ctg gag gaa      672
   Tyr Asp Lys Ser Ile His Glu Lys Gly Leu Tyr Cys Leu Leu Glu Glu
       210                 215                 220
   caa ctt ttg ccg ggt aat gat gca acc att atg aaa ctg agc tcg ctc      720
   Gln Leu Leu Pro Gly Asn Asp Ala Thr Ile Met Lys Leu Ser Ser Leu
   225                 230                 235                 240
   aga gac aga ttg aac agt aag aga gtt ctt gtt gtt ctc gat gac gtg      768
   Arg Asp Arg Leu Asn Ser Lys Arg Val Leu Val Val Leu Asp Asp Val
                   245                 250                 255
   tgc aat gct ctg gtt gca gag tct ttt ctc gag ggg ttt gac tgg cta      816
   Cys Asn Ala Leu Val Ala Glu Ser Phe Leu Glu Gly Phe Asp Trp Leu
               260                 265                 270
   gga ccc gga agc ctg atc atc ata acc tct aga gat aaa caa gtg ttt      864
   Gly Pro Gly Ser Leu Ile Ile Ile Thr Ser Arg Asp Lys Gln Val Phe
           275                 280                 285
   cgc ctt tgc gga atc aat caa ata tat gag gtc cag ggt tta aat gag      912
   Arg Leu Cys Gly Ile Asn Gln Ile Tyr Glu Val Gln Gly Leu Asn Glu
       290                 295                 300
   aaa gag gct cgt caa ctt ttc ttg ctg tct gcg tct ata atg gag gat      960
   Lys Glu Ala Arg Gln Leu Phe Leu Leu Ser Ala Ser Ile Met Glu Asp
   305                 310                 315                 320
   atg gga gag cag aat ctc cat gag ttg tca gtg aga gta ata agt tac     1008
   Met Gly Glu Gln Asn Leu His Glu Leu Ser Val Arg Val Ile Ser Tyr
                   325                 330                 335
   gct aat gga aac ccg tta gct atc agt gtt tat gga aga gag ctg aaa     1056
   Ala Asn Gly Asn Pro Leu Ala Ile Ser Val Tyr Gly Arg Glu Leu Lys
               340                 345                 350
   ggt aag aaa aaa ctc tca gaa atg gag act gca ttc ctc aaa ctc aag     1104
   Gly Lys Lys Lys Leu Ser Glu Met Glu Thr Ala Phe Leu Lys Leu Lys
           355                 360                 365
   cga cgt cct cca ttt aag att gtc gat gca ttt aaa agc agc tac gac     1152
   Arg Arg Pro Pro Phe Lys Ile Val Asp Ala Phe Lys Ser Ser Tyr Asp
       370                 375                 380
   aca ctc agt gac aac gaa aag aac att ttt ttg gac ata gct tgt ttt     1200
   Thr Leu Ser Asp Asn Glu Lys Asn Ile Phe Leu Asp Ile Ala Cys Phe
   385                 390                 395                 400
   ttc cag gga gaa aat gtc aac tac gtg ata caa ctg ctt gag ggt tgt     1248
   Phe Gln Gly Glu Asn Val Asn Tyr Val Ile Gln Leu Leu Glu Gly Cys
               405                 410                 415
   ggt ttc ttt cca cat gtt gaa att gat gtc ctt gtt gac aag tgt ctg      1296
   Gly Phe Phe Pro His Val Glu Ile Asp Val Leu Val Asp Lys Cys Leu
                   420                 425                 430
   gtg act att tca gaa aac cga gtt tgg ttg cat aag ctg acc cag gat      1344
   Val Thr Ile Ser Glu Asn Arg Val Trp Leu His Lys Leu Thr Gln Asp
           435                 440                 445
   atc ggc cga gaa atc ata aat gga gaa aca gta cag atc gag agg cgc      1392
   Ile Gly Arg Glu Ile Ile Asn Gly Glu Thr Val Gln Ile Glu Arg Arg
       450                 455                 460
   aga aga ctg tgg gaa cct tgg agc atc aaa tat tta tta gaa tat aat      1440
   Arg Arg Leu Trp Glu Pro Trp Ser Ile Lys Tyr Leu Leu Glu Tyr Asn
   465                 470                 475                 480
   gaa cac aaa gca aat gga gaa cct aaa aca acc ttc aaa cgt gct cag      1488
   Glu His Lys Ala Asn Gly Glu Pro Lys Thr Thr Phe Lys Arg Ala Gln
                   485                 490                 495
   ggc tct gaa gag atc gaa ggc ctg ttt cta gac aca tca aac tta aga      1536
   Gly Ser Glu Glu Ile Glu Gly Leu Phe Leu Asp Thr Ser Asn Leu Arg
               500                 505                 510
   ttt gat ctg cag ccc tct gcc ttt aag aat atg ttg aac ctt aga ttg      1584
   Phe Asp Leu Gln Pro Ser Ala Phe Lys Asn Met Leu Asn Leu Arg Leu
           515                 520                 525
   ctc aaa att tat tgt tcc aat cct gaa gtc cat cct gta atc aat ttc      1632
   Leu Lys Ile Tyr Cys Ser Asn Pro Glu Val His Pro Val Ile Asn Phe
       530                 535                 540
   cca aca ggc tct ctg cat tct ctt cct aat gag cta aga ctc ctc cat      1680
   Pro Thr Gly Ser Leu His Ser Leu Pro Asn Glu Leu Arg Leu Leu His
   545                 550                 555                 560
   tgg gag aac tat cct ctg aaa tct ttg cct cag aat ttt gat cct agg      1728
   Trp Glu Asn Tyr Pro Leu Lys Ser Leu Pro Gln Asn Phe Asp Pro Arg
                   565                 570                 575
   cac ctt gtc gaa atc aac atg ccg tat agt caa ctt cag aaa ctt tgg      1776
   His Leu Val Glu Ile Asn Met Pro Tyr Ser Gln Leu Gln Lys Leu Trp
               580                 585                 590
   ggt gga acc aag aac ctg gag atg ttg agg acg atc agg ctt tgc cat      1824
   Gly Gly Thr Lys Asn Leu Glu Met Leu Arg Thr Ile Arg Leu Cys His
           595                 600                 605
   tcc cag cat cta gtt gat atc gat gat ctc tta aaa gct gaa aat ctt      1872
   Ser Gln His Leu Val Asp Ile Asp Asp Leu Leu Lys Ala Glu Asn Leu
       610                 615                 620
   gag gta att gat ctc caa ggt tgt acg aga ctg cag aat ttc cca gcc      1920
   Glu Val Ile Asp Leu Gln Gly Cys Thr Arg Leu Gln Asn Phe Pro Ala
   625                 630                 635                 640
   gca ggt cga ttg cta cgt cta cga gtt gta aat ctc tca ggt tgc ata      1968
   Ala Gly Arg Leu Leu Arg Leu Arg Val Val Asn Leu Ser Gly Cys Ile
               645                 650                 655
   aag att aaa agt gtc cta gaa att cca cca aat att gag aaa cta cat      2016
   Lys Ile Lys Ser Val Leu Glu Ile Pro Pro Asn Ile Glu Lys Leu His
               660                 665                 670
   cta cag gga act ggc ata tta gca tta cca gtt tcc act gtt aag cca      2064
   Leu Gln Gly Thr Gly Ile Leu Ala Leu Pro Val Ser Thr Val Lys Pro
           675                 680                 685
   aac cat aga gag ctt gtg aat ttt cta aca gaa att ccg ggt ctt tca      2112
   Asn His Arg Glu Leu Val Asn Phe Leu Thr Glu Ile Pro Gly Leu Ser
       690                 695                 700
   gag gcc tcg aaa ctt gag cgt tta aca agt ctg ctg gaa tct aac tca      2160
   Glu Ala Ser Lys Leu Glu Arg Leu Thr Ser Leu Leu Glu Ser Asn Ser
   705                 710                 715                 720
   tct tgt caa gat ctt ggg aag ctt att tgc ttg gag ctg aaa gat tgc      2208
   Ser Cys Gln Asp Leu Gly Lys Leu Ile Cys Leu Glu Leu Lys Asp Cys
                   725                 730                 735
   tct tgt ttg cag agt ctg cca aac atg gct aat tta gat ctt aat gtt      2256
   Ser Cys Leu Gln Ser Leu Pro Asn Met Ala Asn Leu Asp Leu Asn Val
               740                 745                 750
   ctt gat ctc tcg ggt tgc tca agt ctt aat tct att cag ggt ttc cct      2304
   Leu Asp Leu Ser Gly Cys Ser Ser Leu Asn Ser Ile Gln Gly Phe Pro
           755                 760                 765
   cgt ttt ctg aaa cag tta tat ctt ggt ggc act gca ata aga gaa gtg      2352
   Arg Phe Leu Lys Gln Leu Tyr Leu Gly Gly Thr Ala Ile Arg Glu Val
       770                 775                 780
   cca caa ctt cct caa agt cta gaa atc ttg aat gca cat gga tct tgt      2400
   Pro Gln Leu Pro Gln Ser Leu Glu Ile Leu Asn Ala His Gly Ser Cys
   785                 790                 795                 800
   ttg cga agt ctg cca aac atg gct aat tta gaa ttt ctc aaa gtt ctt      2448
   Leu Arg Ser Leu Pro Asn Met Ala Asn Leu Glu Phe Leu Lys Val Leu
                   805                 810                 815
   gat ctc tct ggt tgc tca gag ctc gag act att cag ggt ttt cct cgg      2496
   Asp Leu Ser Gly Cys Ser Glu Leu Glu Thr Ile Gln Gly Phe Pro Arg
               820                 825                 830
   aac cta aaa gag tta tat ttt gct ggc act acg tta aga gaa gtg ccc      2544
   Asn Leu Lys Glu Leu Tyr Phe Ala Gly Thr Thr Leu Arg Glu Val Pro
           835                 840                 845
   caa ctt cct tta agc cta gag gtc ttg aat gca cat ggt tct gac tcg      2592
   Gln Leu Pro Leu Ser Leu Glu Val Leu Asn Ala His Gly Ser Asp Ser
       850                 855                 860
   gag aag ctt cct atg cat tac aag ttc aac aat ttt ttc gat cta tct      2640
   Glu Lys Leu Pro Met His Tyr Lys Phe Asn Asn Phe Phe Asp Leu Ser
   865                 870                 875                 880
   caa caa gtg gtc aac gat ttt ttc ttg aaa gcg ctg act tat gta aaa      2688
   Gln Gln Val Val Asn Asp Phe Phe Leu Lys Ala Leu Thr Tyr Val Lys
                   885                 890                 895
   cac ata cca aga ggg tat acg cag gaa ctc atc aac aaa gct ccg act      2736
   His Ile Pro Arg Gly Tyr Thr Gln Glu Leu Ile Asn Lys Ala Pro Thr
               900                 905                 910
   ttc agc ttc agt gcg ccc tca cat aca aat caa aac gcc aca ttt gat      2784
   Phe Ser Phe Ser Ala Pro Ser His Thr Asn Gln Asn Ala Thr Phe Asp
           915                 920                 925
   ctg caa cca gga tct tct gta atg aca cga cta aat cat tca tgg agg      2832
   Leu Gln Pro Gly Ser Ser Val Met Thr Arg Leu Asn His Ser Trp Arg
       930                 935                 940
   aac acg ctt gtg gga ttt ggt atg ctg gtg gaa gtt gca ttt ccc gag      2880
   Asn Thr Leu Val Gly Phe Gly Met Leu Val Glu Val Ala Phe Pro Glu
   945                 950                 955                 960
   gac tac tgt gat gct aca gat gtt ggc ata agt tgt gtt tgc aga tgg      2928
   Asp Tyr Cys Asp Ala Thr Asp Val Gly Ile Ser Cys Val Cys Arg Trp
                   965                 970                 975
   agc aac aaa gaa ggc cgc tct tgt agg ata gaa aga aat ttt cat tgt      2976
   Ser Asn Lys Glu Gly Arg Ser Cys Arg Ile Glu Arg Asn Phe His Cys
               980                 985                 990
   tgg gca cca ggg aaa gtt gtt cca  aaa gtt cga aag gat  cat acg ttt    3024
   Trp Ala Pro Gly Lys Val Val Pro  Lys Val Arg Lys Asp  His Thr Phe
           995                 1000                 1005
   gtc ttt  agt gat gtc aac atg  cgc cca agt acc ggt  gaa gga aat      3069
   Val Phe  Ser Asp Val Asn Met  Arg Pro Ser Thr Gly  Glu Gly Asn
       1010                 1015                 1020
   gac cct  gat atc tgg gct gga  tta gtt gta ttt gag  ttc ttt cct      3114
   Asp Pro  Asp Ile Trp Ala Gly  Leu Val Val Phe Glu  Phe Phe Pro
       1025                 1030                 1035
   atc aat  cag cag aca aag tgt  cta aat gat agg ttc  aca gtg aca      3159
   Ile Asn  Gln Gln Thr Lys Cys  Leu Asn Asp Arg Phe  Thr Val Thr
       1040                 1045                 1050
   aga tgt  gga gtc cgt gta ata  aat gtt gca act ggc  aat aca agt      3204
   Arg Cys  Gly Val Arg Val Ile  Asn Val Ala Thr Gly  Asn Thr Ser
       1055                 1060                 1065
   ctt gag  aac ata tca cta gtt  ttg tct ttg gat cca  gtg gag gtt      3249
   Leu Glu  Asn Ile Ser Leu Val  Leu Ser Leu Asp Pro  Val Glu Val
       1070                 1075                 1080
   tct ggt  tat gaa gta ttg aga  gtc agc tat gat gat  tta cag gag      3294
   Ser Gly  Tyr Glu Val Leu Arg  Val Ser Tyr Asp Asp  Leu Gln Glu
       1085                 1090                 1095
   atg gat  aaa gtt cta ttt ctt  tac ata gcg tct ttg  ttc aat gac      3339
   Met Asp  Lys Val Leu Phe Leu  Tyr Ile Ala Ser Leu  Phe Asn Asp
       1100                 1105                 1110
   gag gat  gtt gat ttt gtg gca  cca ctt att gcc ggt  att gac ttg      3384
   Glu Asp  Val Asp Phe Val Ala  Pro Leu Ile Ala Gly  Ile Asp Leu
       1115                 1120                 1125
   gat gtt  agc tct ggg ctc aag  gtc tta gcc gat gtg  tct ctc ata      3429
   Asp Val  Ser Ser Gly Leu Lys  Val Leu Ala Asp Val  Ser Leu Ile
       1130                 1135                 1140
   agt gta  tca tcc aat ggg gaa  ata gtg atg cat agt  ttg caa aga      3474
   Ser Val  Ser Ser Asn Gly Glu  Ile Val Met His Ser  Leu Gln Arg
       1145                 1150                 1155
   caa atg  ggc aaa gaa atc ctc  cat gga caa tcc atg  ctg ctg tct      3519
   Gln Met  Gly Lys Glu Ile Leu  His Gly Gln Ser Met  Leu Leu Ser
       1160                 1165                 1170
   gat tgt  gag agt tcc atg acc  gag aat ttg tct gac  gta cca aaa      3564
   Asp Cys  Glu Ser Ser Met Thr  Glu Asn Leu Ser Asp  Val Pro Lys
       1175                 1180                 1185
   aag gag  aag aaa cat cgc gaa  agt aag gta aag aaa  gtg gtt tcc      3609
   Lys Glu  Lys Lys His Arg Glu  Ser Lys Val Lys Lys  Val Val Ser
       1190                 1195                 1200
   ata ccg  gct ata gac gag gga  gat cta tgg act tgg  cga aag tac      3654
   Ile Pro  Ala Ile Asp Glu Gly  Asp Leu Trp Thr Trp  Arg Lys Tyr
       1205                 1210                 1215
   ggt caa  aaa gac atc tta ggt  tct cgt ttt cca agg  ggt tac tac      3699
   Gly Gln  Lys Asp Ile Leu Gly  Ser Arg Phe Pro Arg  Gly Tyr Tyr
       1220                 1225                 1230
   agg tgc  gct tac aag ttc acg  cat ggt tgt aaa gct  aca aaa caa      3744
   Arg Cys  Ala Tyr Lys Phe Thr  His Gly Cys Lys Ala  Thr Lys Gln
       1235                 1240                 1245
   gtc caa  cgg agc gag acc gat  tca aac atg tta gct  att act tac      3789
   Val Gln  Arg Ser Glu Thr Asp  Ser Asn Met Leu Ala  Ile Thr Tyr
       1250                 1255                 1260
   cta tct  gag cat aac cat cca  cgg ccc act aaa cgc  aag gct ctc      3834
   Leu Ser  Glu His Asn His Pro  Arg Pro Thr Lys Arg  Lys Ala Leu
       1265                 1270                 1275
   gct gac  tcc act cgt tcc act  tcc tcc tcc atc tgc  tca gcc ata      3879
   Ala Asp  Ser Thr Arg Ser Thr  Ser Ser Ser Ile Cys  Ser Ala Ile
       1280                 1285                 1290
   act acc  tct gcc tca tct aga  gtc ttc caa aac aaa  gac gaa cca      3924
   Thr Thr  Ser Ala Ser Ser Arg  Val Phe Gln Asn Lys  Asp Glu Pro
       1295                 1300                 1305
   aat caa  ccc cac ttg cct tcc  tcc tcc act cct cct  aga aac gcg      3969
   Asn Gln  Pro His Leu Pro Ser  Ser Ser Thr Pro Pro  Arg Asn Ala
       1310                 1315                 1320
   gct gtc  ttg ttt aaa atg acg  gac atg gag gag ttt  cag gac aat      4014
   Ala Val  Leu Phe Lys Met Thr  Asp Met Glu Glu Phe  Gln Asp Asn
       1325                 1330                 1335
   atg gag  gtg gat aat gac gtc  gta gat aca cgt aca  ctg gca ttg      4059
   Met Glu  Val Asp Asn Asp Val  Val Asp Thr Arg Thr  Leu Ala Leu
       1340                 1345                 1350
   ttt cca  gag ttt caa cat cag  ccg gag gaa gaa gac  cca tgg tca      4104
   Phe Pro  Glu Phe Gln His Gln  Pro Glu Glu Glu Asp  Pro Trp Ser
       1355                 1360                 1365
   aca ttc  ttc gat gat tat aat  ttt tac ttt tga                    4137
   Thr Phe  Phe Asp Asp Tyr Asn  Phe Tyr Phe
       1370                 1375
<210> 12
   <211> 1378
   <212> PRT
   <213> Arabidopsis thaliana Nd-1
<400> 12
   Met Thr Asn Cys Glu Lys Asp Glu Glu Phe Val Cys Ile Ser Cys Val
   1               5                   10                  15
   Glu Glu Val Arg Tyr Ser Phe Val Ser His Leu Ser Glu Ala Leu Arg
               20                  25                  30
   Arg Lys Gly Ile Asn Asn Val Val Val Asp Val Asp Ile Asp Asp Leu
           35                  40                  45
   Leu Phe Lys Glu Ser Gln Ala Lys Ile Glu Lys Ala Gly Val Ser Val
       50                  55                  60
   Met Val Leu Pro Gly Asn Cys Asp Pro Ser Glu Val Trp Leu Asp Lys
   65                  70                  75                  80
   Phe Ala Lys Val Leu Glu Cys Gln Arg Asn Asn Lys Asp Gln Ala Val
                   85                  90                  95
   Val Ser Val Leu Tyr Gly Asp Ser Leu Leu Arg Asp Gln Trp Leu Ser
               100                 105                 110
   Glu Leu Asp Phe Arg Gly Leu Ser Arg Ile His Gln Ser Arg Lys Glu
           115                 120                 125
   Cys Ser Asp Ser Ile Leu Val Glu Glu Ile Val Arg Asp Val Tyr Glu
       130                 135                 140
   Thr His Phe Tyr Val Gly Arg Ile Gly Ile Tyr Ser Lys Leu Leu Glu
   145                 150                 155                 160
   Ile Glu Asn Met Val Asn Lys Gln Pro Ile Gly Ile Arg Cys Val Gly
                   165                 170                 175
   Ile Trp Gly Met Pro Gly Ile Gly Lys Thr Thr Leu Ala Lys Ala Val
               180                 185                 190
   Phe Asp Gln Met Ser Ser Ala Phe Asp Ala Ser Cys Phe Ile Glu Asp
           195                 200                 205
   Tyr Asp Lys Ser Ile His Glu Lys Gly Leu Tyr Cys Leu Leu Glu Glu
       210                 215                 220
   Gln Leu Leu Pro Gly Asn Asp Ala Thr Ile Met Lys Leu Ser Ser Leu
   225                 230                 235                 240
   Arg Asp Arg Leu Asn Ser Lys Arg Val Leu Val Val Leu Asp Asp Val
                   245                 250                 255
   Cys Asn Ala Leu Val Ala Glu Ser Phe Leu Glu Gly Phe Asp Trp Leu
               260                 265                 270
   Gly Pro Gly Ser Leu Ile Ile Ile Thr Ser Arg Asp Lys Gln Val Phe
           275                 280                 285
   Arg Leu Cys Gly Ile Asn Gln Ile Tyr Glu Val Gln Gly Leu Asn Glu
       290                 295                 300
   Lys Glu Ala Arg Gln Leu Phe Leu Leu Ser Ala Ser Ile Met Glu Asp
   305                 310                 315                 320
   Met Gly Glu Gln Asn Leu His Glu Leu Ser Val Arg Val Ile Ser Tyr
                   325                 330                 335
   Ala Asn Gly Asn Pro Leu Ala Ile Ser Val Tyr Gly Arg Glu Leu Lys
               340                 345                 350
   Gly Lys Lys Lys Leu Ser Glu Met Glu Thr Ala Phe Leu Lys Leu Lys
           355                 360                 365
   Arg Arg Pro Pro Phe Lys Ile Val Asp Ala Phe Lys Ser Ser Tyr Asp
       370                 375                 380
   Thr Leu Ser Asp Asn Glu Lys Asn Ile Phe Leu Asp Ile Ala Cys Phe
   385                 390                 395                 400
   Phe Gln Gly Glu Asn Val Asn Tyr Val Ile Gln Leu Leu Glu Gly Cys
                   405                 410                 415
   Gly Phe Phe Pro His Val Glu Ile Asp Val Leu Val Asp Lys Cys Leu
               420                 425                 430
   Val Thr Ile Ser Glu Asn Arg Val Trp Leu His Lys Leu Thr Gln Asp
           435                 440                 445
   Ile Gly Arg Glu Ile Ile Asn Gly Glu Thr Val Gln Ile Glu Arg Arg
       450                 455                 460
   Arg Arg Leu Trp Glu Pro Trp Ser Ile Lys Tyr Leu Leu Glu Tyr Asn
   465                 470                 475                 480
   Glu His Lys Ala Asn Gly Glu Pro Lys Thr Thr Phe Lys Arg Ala Gln
                   485                 490                 495
   Gly Ser Glu Glu Ile Glu Gly Leu Phe Leu Asp Thr Ser Asn Leu Arg
               500                 505                 510
   Phe Asp Leu Gln Pro Ser Ala Phe Lys Asn Met Leu Asn Leu Arg Leu
           515                 520                 525
   Leu Lys Ile Tyr Cys Ser Asn Pro Glu Val His Pro Val Ile Asn Phe
       530                 535                 540
   Pro Thr Gly Ser Leu His Ser Leu Pro Asn Glu Leu Arg Leu Leu His
   545                 550                 555                 560
   Trp Glu Asn Tyr Pro Leu Lys Ser Leu Pro Gln Asn Phe Asp Pro Arg
                   565                 570                 575
   His Leu Val Glu Ile Asn Met Pro Tyr Ser Gln Leu Gln Lys Leu Trp
               580                 585                 590
   Gly Gly Thr Lys Asn Leu Glu Met Leu Arg Thr Ile Arg Leu Cys His
           595                 600                 605
   Ser Gln His Leu Val Asp Ile Asp Asp Leu Leu Lys Ala Glu Asn Leu
       610                 615                 620
   Glu Val Ile Asp Leu Gln Gly Cys Thr Arg Leu Gln Asn Phe Pro Ala
   625                 630                 635                 640
   Ala Gly Arg Leu Leu Arg Leu Arg Val Val Asn Leu Ser Gly Cys Ile
                   645                 650                 655
   Lys Ile Lys Ser Val Leu Glu Ile Pro Pro Asn Ile Glu Lys Leu His
               660                 665                 670
   Leu Gln Gly Thr Gly Ile Leu Ala Leu Pro Val Ser Thr Val Lys Pro
               675                 680                 685
   Asn His Arg Glu Leu Val Asn Phe Leu Thr Glu Ile Pro Gly Leu Ser
       690                 695                 700
   Glu Ala Ser Lys Leu Glu Arg Leu Thr Ser Leu Leu Glu Ser Asn Ser
   705                 710                 715                 720
   Ser Cys Gln Asp Leu Gly Lys Leu Ile Cys Leu Glu Leu Lys Asp Cys
                   725                 730                 735
   Ser Cys Leu Gln Ser Leu Pro Asn Met Ala Asn Leu Asp Leu Asn Val
               740                 745                 750
   Leu Asp Leu Ser Gly Cys Ser Ser Leu Asn Ser Ile Gln Gly Phe Pro
           755                 760                 765
   Arg Phe Leu Lys Gln Leu Tyr Leu Gly Gly Thr Ala Ile Arg Glu Val
       770                 775                 780
   Pro Gln Leu Pro Gln Ser Leu Glu Ile Leu Asn Ala His Gly Ser Cys
   785                 790                 795                 800
   Leu Arg Ser Leu Pro Asn Met Ala Asn Leu Glu Phe Leu Lys Val Leu
                   805                 810                 815
   Asp Leu Ser Gly Cys Ser Glu Leu Glu Thr Ile Gln Gly Phe Pro Arg
               820                 825                 830
   Asn Leu Lys Glu Leu Tyr Phe Ala Gly Thr Thr Leu Arg Glu Val Pro
           835                 840                 845
   Gln Leu Pro Leu Ser Leu Glu Val Leu Asn Ala His Gly Ser Asp Ser
       850                 855                 860
   Glu Lys Leu Pro Met His Tyr Lys Phe Asn Asn Phe Phe Asp Leu Ser
   865                 870                 875                 880
   Gln Gln Val Val Asn Asp Phe Phe Leu Lys Ala Leu Thr Tyr Val Lys
                   885                 890                 895
   His Ile Pro Arg Gly Tyr Thr Gln Glu Leu Ile Asn Lys Ala Pro Thr
               900                 905                 910
   Phe Ser Phe Ser Ala Pro Ser His Thr Asn Gln Asn Ala Thr Phe Asp
           915                 920                 925
   Leu Gln Pro Gly Ser Ser Val Met Thr Arg Leu Asn His Ser Trp Arg
       930                 935                 940
   Asn Thr Leu Val Gly Phe Gly Met Leu Val Glu Val Ala Phe Pro Glu
   945                 950                 955                 960
   Asp Tyr Cys Asp Ala Thr Asp Val Gly Ile Ser Cys Val Cys Arg Trp
                   965                 970                 975
   Ser Asn Lys Glu Gly Arg Ser Cys Arg Ile Glu Arg Asn Phe His Cys
               980                 985                 990
   Trp Ala Pro Gly Lys Val Val Pro  Lys Val Arg Lys Asp  His Thr Phe
           995                 1000                 1005
   Val Phe  Ser Asp Val Asn Met  Arg Pro Ser Thr Gly  Glu Gly Asn
       1010                 1015                 1020
   Asp Pro  Asp Ile Trp Ala Gly  Leu Val Val Phe Glu  Phe Phe Pro
       1025                 1030                 1035
   Ile Asn  Gln Gln Thr Lys Cys  Leu Asn Asp Arg Phe  Thr Val Thr
       1040                 1045                 1050
   Arg Cys  Gly Val Arg Val Ile  Asn Val Ala Thr Gly  Asn Thr Ser
       1055                 1060                 1065
   Leu Glu  Asn Ile Ser Leu Val  Leu Ser Leu Asp Pro  Val Glu Val
       1070                 1075                 1080
   Ser Gly  Tyr Glu Val Leu Arg  Val Ser Tyr Asp Asp  Leu Gln Glu
       1085                 1090                 1095
   Met Asp  Lys Val Leu Phe Leu  Tyr Ile Ala Ser Leu  Phe Asn Asp
       1100                 1105                 1110
   Glu Asp  Val Asp Phe Val Ala  Pro Leu Ile Ala Gly  Ile Asp Leu
       1115                 1120                 1125
   Asp Val  Ser Ser Gly Leu Lys  Val Leu Ala Asp Val  Ser Leu Ile
       1130                 1135                 1140
   Ser Val  Ser Ser Asn Gly Glu  Ile Val Met His Ser  Leu Gln Arg
       1145                 1150                 1155
   Gln Met  Gly Lys Glu Ile Leu  His Gly Gln Ser Met  Leu Leu Ser
       1160                 1165                 1170
   Asp Cys  Glu Ser Ser Met Thr  Glu Asn Leu Ser Asp  Val Pro Lys
       1175                 1180                 1185
   Lys Glu  Lys Lys His Arg Glu  Ser Lys Val Lys Lys  Val Val Ser
       1190                 1195                 1200
   Ile Pro  Ala Ile Asp Glu Gly  Asp Leu Trp Thr Trp  Arg Lys Tyr
       1205                 1210                 1215
   Gly Gln  Lys Asp Ile Leu Gly  Ser Arg Phe Pro Arg  Gly Tyr Tyr
       1220                 1225                 1230
   Arg Cys  Ala Tyr Lys Phe Thr  His Gly Cys Lys Ala  Thr Lys Gln
       1235                 1240                 1245
   Val Gln  Arg Ser Glu Thr Asp  Ser Asn Met Leu Ala  Ile Thr Tyr
       1250                 1255                 1260
   Leu Ser  Glu His Asn His Pro  Arg Pro Thr Lys Arg  Lys Ala Leu
       1265                 1270                 1275
   Ala Asp  Ser Thr Arg Ser Thr  Ser Ser Ser Ile Cys  Ser Ala Ile
       1280                 1285                 1290
   Thr Thr  Ser Ala Ser Ser Arg  Val Phe Gln Asn Lys  Asp Glu Pro
       1295                 1300                 1305
   Asn Gln  Pro His Leu Pro Ser  Ser Ser Thr Pro Pro  Arg Asn Ala
       1310                 1315                 1320
   Ala Val  Leu Phe Lys Met Thr  Asp Met Glu Glu Phe  Gln Asp Asn
       1325                 1330                 1335
   Met Glu  Val Asp Asn Asp Val  Val Asp Thr Arg Thr  Leu Ala Leu
       1340                 1345                 1350
   Phe Pro  Glu Phe Gln His Gln  Pro Glu Glu Glu Asp  Pro Trp Ser
       1355                 1360                 1365
   Thr Phe  Phe Asp Asp Tyr Asn  Phe Tyr Phe
       1370                 1375
   1

## Description

### [Technical Field]

The present invention relates to a method for providing a plant with resistance to two or more pathogens by introducing a combination of two or more resistance genes to the pathogens into the plant; a transgenic plant produced by the method; and a portion thereof or a propagation material.

### [Background Art]

It is said that 10 to 20% of agricultural production worldwide suffers from diseases. This corresponds to foods for 800 million people. Since the world starving population is estimated to be 800 million, to prevent such diseases is the most important issue for stable food supply. The factor of causing diseases in plants includes an infectious biological agent (pathogen) and a non-infectious environmental agent. Filamentous fungi (molds, fungi) cause 80% or more of plant infectious diseases, whereas the rest of the plant infectious diseases are caused by bacteria, viruses, viroids, phytoplasma, rickettsia-like microorganisms, nematodes, protists, and the like. Plants are always exposed to attacks from these pathogens, and protect themselves from the pathogenic infection by own biological defense reactions.

For example, Cruciferous vegetable anthracnose is a disease caused by a filamentous fungus (Colletotrichum higginsianum) in the genus Colletotrichum as a pathogen. Cruciferous crops such as Japanese mustard spinach, Chinese cabbage and white radish are infected with and damaged by the disease. Recently, a number of Arabidopsis ecotypes have been found which show resistance to this pathogen (Non Patent Literature 1). Moreover, the present inventors have found that a gene in a specific region on chromosome 5 of the resistant ecotype Ws-0 has a function to provide Arabidopsis with resistance to Cruciferous vegetable anthracnose.

In addition, Arabidopsis has been discovered to have a resistance gene to a pathogen of bacterial leaf spot of tomato (pathogenic bacterium Pseudomonas syringae pv. tomato) which infects mainly Solanaceae plants and which has an avirulence gene avrRps4 (Non Patent Literature 2). Furthermore, Arabidopsis has also been discovered to have a resistance gene to a bacterial wilt (Ralstonia solanacearum) which infects and blights over 200 species of plants such as Solanaceae and Cruciferous plants and which causes detrimental damage in agriculture (Non Patent Literatures 3 to 5).

Such biological defense reactions of plants are thought, according to the gene-for-gene hypothesis proposed by Flor (Non Patent Literature 6), to take place when a resistance gene product of a plant recognizes an elicitor (avirulence gene product) derived from a pathogen and expresses a series of resistance reactions to the pathogen. In addition, in this hypothesis, one resistance gene acts on one pathogen on a one-for-one basis.

Although hundreds of thousands of filamentous fungi exist on the earth, the majority thereof do not infect plants. Only approximately 8000 species are plant pathogens, among which no more than 10 species cause severe damages to single plant species. In other words, plants show resistance to most of the pathogens. Despite this, only approximately 150 (Non Patent Literature 7) and approximately 600 (Non Patent Literature 8) resistance genes are present on the Arabidopsis genome and on the rice genome, respectively. Accordingly, plant resistance mechanisms to various pathogens cannot be explained only by the gene-for-gene hypothesis. To date, the details thereof have not been elucidated yet.

### [Citation List]

### [Non Patent Literatures]

[NPL 1] Y. Narusaka et al. Molecular Plant-Microbe Interactions, 17: 749-762 (2004)
[NPL 2] W. Gassmann et al. Plant J. 20: 265-277 (1999)
[NPL 3] L. Deslandes et al. Molecular Plant-Microbe Interactions, 11 (7): 659-667 (1998)
[NPL 4] L. Deslandes et al. Proc Natl Acad Sci USA., 99 (4): 2404-2409 (2002)
[NPL 5] L. Deslandes et al. Proc Natl Acad Sci USA., 100: 8024-8029 (2003)
[NPL 6] H. Flor. Annu. Rev. Phytopathol. 9: 275-296 (1971)
[NPL 7] Meyers, B. C. et al. Plant Cell, 15: 809-834 (2003)
[NPL 8] Goff, S. A. et al., Science, 296: 92-100 (2002)
[NPL 9] Tsuchiya et al. , Plant Protection 54:87-92 (2000)
[NPL 10] Clark et al., Science, 317: 338-342 (2007)
[NPL 11] Fedoroff NV. and Smith D. Plant J. 3: 273-289 (1993)
[NPL 12] Noutoshi et al. Plant J., 43: 873-888 (2005)

### [Summary of Invention]

### [Technical Problem]

The present invention has been made in view of such circumstances. An object thereof is to elucidate a mechanism in plant resistance to various pathogens, and to produce a plant showing resistance to various pathogens by utilizing the mechanism.

### [Solution to Problem]

The present inventors have revealed for the first time in the world that a Cruciferous vegetable anthracnose pathogen (Colletotrichum higginsianum) infects the Arabidopsis ecotype Col-0, and established a model experimental system for plant immunology research (Non Patent Literature 1). As a result of infection-physiological and genetic analyses on over 100 Arabidopsis ecotypes, 11 ecotypes showing resistance to this pathogen are obtained. Then, the inventors have revealed that the resistance of Eil-0 showing particularly strong resistance among these is dominant and controlled at a single locus, and this gene is present on chromosome 4 among five chromosomes of Arabidopsis (Non Patent Literature 1). The gene (locus) is highly likely to be a resistance gene (R-gene, a receptor on the plant side that recognizes the pathogen), and is named as RCH1 (for Recognition of C. higginsianum).

It has been suggested that, in other resistant ecotypes, a resistance gene is present at a locus different from that in Eil-0. Accordingly, the present inventors have proceeded with the analysis on this gene and found out that the resistance gene in the ecotype Ws-0 is present on chromosome 5. As a result of detailed mapping of the resistance gene in the ecotype Ws-0, the target gene is located in a region where a cluster of over ten resistance genes is formed (MRC-J -region). Then, the present inventors have screened for mutants whose phenotype changed from resistant to susceptible to an anthracnose pathogen through the reverse genetic analysis using a mutant library where a T-DNA is inserted into the ecotype Ws-0 (30,000 individuals). The analysis on the T-DNA insertion sites and mutation sites of eight mutants thus obtained has revealed that the susceptible mutants have a mutation in Ws-At5g45250 (designated as RPS4 by Gassmann) or Ws-At5g45260 gene (designated as RCH2 by the present inventors).

Several reports have been made so far on the relationships between RPS4, RCH2 and the resistance to pathogen. For example, Gassmann et al. have already identified that RPS4 in the Arabidopsis ecotypes Col-0 and Ler-0 is a resistance gene to a pathogen of bacterial leaf spot of tomato (pathogenic bacterium Pseudomonas syringae pv. tomato) (Non Patent Literature 2). Meanwhile, Gassmann et al. states that RCH2 (in the literature, referred to as RSH4) present adjacent to RPS4 has nothing to do with the pathogen of bacterial leaf spot of tomato (Non Patent Literature 2).

Moreover, a different research group than Gassmann et al. has identified that RCH2 (in the literature, referred to as RRS1-R) in the Arabidopsis ecotypes Nd-1 and Ct-1 is a resistance gene to a bacterial wilt pathogen (soilborne pathogenic bacterium Ralstonia solanacearum) which infects Solanaceae and Cruciferous plants (Non Patent Literatures 3 to 5). Meanwhile, this research group states that RPS4 has nothing to do with the resistance to the bacterial wilt pathogen (Non Patent Literature 4).

As described above, RPS4 and RCH2 have been heretofore identified as the resistance genes to pathogens other than the anthracnose pathogen. However, the present inventors have revealed that the genes function as the resistance genes to the anthracnose pathogen.

Furthermore, as a result of analyzing the relationship between the resistance/susceptibility and the two genes in various ecotypes, the present inventors have revealed that both RPS4 and RCH2 are necessary to induce the resistance to the pathogens of anthracnose, bacterial leaf spot of tomato, and bacterial wilt.

This result demolishes the conventional reports that RPS4 and RCH2 respectively have nothing to do with the pathogens of bacterial wilt and bacterial leaf spot of tomato, and also changes the conventional concept on the mechanism in the resistance reactions of plants to pathogens.

Specifically, it has been thought, according to the gene-for-gene hypothesis proposed by Flor, that the resistant reactions of plants to pathogens are determined by a 1:1 combination of a resistance gene of a plant with a corresponding avirulence gene (Avr-gene) of a pathogen (Non Patent Literature 6). However, the present inventors have discovered for the first time in the world that two different resistance genes adjacent to each other on the genome of Arabidopsis recognize attacks from three different pathogens and activate the resistance reactions. The present invention has reveals that the immune systemofplants, as similar to that of animals, recognizes various pathogens by combining a few genes and expresses the defense system.

In other words, the present invention is based on the discovery of the novel mechanism in the resistance reactions of plants to pathogens. More specifically, the present invention is as follows.
<1> A method for providing a plant with resistance to two or more pathogens, the method characterized by comprising: introducing a combination of two or more genes into the plant, wherein the genes do not provide the plant with the resistance to the two or more pathogens when each of the genes is introduced alone, but provide the plant with the resistance to the pathogens when introduced in the combination.
<2> The method according to <1>, wherein the pathogens are pathogens of Cruciferous vegetable anthracnose and bacterial leaf spot of tomato, and the combination of the genes is a combination of an RPS4 gene and an RCH2 gene.
<3> The method according to <2>, wherein the RPS4 gene and the RCH2 gene are derived from an Arabidopsis ecotype selected from the group consisting of Ws-0, No-0, Nd-1, Aa-0, Eil-0, Rrs-7, Sha, Tamm-2, Tsu-1, Fei-0, Ts-1, Bsch-0, Br-0, Est-1, Rrs-10, Van-0, Nfa-8, and Bay-0.
<4> The method according to any one of <2> and <3>, wherein the pathogens further include a bacterial wilt pathogen.
<5> A transgenic plant provided with resistance to two or more pathogens, comprising: a combination of two or more genes introduced therein, wherein the genes do not provide the plant with the resistance to the two or more pathogens when each of the genes is introduced alone, but provide the plant with the resistance to the pathogens when introduced in the combination.
<6> The transgenic plant according to <5>, wherein the pathogens are pathogens of Cruciferous vegetable anthracnose and bacterial leaf spot of tomato, and the combination of the genes is a combination of an RPS4 gene and an RCH2 gene.
<7> The transgenic plant according to <6>, wherein the RPS4 gene and the RCH2 gene are derived from an Arabidopsis ecotype selected from the group consisting of Ws-0, No-0, Nd-1, Aa-0, Eil-0, Rrs-7, Sha, Tamm-2, Tsu-1, Fei-0, Ts-1, Bsch-0, Br-0, Est-1, Rrs-10, Van-0, Nfa-8, and Bay-0.
<8> The transgenic plant according to any one of <6> and <7>, which further has resistance to a bacterial wilt pathogen.
<9> A transgenic plant which is any one of a progeny and a clone of the transgenic plant according to any one of <5> to <8>.
<10> Any one of a portion and a propagation material of the transgenic plant according to any one of <5> to <9>.

### [Advantageous Effects of Invention]

According to the present invention, a combination of multiple different resistance genes is introduced to a plant, enabling the plant to recognize attacks from multiple different pathogens and to activate the resistance reaction. Utilization of the mechanism of the resistance inplants to diseases discovered by the present inventors makes it possible to produce plants showing resistance to a wide range of diseases. For example, by introducing the diseases resistance genes RPS4 and RCH2 into a plant simultaneously, it is possible to provide the plant with resistance to pathogens of Cruciferous vegetable anthracnose, bacterial leaf spot, and bacterial wilt. Thereby, crops are protected from diseases due to the pathogens of Cruciferous vegetable anthracnose, bacterial leaf spot, and bacterial wilt, thus improving the productivity of Cruciferous crops, Solanaceae crops, and the like. Moreover, cases are reported that, when a disease resistance gene is introduced solely, the plant is generally dwarfed. Nevertheless, the combination of multiple genes of the present invention can avoid such a problem of dwarfed plants in producing resistant plants.

### [Brief Description of Drawings]

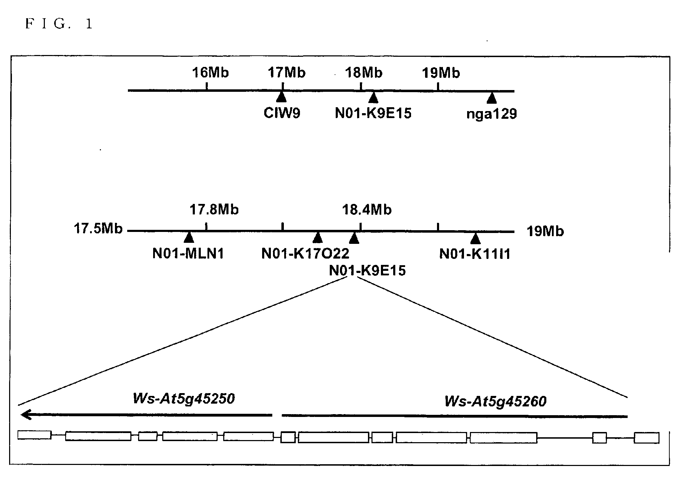

[Fig. 1] Fig. 1 is a drawing showing a region where resistance genes exist, the region being narrowed down by an SSLP (simple sequence length polymorphism) analysis. In the Arabidopsis ecotype (Ws-0), resistance genes At5g45250 and At5g45260 are present adjacent to each other. CIW9, N01-K9E15, nga129, N01-MLN1, N01-K17022, and N01-K11I1 indicate SSLP markers.

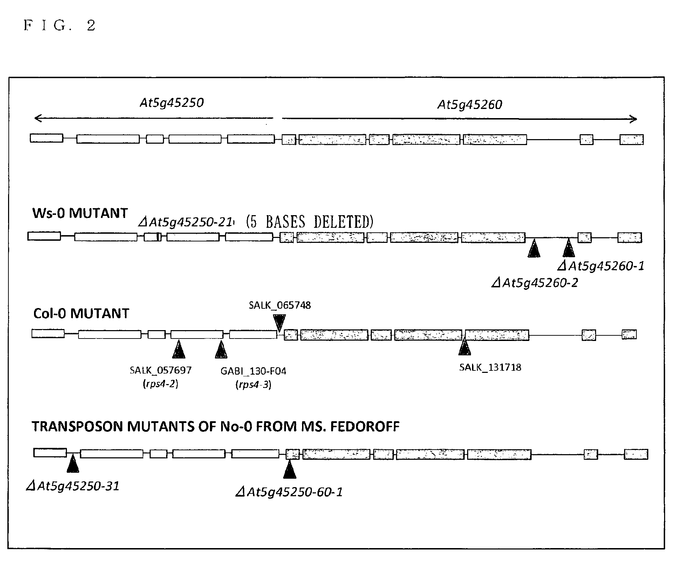

[Fig. 2] Fig. 2 is a drawing showing mutants of the Arabidopsis ecotypes Ws-0 and Col-0 as well as No-0 from Ms. Fedoroff.

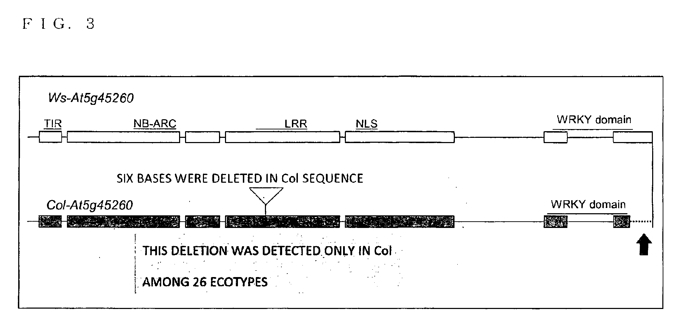

[Fig. 3] Fig. 3 is a drawing showing comparison of the At5g45260 amino acid sequence between the Arabidopsis ecotypes Ws-0 and Col-0. From At5g45260 of Col-0, 83 amino acids at a portion indicated by the arrow in the drawing are deleted as compared with Ws-0.

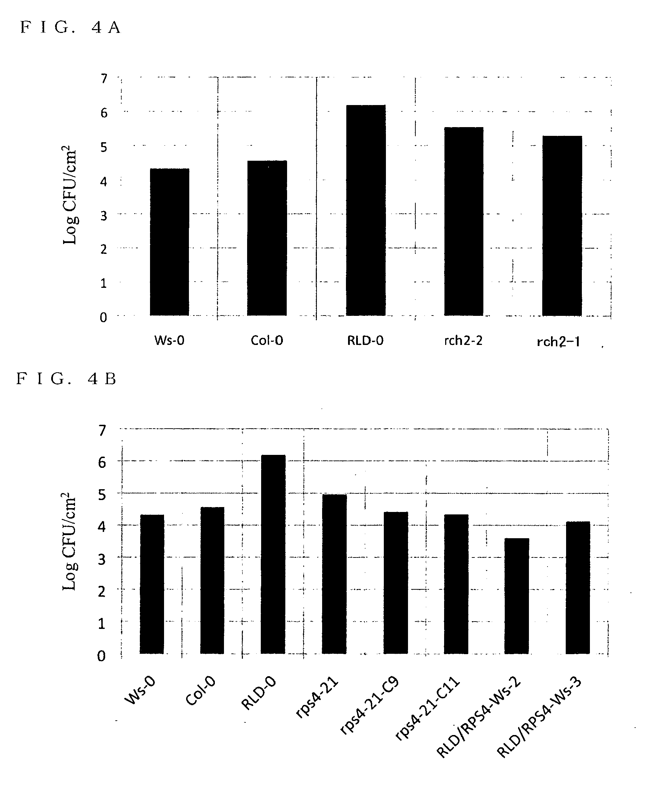

[Fig. 4A] Fig. 4A is a graph showing the result of analyzing the relationship between a mutation in At5g45260 and resistance to bacterial leaf spot, illustrating the influence of disruption of At5g45260 (RCH2) in the ecotype Ws-0 resistant to an anthracnose pathogen. In the graph, each of Ws-0, Col-0, and RLD-0 indicates a wildtype. In addition, "rch2-2" (ΔAt5g45260-2) and "rch2-1" (ΔAt5g45260-1) indicate disruptants (2 cases) of At5g45260 in Ws.
[Fig. 4B] Fig. 4B is a graph showing the result of analyzing the relationship between a mutation in At5g45250 and resistance to bacterial leaf spot, illustrating the influence of disruption of At5g45250 (RPS4) in the ecotype Ws-0 resistant to the anthracnose pathogen and the influence of introduction of ΔAt5g45250 in the ecotype RLD. In the graph, each of Ws-0, Col-0, and RLD-0 indicates a wildtype. Moreover, "rps4-21" (ΔAt5g45250-21) indicates a disruptant of Ws-At5g45250; "rps4-21-C9" and "rps4-21-C11" indicate revertants (2 cases) obtained by reintroducing Ws-At5g45250 into disruptants of Ws-At5g45250; and "RLD/RPS4-Ws-2" and "RLD/RPS4-Ws-3" indicate transformants (2 cases) obtained by introducing Ws-At5g45250 into RLD-0.

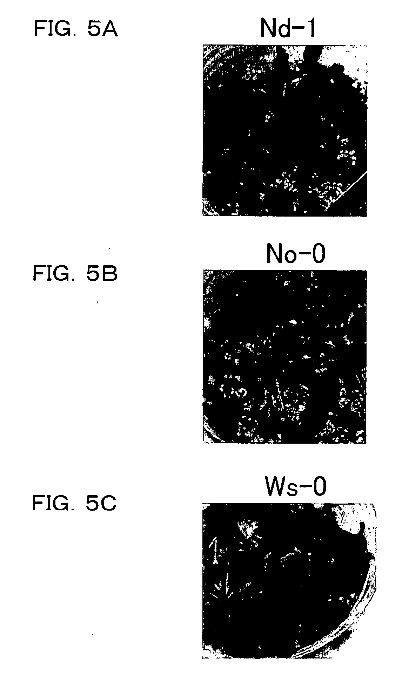

[Figs. 5A-G] Figs. 5A to G are pictures showing the result (morphology of organisms) of analyzing the relationship between mutations in At5g45250 and At5g45260 and resistance to bacterial wilt. In the pictures, Nd-1 (resistant), Ws-0 (moderately resistant), No-0 (resistant), and Col-0 (susceptible) indicate wildtypes. rps4-31 (ΔAt5g45250-31) is a mutant of No-0 from Ms. Fedoroff having At5g45250 disrupted, and is susceptible to a bacterial wilt pathogen. rps4-21 (ΔAt5g45250-21) is a mutant of Ws-0 having At5g45250 disrupted, and is susceptible to the bacterial wilt pathogen. rch2-1 (ΔAt5g45260-1) is a mutant of Ws-0 having At5g45260 disrupted, and is susceptible to the bacterial wilt pathogen.

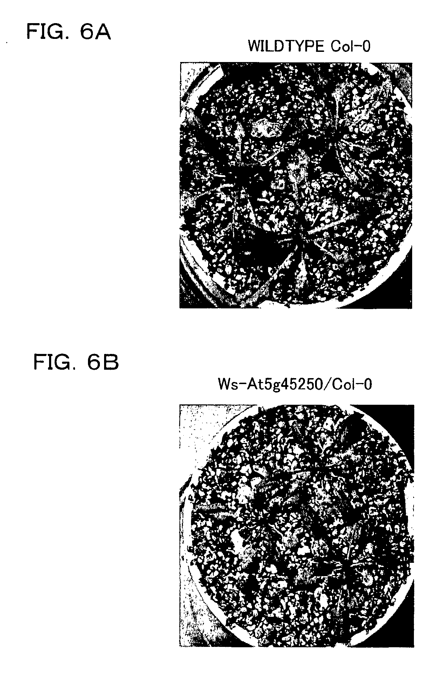

[Figs. 6A-D] Figs. 6A to 6D are pictures showing the result (morphology of organisms) of introducing a resistance gene into the Arabidopsis ecotypes Col-0 and RLD-0 susceptible to the anthracnose pathogen. Ws-At5g45250/Col-0 is a transformant obtained by introducing the resistance gene Ws-At5g45250 into the wildtype Col-0. Ws-At5g45250/RLD is a transformant obtained by introducing the resistance gene Ws-At5g45250 into the wildtype RLD-0. No-0-At5g45260/Col-0 is a transformant obtained by introducing a resistance gene No-0-At5g45260 from Ms. Fedoroff into the wildtype Col-0.

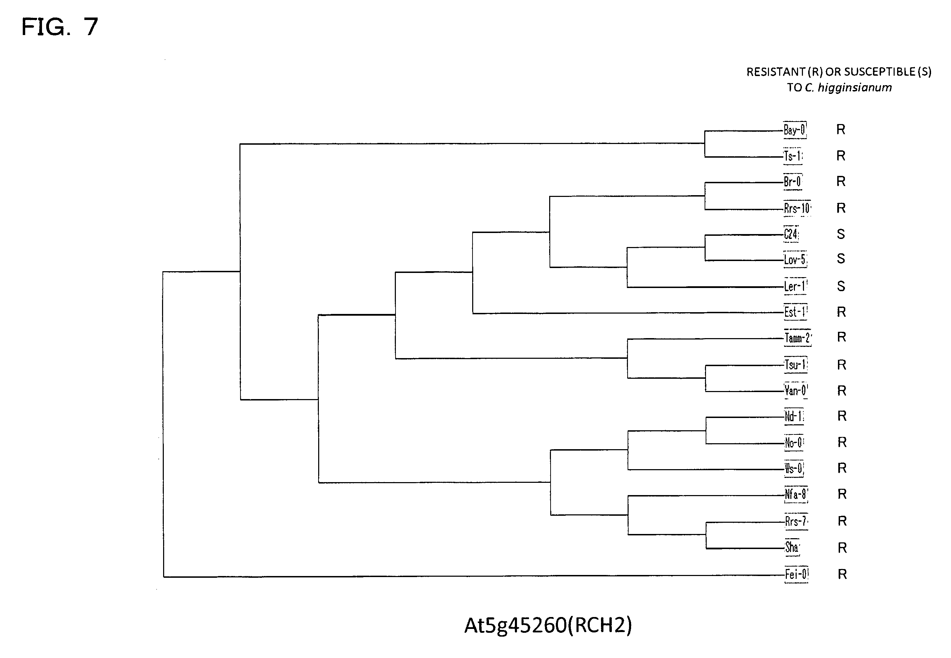

[Fig. 7] Fig. 7 shows the result of a phylogenetic tree analysis conducted on the amino acid sequences of At5g45250 and At5g45260 by utilizing data analyzed by the present inventors and SNP analysis data on Arabidopsis (Non Patent Literature 10).

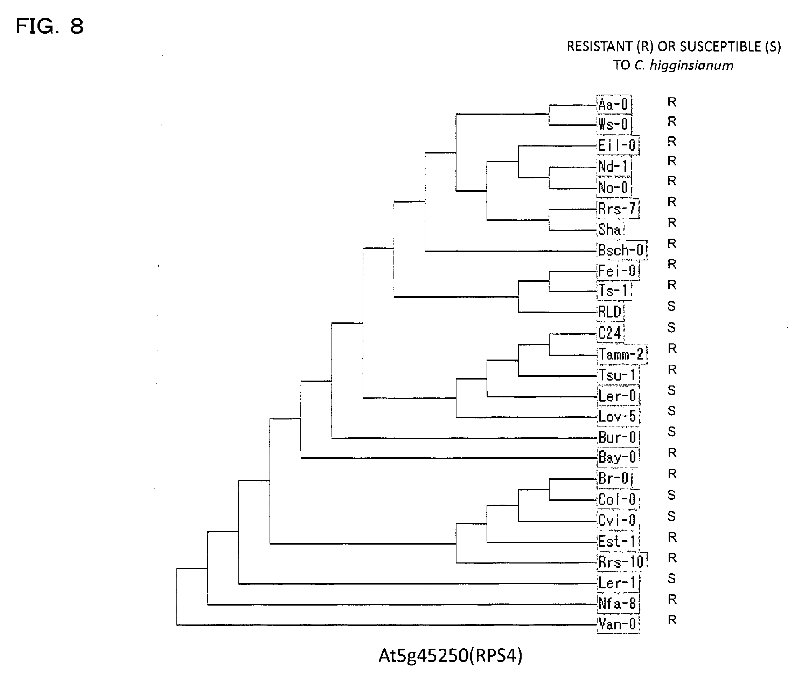

[Fig. 8] Fig. 8 is a continuation of Fig. 7.

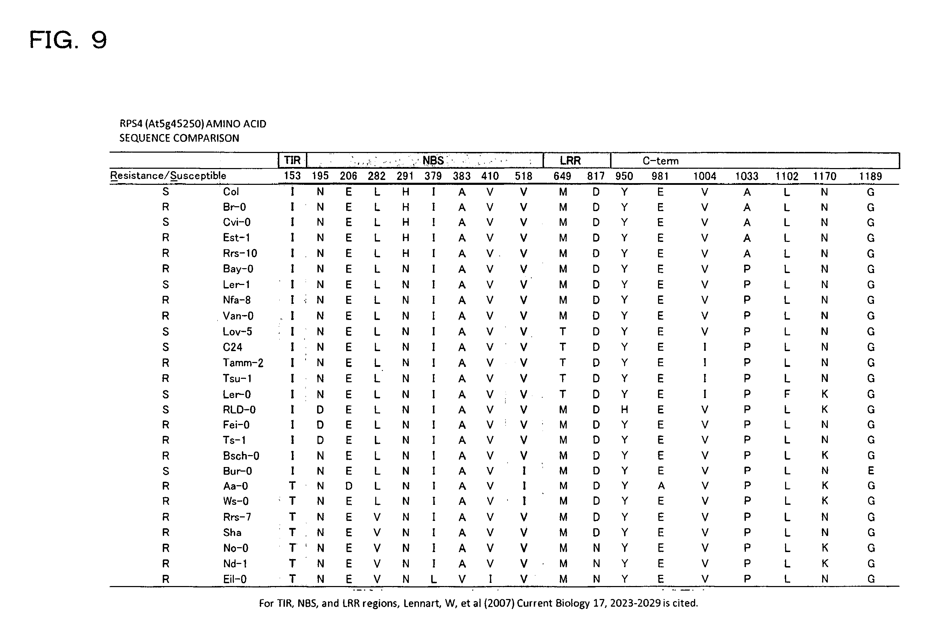

[Fig. 9] Fig. 9 shows comparison of the RPS4 (At5g45250) amino acid sequence among ecotypes. The table shows amino acids not conserved among the ecotypes. The 950th tyrosine of RLD-0 susceptible to all of Cruciferous vegetable anthracnose, bacterial leaf spot, and bacterial wilt is substituted with histidine.

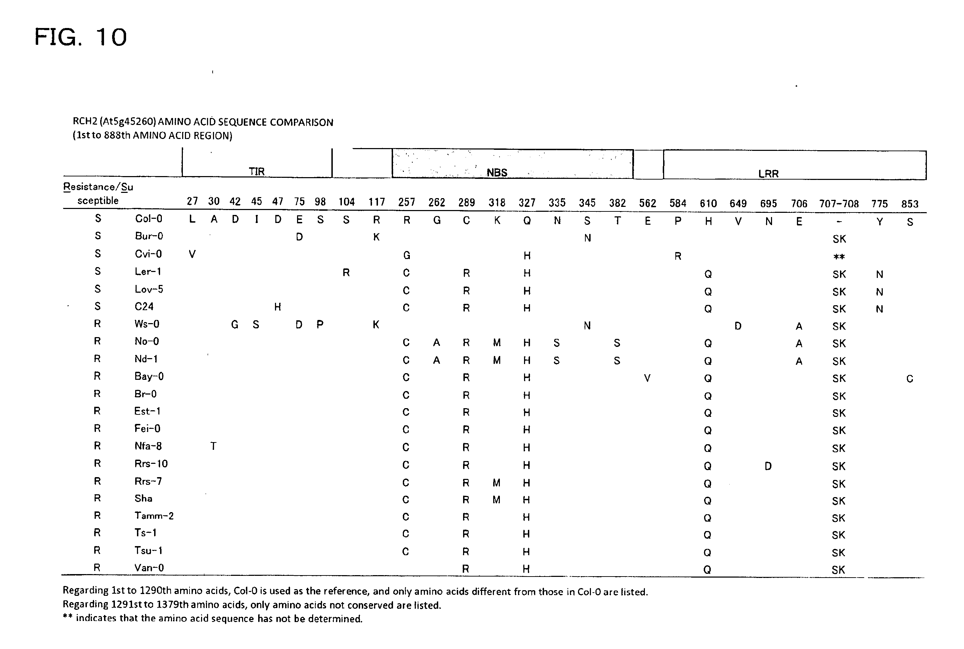

[Fig. 10] Fig. 10 shows the comparison of the RCH2 (At5g45260) amino acid sequence among ecotypes. In the region of the lst to 1290thamino acids, amino acids not conserved among the ecotypes are shown based on the amino acid sequence of Col-0 . In the region of the 1291th to 1379th amino acids, amino acids not conserved among the ecotypes are shown. The 775th tyrosine of the ecotypes (Ler-1, Lov-5, C24) susceptible to the anthracnose pathogen is substituted with asparagine. Approximately 80 to 90 amino acids are deleted from the WRKY domain of Col-0 toward the C terminal (Fig. 3). The ecotypes Cvi-0 and Bur-0 susceptible to the anthracnose pathogen are also recognized to have deletion toward the C terminal as in Col-At5g45260.

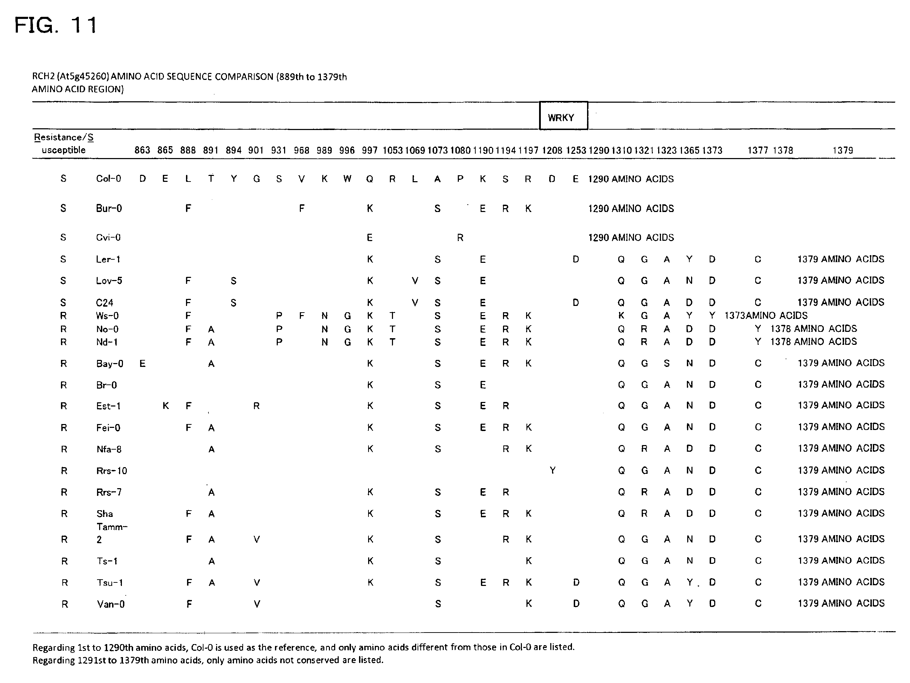

[Fig. 11] Fig. 11 is a continuation of Fig. 10.

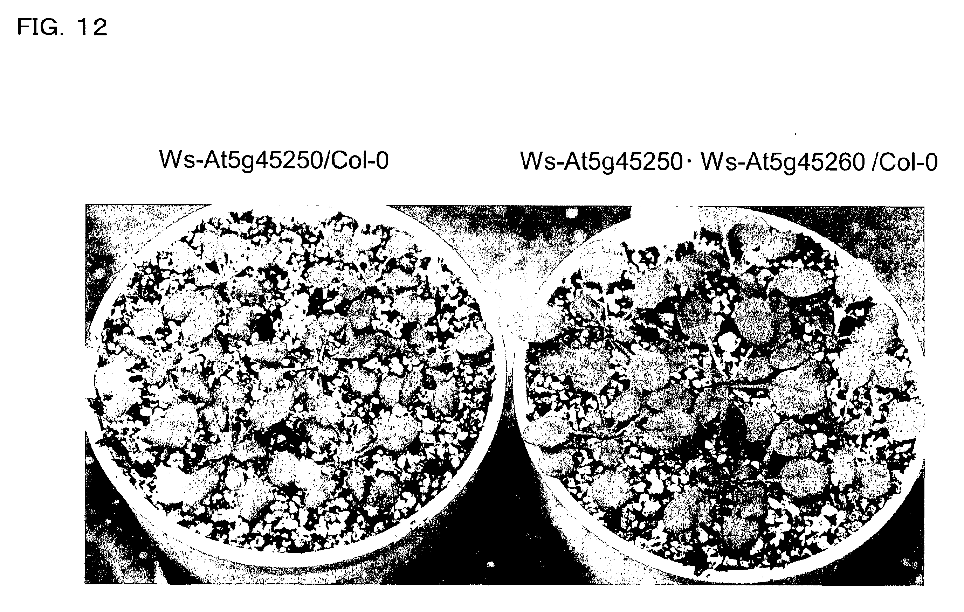

[Fig. 12] Fig. 12 is a picture showing the result (morphology of organisms) of introduction of resistance genes into the Arabidopsis ecotype Col-0 susceptible to the anthracnose pathogen. Ws-At5g45250/Col-0 is a transformant obtained by introducing the resistance gene Ws-At5g45250 into the wildtype Col-0. Ws-At5g45250·At5g45260/Col-0 is a transformant obtained by introducing the resistance gene Ws-At5g45260 into Ws-At5g45250/Col-0.

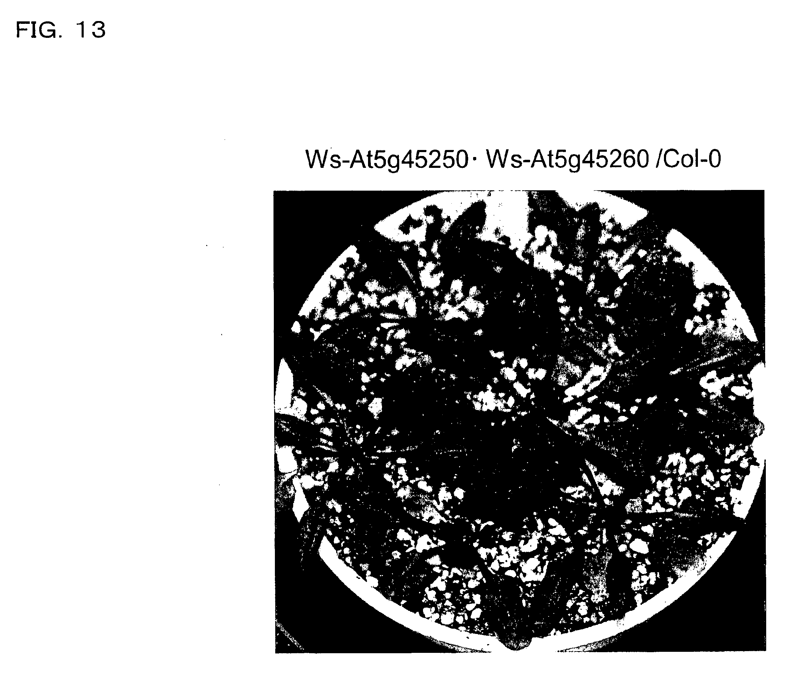

[Fig. 13] Fig. 13 is a picture showing the symptom (morphology of an organism) six days after the anthracnose pathogen is sprayed on and inoculated into Ws-At5g45250-At5g45260/Col-0 shown in Fig. 12.

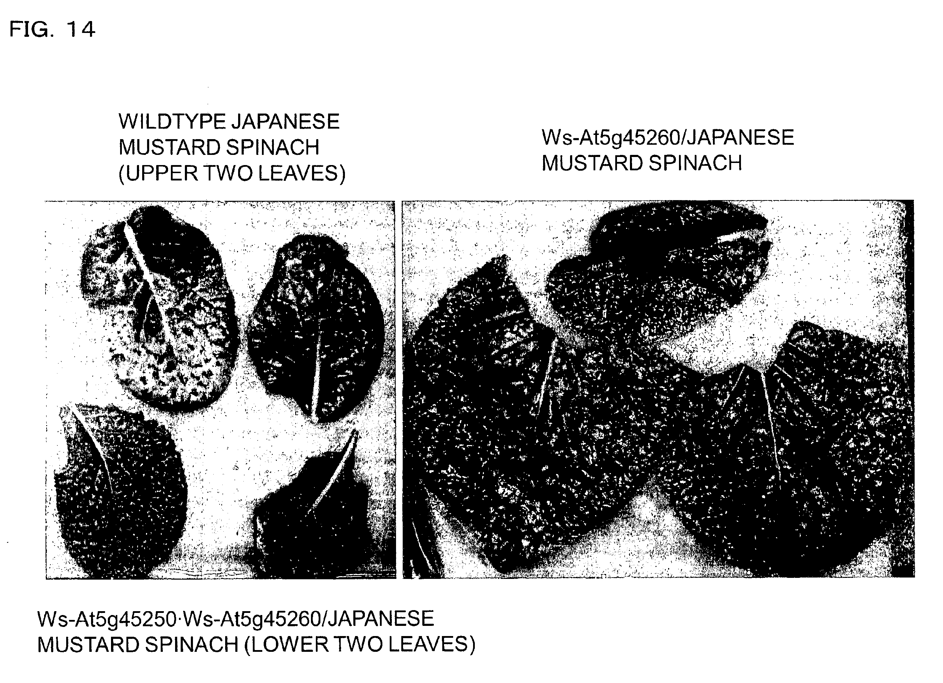

[Fig. 14] Fig. 14 is pictures showing the result (morphology of organisms) of introduction of resistance genes into an anthracnose-pathogen susceptible cultivar "Osome" of Cruciferous crop Japanese mustard spinach. Ws-At5g45260/Japanese mustard spinach is a transformant obtained by introducing the Arabidopsis resistance gene Ws-At5g45260 into the wildtype Japanese mustard spinach. Ws-At5g45250-At5g45260/Japanese mustard spinach is a transformant obtained by introducing the Arabidopsis resistance genes Ws-At5g45250 and Ws-At5g45260 into the wildtype Japanese mustard spinach at the same time. Shown are the lesions six days after the anthracnose pathogen is sprayed on and inoculated into the wildtype Japanese mustard spinach and the transformed Japanese mustard spinach.

### [Description of Embodiments]

The present invention is based on an innovative finding in resistance reactions of plants to pathogens that a combination of multiple different resistance genes allows a plant to recognize attacks from multiple different pathogens and to activate the resistance reactions. Accordingly, a method of the present invention is a method for providing a plant with resistance to two or more pathogens, the method characterized by comprising: introducing a combination of two or more genes into the plant, wherein the genes do not provide the plant with sufficient resistance to these pathogens when each of the genes is introduced alone, but provide the plant with these resistance to the pathogens when introduced in the combination.

In the present invention, a "combination of genes that provides a plant with a resistance to two or more pathogens" is not limited, as long as the combination is capable of activating the resistance reactions of the plant to the pathogens in accordance with the above-described mechanisms. For example, in a case where the pathogens are pathogens of Cruciferous vegetable anthracnose and bacterial leaf spot of tomato, the combination is a combination of an RPS4 gene and an RCH2 gene. Herein, "Cruciferous vegetable anthracnose" is a disease caused by a mold (filamentous fungus) called Colletotrichumhigginsianum as a pathogen. The disease occurs in Cruciferous crops such as Japanese mustard spinach, turnip, Chinese cabbage, and white radish. Meanwhile, "bacterial leaf spot of tomato" is a disease caused by a bacterium called Pseudomonas syringae pv. tomato as a pathogen. The disease mainly occurs in Solanaceae plants and Arabidopsis. Examples of the pathogens which are targets of the resistance provided to a plant by utilizing the combination of the RPS4 gene and the RCH2 gene further include a bacterial wilt pathogen. "Bacterial wilt" is a disease caused by a bacterium called Ralstonia solanacearum as a pathogen. Over 200 plants including Solanaceae crops such as eggplant and tomato, Cruciferous crops such as white radish, and strawberry are infected with and damaged by the disease. In our country, it is known that this disease has occurred in plants in 38 species in 20 families so far (Non Patent Literature 9).

The RPS4 gene and the RCH2 gene used in the present invention are not particularly limited, as long as the genes are derived from a plant which is an ecotype resistant to the pathogens of Cruciferous vegetable anthracnose and bacterial leaf spot of tomato. Preferred examples of Arabidopsis ecotypes from which the RPS4 gene and the RCH2 gene are derived include Ws-0, No-0, Nd-1, Aa-0, Eil-0, Rrs-7, Sha, Tamm-2, Tsu-1, Fei-0, Ts-1, Bsch-0, Br-0, Est-1, Rrs-10, Van-0, Nfa-8, and Bay-0 (see Figs. 7 to 11). The RPS4 gene and the RCH2 gene of Arabidopsis are respectively provided with AGI codes, At5g45250 and At5g45260. Specific sequences of these can be extracted from Non Patent Literature 10 or the site of POLYMOLPH: http://polymorph.weigelworld.org/". Note that, regarding the RPS4 gene, the base sequence of a DNA derived from the Arabidopsis ecotype Ws-0 is represented by SEQ ID NO: 1, and the amino acid sequence of a protein thereof is represented by SEQ ID NO: 2; the base sequence of a DNA derived from the Arabidopsis ecotype No-0 (Ms. Fedoroff: Non Patent Literature 11) is represented by SEQ ID NO: 5, and the amino acid sequence of a protein thereof is represented by SEQ ID NO: 6; and the base sequence of a DNA derived from the Arabidopsis ecotype Nd-1 is represented by SEQ ID NO: 9, and the amino acid sequence of a protein thereof is represented by SEQ ID NO: 10. Regarding the RCH2 gene, the base sequence of a DNA derived from the Arabidopsis ecotype Ws-0 is represented by SEQ ID NO: 3, and the amino acid sequence of a protein thereof is represented by SEQ ID NO: 4; the base sequence of a DNA derived from the Arabidopsis ecotype No-0 (Ms. Fedoroff: Non Patent Literature 11) is represented by SEQ ID NO: 7, and the amino acid sequence of a protein thereof is represented by SEQ ID NO: 8; and the base sequence of a DNA derived from the Arabidopsis ecotype Nd-1 is represented by SEQ ID NO: 11, and the amino acid sequence of a protein thereof is represented by SEQ ID NO: 12.

Particularly, when the resistance to bacterial wilt is provided to a plant, a combination of the RPS4 gene and the RCH2 gene derived from the Arabidopsis resistant ecotypes Nd-1, No-0 (Ms. Fedoroff: Non Patent Literature 11), and moderately resistant Ws-0 is particularly preferably used.

Furthermore, a DNA (for example, mutants in the RPS4 gene and the RCH2 gene, or homologs in plants other than Arabidopsis) encoding a protein structurally similar to the protein encoded by the RPS4 gene or the RCH2 gene derived from Arabidopsis can also be used in the present invention. Examples of the DNA encoding a protein structurally similar to a protein encoded by the RPS4 gene or the RCH2 gene include (i) a DNA encoding a protein having an amino acid sequence in which one or multiple amino acids are substituted, deleted, inserted, and/or added in the amino acid sequence of the protein encoded by the RPS4 gene or the RCH2 gene derived from an Arabidopsis line of an ecotype resistant to the pathogens of Cruciferous vegetable anthracnose and bacterial leaf spot of tomato, (ii) a DNA which hybridizes under stringent conditions to a DNA having the base sequence of the RPS4 gene or the RCH2 gene derived from an Arabidopsis line of an ecotype resistant to the pathogens of Cruciferous vegetable anthracnose and bacterial leaf spot of tomato, and (iii) a DNA encoding a protein having an amino acid sequence having 90% or higher homology to the amino acid sequence of the protein encoded by the RPS4 gene or the RCH2 gene derived from an Arabidopsis line of an ecotype resistant to the pathogens of Cruciferous vegetable anthracnose and bacterial leaf spot of tomato. The combination of the RPS4 gene or the DNA encoding a protein structurally similar to a protein encoded by the RPS4 gene with the RCH2 gene or the DNA encoding a protein structurally similar to a protein encoded by the RCH2 gene is not particularly limited, as long as the introduction of the combination provides a plant with resistance to at least the pathogens of Cruciferous vegetable anthracnose and bacterial leaf spot of tomato.

Herein, "multiple amino acids" subjected to the substitution or the like are generally in a range from 1 to 30 amino acids, preferably from 1 to 10 amino acids, more preferably from 1 to 5 amino acids, and further preferably from 1 to 3 amino acids. The region where such amino acids may be substituted, deleted, added and/or inserted is also not particularly limited, as long as the above resistance is maintained. The "stringent conditions" refer to a sodium concentration of 25 to 500 mM, preferably 25 to 300 mM, anda temperature of 42 to 68°C, preferably 42 to 65°C. For example, the concentration is 5×SSC (83 mM NaCl, 83 mM sodium citrate), and the temperature is 42°C. The "90% or higher homology" indicates a sequence homology of 90% or higher, preferably 95% or higher (for example, 96% or higher, 97% or higher, 98% or higher, and 99% or higher) to the entire amino acid sequence. The homology of the amino acid sequence or the base sequence can be determined by using an algorithm BLAST by Karlin and Altschul (Proc. Natl. Acad. Sci. USA, 1990, 87, 2264-2268., Karlin, S. & Altschul, SF., Proc. Natl. Acad. Sci. USA, 1993, 90, 5873). Programs called BLASTN and BLASTX based on BLAST algorithm have been developed (Altschul, SF. et al., J Mol Biol, 1990, 215, 403). When the base sequence is analyzed using BLASTN, the parameters are set at, for example, score=100 and wordlength=12. Alternatively, when the amino acid sequence is analyzed using BLASTX, the parameters are set at, for example, score=50 and wordlength=3. When BLAST and Gapped BLAST programs are used, the default parameters of each program are used. The specific procedures of these analysis methods are known (http://www.ncbi.nlm.nih.gov/).

The DNA encoding a protein structurally similar to the protein encoded by the RPS4 gene or the RCH2 gene can be prepared by methods well-known to those skilled in the art, for example, hybridization techniques (Southern, EM., J Mol Biol, 1975, 98, 503), polymerase chain reaction (PCR) techniques (Saiki, RK. et al., Science, 1985, 230, 1350., Saiki, RK. et al., Science, 1988, 239, 487), or a method in which a mutation is introduced into the DNA by site-directed mutagenesis (Kramer, W. & Fritz, HJ., Methods Enzymol, 1987, 154, 350). Moreover, in nature also, a mutation in the amino acid sequence of the protein to be encoded may occur by a mutation at the base sequence. Examples of the form of these DNAs include genomic DNA, cDNA, and chemically synthesized DNA, which can be prepared by using the conventional methods.

Whether or not the combination of the DNAs thus prepared provides a plant with the resistance to the pathogens can be assessed as follows. Specifically, a transgenic plant in which the combination of these DNAs is introduced is prepared, and it is examined whether or not the occurrence of a disease is suppressed in such a state that the transgenic plant is exposed to a disease stress.

The preparation of the transgenic plant can be carried out, for example, by inserting the combination of the DNAs of the present invention into a vector which guarantees the expression in a plant, and then causing expression of the combination of the DNAs of the present invention in the plant by utilizing the vector. A promoter used in the vector is not limited to a dedicated promoter which controls the expression of the DNAs of the present invention in a natural condition. For example, it is possible to use a CaMV35S promoter which is derived from cauliflower mosaic virus and widely used for plants. The target of the transformation may be a whole plant, a plant organ (for example, seed, leaf, petal, stem, root, or the like), a plant tissue (for example, epidermis, phloem, parenchyma, xylem, vascular bundle, or the like), or a plant cultured cell. The plant used for the transformation is, according to the object of the present invention, suitably a plant (ecotype) susceptible to pathogens of Cruciferous vegetable anthracnose and bacterial leaf spot of tomato. Examples of the plant species include Cruciferous plants such as Arabidopsis (Arabidopsis thaliana), Chinese cabbage, Japanese mustard spinach, turnip (hereinabove, Brassica rapa), cabbage (Brassica oleracea), rapeseed (Brassica napus), and white radish (Raphanus sativus), Solanaceae plants such as tomato (Solanum lycopersicum L.), eggplant (Solanum melongena), bell pepper (Capsicum annuum cv. Grossum), potato (Solanum tuberosum), and tobacco (Nicotiana tabacum), chrysanthemum (Chrysanthemum morifolium) in the family Asteraceae, banana (Musa spp. , Musa acuminata, Musa balbisiana) in the family Musaceae, strawberry (Fragaria ananassa) in the family Rosaceae, bitter melon (Momordica charantia L. var. pavel Crantz) in the family Cucurbitaceae, and the like, but not limited to these exemplified plants.

The expression vector can be introduced into the plant by generally-used transformation processes, for example, an electroporation method, an agrobacterium method, a particle gun method, a PEG method, and the like. For example, when the electroporation method is used, a sample is treated at a voltage of 500 to 1600 V under 25 to 1000 µF and 20 to 30 msec with an electroporator equipped with a pulse controller, and thus the genes are introduced into the host. Meanwhile, when the particle gun method is used, the whole plant, the plant organ or the plant tissue itself may be used directly, or a segment thereof may be prepared and then used. Alternatively, a protoplast thereof may be prepared and used. The sample thus prepared can be treated using a gene delivery system (for example, PDS-1000/He manufactured by Bio-Rad Laboratories, Inc., or the like). The treatment conditions vary depending on the plant or sample, but generally the treatment is performed at a pressure of approximately 1000 to 1800 psi and a distance of approximately 5 to 6 cm.

Moreover, using a plant virus as the vector, the DNAs of the present invention can be introduced into the plant. An example of the plant virus utilizable includes cauliflower mosaic virus. Specifically, first, the virus genome is inserted into a vector derived from Escherichia coli, or the like to prepare a recombinant. Then, these target DNAs are introduced into the virus genome. The virus genome thus modified is cut from the recombinant with a restriction enzyme and inoculated into the plant host. Thereby, the target DNAs can be introduced into the plant host.

In a method in which a Ti plasmid of Agrobacterium is utilized, the target DNA can be introduced into the plant host by utilizing the ability that, when a bacterium in the genus Agrobacterium infects a plant, part of the plasmid DNA the bacterium has is transferred into the plant genome. Among bacteria in the genus Agrobacterium, Agrobacterium tumefaciens infects a plant and forms tumors called crown galls, while Agrobacterium rhizogenes infects and causes a plant to form hairy roots. These are based on the ability: at the infection, a region called a T-DNA region (Transferred DNAs) on a plasmid, called a Ti plasmid or an Ri plasmid, present in each bacterium is transferred into a plant and incorporated into the genome of the plant. If DNAs desired to be incorporated into the plant genome are inserted in the T-DNA region on the Ti or Ri plasmid, the target DNAs can be incorporated into the plant genome when the bacterium in the genus Agrobacterium infects the plant host.

The tumor tissue, shoot, hairy root, or the like obtained as a result of the transformation can be used directly for the cell culture, tissue culture or organ culture. Furthermore, using conventionally known plant tissue culture techniques, the tumor tissue or the like can be regenerated to a whole plant by, for example, administration of a plant hormone (auxin, cytokinin, gibberellin, abscisic acid, ethylene, brassinolide, or the like) of appropriate concentration.

As the regeneration method from the transformed plant cell or the like to the transgenic plant, the following method is adopted, for example. Specifically, the transformed callus cell is transferred on media with hormones of different types and concentrations to form an adventitious embryo, and then a complete plant is obtained. Examples of the media used include a LS medium, a MS medium, and the like. The production steps of the transgenic plant in the present invention include: steps of introducing the plant expression vector in which the DNAs of the present invention are inserted into the host cell to obtain a transformed plant cell, and regenerating a transgenic plant from the transformed plant cell.

Moreover, the transgenic plant in which the DNAs of the present invention are introduced can be produced by crossing. Specifically, a plant which contains the DNAs (resistance genes) of the present invention is crossed with a plant which is crossable with the plant but does not contain the DNAs of the present invention. Thereby, the DNAs of the present invention are introduced into the plant which does not contain the DNAs of the present invention, and thus the resistance to the pathogens can be provided. Such plants are however not part of the invention.

Once the transgenic plant having the chromosomes in which the DNAs of the present invention are introduced is obtained, a progeny can be obtained from the plant through sexual reproduction or asexual reproduction. In addition, seeds or the like are obtained from the plant, the progeny, or a clone thereof, and the plant can be produced in mass therefrom.

Moreover, a portion of the transgenic plant of the present invention can be used as, for example, foods and the like as agricultural crops. The present invention includes "a portion or a propagation material" of the transgenic plant of the present invention, such as a plant organ (for example, seed, leaf, petal, stem, root, or the like), a plant tissue (for example, epidermis, phloem, parenchyma, xylem, vascular bundle, or the like), or a plant cultured cell.

To obtain plant seeds from the transgenic plant, for example, the transgenic plant is taken out from the rooting medium, transplanted to a pot with a soil containing water, grown at a certain temperature, and caused to flower, and finally seeds are formed. Meanwhile, to produce the plant from the seed, for example, when the seeds formed on the transgenic plant mature, the seeds are isolated, seeded into a soil containing water, and grown at certain temperature and illumination to produce the plant. The plant thus nurtured is a diseases-stress tolerant plant that has acquired the resistance to multiple pathogens by the expression of the combination of the DNAs thus introduced.

### [Examples]

Hereinbelow, the present invention will be described in more details using Examples. However, the technical scope of the present invention is not limited to Examples below.

### [Example 1] Search for Resistance Genes by SSLP (Simple Sequence Length Polymorphism) Analysis

The Arabidopsis ecotype Wassilewskija (Ws-0) resistant to a pathogen of Cruciferous vegetable anthracnose (Colletotrichum higginsianum) was crossed with susceptible Columbia (Col-0), and F1 seeds were obtained. Furthermore, the F1 individuals were self-pollinated, and F2 seeds were obtained. The Arabidopsis F1 plants were grown (at 22°C and cycles of a 12-hour light period and 12-hour dark period) for four weeks after seeded. Then, 5×10⁵/ml of an anthracnose pathogen spore suspension was sprayed on and inoculated into the plants. After six days, an assay was conducted, and all showed the resistance. Next, the F2 plants were grown similarly, and 5×10⁵/ml of an anthracnose pathogen spore suspension was sprayed on and inoculated into the plants. As a result, it was found out that the resistant individuals and the susceptible individuals were segregated in 3:1, that the resistance was dominant, and that this resistance was controlled at one locus or multiple loci adjacent to each other. Hence, it was expected that, when an individual having a susceptible phenotype to the pathogen was selected from the F2 group, the individual would have a homozygous Col-0 gene (alleles) at the corresponding target locus of Ws-0. Thus, it was also expected that, when the chromosome structures of a number of F2 individuals selected based on phenotype were examined by polymorphic markers, the alleles of the target genes existed in the vicinity of a region where the Col type was shown most frequently with the homozygous genotype. Based on the expectations, an SSLP analysis was conducted to identify the target genes. As a result, a region where the target genes existed was successfully narrowed down within a region between the polymorphic markers N01-K11I1 and N01-K17022 (Fig. 1).

### [Example 2] Analysis of Relationship between Resistance to Anthracnose Pathogen and Mutations in RPS4 Gene (At5g45250) and RCH2 Gene (At5g45260)

By the method of Example 1, it was no longer possible to generate polymorphic markers utilizable in narrowing down the region where the target genes existed, and the SSLP analysis came to the limit. For this reason, approximately 30,000 lines were screened using a T-DNA insertion library of the resistant ecotype Ws-0 . As a result, obtained were eight individuals which were T-DNA insertedmutants susceptible to anthracnose. The base sequences of Ws-At5g45250 and Ws-At5g45260 of these mutants were analyzed, and mutants in which mutations were introduced into these genes were discovered. A mutant ΔAt5g45250-21 having five bases deleted from At5g45250 as well as mutants ΔAt5g45260-1 and ΔAt5g45260-2 having T-DNA tags inserted in At5g45260 showed susceptibility to the anthracnose pathogen (Fig. 2, "Ws-0 mutant").

The present inventors had elucidated that Ws-At5g45250 was a resistance gene (Japanese Patent Application No. 2007-86343). It was also assumed that At5g45260 was possibly a resistance gene. For this reason, the At5g45260 amino acid sequences were compared between the ecotype Ws-0 and the ecotype Col-0 which were respectively resistant and susceptible to the anthracnose pathogen (Fig. 3). In Col-At5g45260, approximately 80 to 90 amino acids were deleted from the WRKY domain toward the C terminal. Similarly to Col-At5g45260, the ecotypes Cvi-0 and Bur-0 in which deletions toward the C terminal were recognized also showed susceptibility to the anthracnose pathogen. From this, it was found out that At5g45260 was also involved in the resistance to the anthracnose pathogen, and that the presence of at least approximately 80 to 90 amino acids from the WRKY domain of At5g45260 toward the C terminal was important to acquire the resistance.

Furthermore, in the ecotypes (Ler-1, Lov-5, C24) susceptible to the anthracnose pathogen, the 775th tyrosine was substituted with asparagine. It was indicated that this amino acid was important in the resistance (Figs. 10 and 11).

### [Example 3] Analysis of Relationship between Resistance to Bacterial Leaf Spot having avrRps4 Gene and Mutations in At5g45250 and At5g45260

The resistance to a plant pathogen was examined using a bacterium of tomato leaf spot (Pseudomonas syringae pv. tomato) having the avrRps4 gene and known to infect Arabidopsis also. The bacterium was shake-cultured overnight in a King's B liquid medium to which kanamycin (25 µg/ml) and rifampicin (25 µg/ml) had been added. The bacterial suspension was prepared to 1×10⁵ (cfu)/ml. This bacterial suspension was injected into a rosette leaf of Arabidopsis grown (at 22°C and cycles of an 8-hour light period and a 16-hour dark period) for seven weeks after seeded, in such a way that a 1-ml needless syringe was pressed on the back side of the leaf, and a pressure was applied thereon. Thereby, the pathogen was inoculated. On the third day after the inoculation, the inoculated leaf was cut out with a cork borer and fragmented in a 10-mM MgSO₄ liquid. The bacterial suspension was seeded on a solid medium. Two days later, the number of colonies appeared was counted. Thereby, the growth of the bacterium per leaf area was determined. Figs. 4A and 4B show the results of the susceptibility (growth of the pathogenic bacterium) of plants (wild strains, overexpressors, gene disruptants) inoculated with the bacterium of tomato leaf spot.

### (1) Relationship between At5g45260 and Susceptibility to Bacterial Leaf Spot (Fig. 4A)

In the mutants having At5g45260 disrupted, the growth of the bacterium was high relative to that in the Ws-0 wildtype, and the susceptibility to bacterial leaf spot was increased.

### (2) Relationship between At5g45250 and Susceptibility to Bacterial Leaf Spot (Fig. 4B)

In the mutants having At5g45250 disrupted, the growth of the bacteria was also high relative to that in the Ws-0 wildtype, and the susceptibility to bacterial leaf spot was increased. Additionally, revertants which were each obtained by transforming the At5g45250-disrupted mutant with approximately 6 kb of the Ws-At5g45250 gene including approximately 2 kb of a promoter region showed similar resistance to that of the Ws-0 wildtype. Moreover, transformants which were each obtained by introducing approximately 6 kb of the Ws-At5g45250 gene including approximately 2 kb of a promoter region into an ecotype RLD susceptible to the anthracnose pathogen and bacterial leaf spot showed strong resistance to bacterial leaf spot.

From the above, it was found out that both At5g45250 and At5g45260 were essential for expression of resistance to bacterial leaf spot. So far, it had been reported that only At5g45250 was involved in the expression of the resistance to bacterial leaf spot (Non Patent Literature 2). However, from this result, it was shown that At5g45260 was also involved therein. Particularly, RLD-0, which was an only susceptible ecotype among the reported Arabidopsis ecotypes, acquired the resistance to the bacterial leaf spot and anthracnose pathogens by introducing Ws-At5g45250 into RLD-0. Therefore, it was clarified that RLD-0 originally had At5g45260 for resistance to these pathogens, and that the cause of the susceptibility was At5g45250.

### [Example 4] Analysis of Relationship between Mutations in At5g45250, At5g45260 of Arabidopsis Ecotypes No-0, Ws-0 and Resistance to Anthracnose Pathogen, Bacterial Wilt Pathogen

First, No-0 from Ms. Fedoroff, which was reported to be resistant to a bacterial wilt pathogen (Non Patent Literature 12), was examined on the resistance to the anthracnose pathogen and bacterial leaf spot. As a result, this ecotype showed the resistance to both of the pathogens. Then, using transposon mutants of No-0, the relationship between mutations inAt5g45250, At5g45260 and the resistance to these pathogens was examined (Fig. 2 "transposon mutants of No-0 from Ms. Fedoroff"). As a result, a mutant ΔAt5g45250-31 (rps4-31) having a transposon tag inserted into At5g45250 showed susceptibility to the anthracnose pathogen. Additionally, a mutant ΔAt5g45250-60-1 having transposon tags inserted into a promoter region of At5g45250 and into At5g45260 also showed susceptibility to the anthracnose pathogen. In the Arabidopsis ecotype No-0 (from Ms. Fedoroff) also, At5g45250 and At5g45260 were involved in the resistance to the anthracnose pathogen.

A bacterial wilt pathogen suspension was prepared to 1×10⁸ (cfu)/ml. Arabidopsis was grown (at 22°C and cycles of an 8-hour light period and a 16-hour dark period) on rock wool for seven weeks after seeded. The root was cut off together with the rock wool, and 20 ml or more of the bacterial suspension was poured onto the root portion thus cut. The disease onset was assayed on the seventh day after the inoculation. Figs. 5A to 5G show the results of the susceptibility of plants (wild strains, gene disruptants) inoculated with the bacterial wilt pathogen.

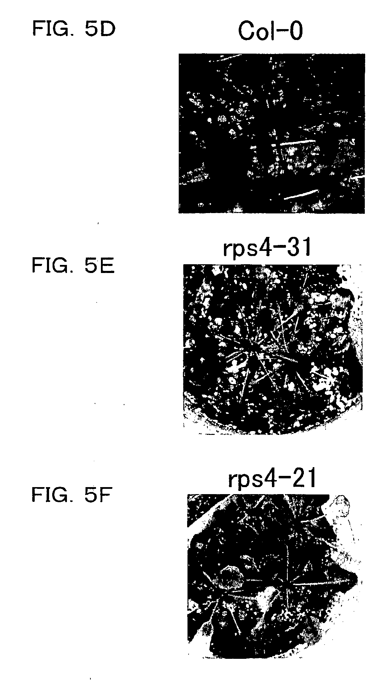

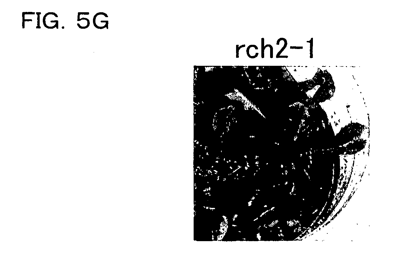

The ecotypes Nd-1, No-0, and Ws-0 were resistant to bacterial wilt (Figs. 5A to 5C), whereas Col-0 was susceptible (Fig. 5D). It was reported that ΔAt5g45250-60-1 was susceptible to the bacterial wilt pathogen (Non Patent Literature 12). The present invention proved that the mutant ΔAt5g45250-31 (rps4-31) of No-0 from Ms. Fedoroff having the tag inserted into At5g45250 was susceptible to the bacterial wilt pathogen (Fig. 5E). A mutant ΔAt5g45250-21 (rps4-21) of the ecotype Ws-0 having five bases deleted in At5g45250 and a mutant ΔAt5g45260-1 (rch2-1) of Ws-0 having a T-DNA tag inserted in At5g45260 showed strong susceptibility to the bacterial wilt pathogen (Figs. 5F and 5G) . The above results suggest that, if a plant susceptible to these pathogens is transformed with At5g45250 and At5g45260 of Ws-0 and No-0 from Ms. Fedoroff, a plant resistant to the anthracnose pathogen, the bacterial leaf spot and the bacterial wilt pathogen can be produced.

### [Example 5] Introduction of Resistance Genes into Arabidopsis Ecotypes Col-0, RLD-0 Susceptible to Anthracnose Pathogen, and Effects of Introduction

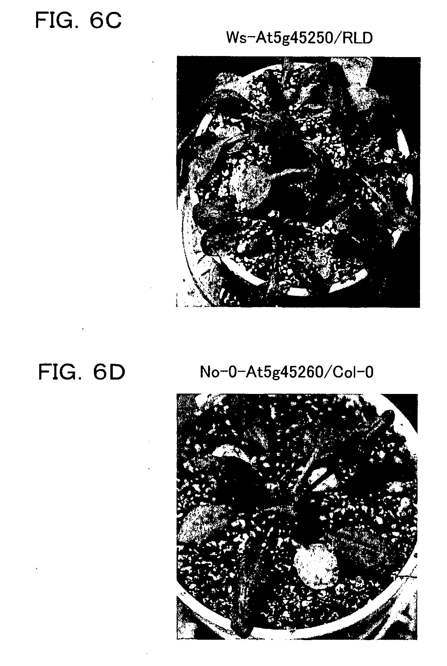

Into the Arabidopsis ecotypes Col-0 and RLD-0 susceptible to the anthracnose pathogen, At5g45250 or At5g45260 of the ecotype Ws-0 resistant to the anthracnose pathogen was introduced, and it was examined whether or not the ecotypes acquired the resistance to the anthracnose pathogen (Figs. 6A to 6D). As a result, transformants obtained by introducing the resistance gene Ws-At5g45250 into the wildtype Col-0 showed the resistance to this fungal pathogen, but the plants were dwarfed (Fig. 6B). Meanwhile, transformants obtained by introducing the resistance gene Ws-At5g45250 into the wildtype RLD normally grew, and showed the resistance to this fungal pathogen (Fig. 6C). Transformants obtained by introducing the resistance gene No-0-At5g45260 from Ms. Fedoroff into the wildtype Col-0 normally grew, and showed the resistance to fungal pathogen (Fig. 6D). From the above, it was found out that, even if anthracnose pathogen-resistant Ws-At5g45250 was introduced into the wildtype Col-0, the resultant plants were not able to be stably maintained, but that the transformants obtained by introducing anthracnose pathogen-resistant No-O-At5g45260 from Ms. Fedoroff were stably maintained and were resistant to the anthracnose pathogen. RLD-0 originally has resistant At5g45260, and, when Resistant Ws-At5g45250 was introduced thereinto, RLD-0 was stably maintained in the plant, which showed the resistance to the anthracnose pathogen. By introducing two different resistance genes in this manner, the plants showing resistance to the anthracnose pathogen were successfully produced.

Note that Col-0 having No-0-At5g45260 from Ms. Fedoroff introduced thereinto has At5g45250 for resistance to bacterial leaf spot and At5g45260 for resistance to bacterial wilt. Accordingly, it would be easily understood by those skilled in the art that such Col-0 shows resistance to both bacterial leaf spot and bacterial wilt.

### [Example 6] Relationship between Susceptibility of Various Arabidopsis Ecotypes to Anthracnose and Amino Acid Sequences of At5g45250, At5g45260

The Arabidopsis ecotypes were examined on the susceptibility to anthracnose (Figs. 7 and 8). A phylogenetic tree analysis was conducted on the amino acid sequences of At5g45250 and At5g45260 by utilizing SNP analysis data on these ecotypes of Arabidopsis (Non Patent Literature 10) (Figs. 7 and 8). As to At5g45250, the ecotypes which were susceptible and resistance to anthracnose were present randomly when classified. The At5g45250 amino acid sequences of the ecotypes were compared with one another. The 950th tyrosine was substituted with histidine in RLD-0 susceptible to all of Cruciferous vegetable anthracnose, bacterial leaf spot, and bacterial wilt (Fig. 9). Hence, a construct was created in which the 950th tyrosine of Ws-At5g45250 in Ws-0 resistance to anthracnose was substituted with histidine. The construct was introduced into a disruptant ΔAt5g45250-21 (rps4-21) of Ws-At5g45250 using Ws-0 as a background. This transformant showed susceptibility to anthracnose, and was not able to recover the resistance. Thus, it was indicated that this amino acid was important in the resistance. Meanwhile, as a result of the phylogenetic tree analysis on the At5g45260 amino acid sequence, as to At5g45260, susceptible ecotypes were classified into one group (Fig. 7). In this group (Ler-1, Lov-5, C24), the 775th tyrosine was substituted with asparagine as described in Example 2. It was indicated that this amino acid was important in the resistance (Figs. 10 and 11).

### [Example 7] Introductionof At5g45250 andAt5g45260 derived from Arabidopsis Ecotype Ws-0 into Ecotype Col-0 Susceptible to Anthracnose Pathogen, and Effects of Introduction

The results in Examples 2 to 6 indicated that the two genes At5g45250 and At5g45260 were essential for resistance of plant to the anthracnose pathogen, and that, by introducing these two genes into an ecotype plant susceptible to the anthracnose pathogen, the plant was successfully transformed to an ecotype resistant to the anthracnose pathogen. To reconfirm this fact, the present inventors introduced At5g45250 and At5g45260 of the Arabidopsis ecotype Ws-0 resistant to the anthracnose pathogen into the ecotype Col-0 susceptible to the anthracnose pathogen, and it was examined whether or not the ecotype acquired the resistance to the anthracnose pathogen.

As a result, as already proved in Example 5 (Fig. 6B), transformants obtained by introducing the resistance gene Ws-At5g45250 into the wildtype Col-0 showed the resistance to this fugal pathogen, but the plants were dwarfed (Fig. 12, left) . Transformants obtained by introducing Ws-At5g45260 derived from the ecotype Ws-0 resistant to the anthracnose pathogen into the dwarfed transformants were stably maintained in the plants, normally grew without being dwarfed (Fig. 12, right), and acquired the resistance to the anthracnose pathogen (Fig. 13). From the above, it was reconfirmed that, by introducing two different resistance genes, a plant showing the resistance to the anthracnose pathogen was successfully produced. Meanwhile, instances are reported that, when a disease resistance gene alone is introduced, the plant is dwarfed generally. Nevertheless, it was also reconfirmed that such a problem of a dwarfed plant in producing resistant plants were successfully avoided by introducing multiple genes in combination.

Note that Col-0 having Ws-At5g45250 and Ws-At5g45260 derived from Ws-0 introduced at the same time has Ws-At5g45250 for resistance to bacterial leaf spot and Ws-At5g45260 for resistance to bacterial wilt. Accordingly, it would be easily understood by those skilled in the art that such Col-0 shows resistance to both bacterial leaf spot and bacterial wilt.

### [Example 8] Introductionof At5g45250 and At5g45260 Derived from Arabidopsis Ecotype Ws-0 into Japanese Mustard Spinach Cultivar "Osome" Susceptible to anthracnose pathogen, and Effects of Introduction

The results in Examples 2 to 6 indicated that the two genes At5g45250 and At5g45260 were essential for resistance of plant to the anthracnose pathogen, and that, by introducing these two genes into an ecotype plant susceptible to the anthracnose pathogen, the plant was successfully transformed to an ecotype resistant to the anthracnose pathogen. To reconfirm this fact, the present inventors introduced At5g45250 and At5g45260 of the Arabidopsis ecotype Ws-0 resistant to the anthracnose pathogen into the Japanese mustard spinach cultivar "Osome" susceptible to the anthracnose pathogen, and it was examined whether or not the cultivar acquired the resistance to the anthracnose pathogen.

As a result, most of transformants obtained by introducing Ws-At5g45250 into the wildtype Japanese mustard spinach were dwarfed, and the individuals were not able to be maintained. Individual plants that somehow survived were dwarfed. Moreover, transformants obtained by introducing the resistance gene Ws-At5g45260 into the wildtype Japanese mustard spinach were not able to stably acquire the resistance to the anthracnose pathogen (Fig. 14, right). Meanwhile, transformants obtained by introducing At5g45250 and At5g45260 of the Arabidopsis ecotype Ws-0 into the wildtype Japanese mustard spinach at the same time were stably maintained in the plants, and the plants also normally grew and were resistant to the anthracnose pathogen (Fig. 14, left). From the above, it was reconfirmed that, by introducing two different resistance genes, a plant showing the resistance to the anthracnose pathogen was successfully produced. Meanwhile, instances are reported that, when a disease resistance gene alone is introduced, the plant is dwarfed generally. Nevertheless, it was also reconfirmed that such a problem of a dwarfed plant in producing resistant plants were successfully avoided by introducing multiple genes in combination.

Note that the Japanese mustard spinach having Ws-At5g45250 and Ws-At5g45260 derived from Ws-0 introduced at the same time has Ws-At5g45250 for resistance to bacterial leaf spot and Ws-At5g45260 for resistance to bacterial wilt. Accordingly, it would be easily understood by those skilled in the art that such Japanese mustard spinach shows resistance to both bacterial leaf spot and bacterial wilt.

### [Industrial Applicability]

The present invention is suitably utilizable in the fields of breeding, for example, Cruciferous crops such as Chinese cabbage, Japanese mustard spinach, turnip, cabbage, rapeseed, and white radish and Solanaceae plants such as tomato, eggplant, bell pepper, and potato. The use of the present invention makes it possible to protect these crops from diseases due to pathogens of Cruciferous vegetable anthracnose, bacterial leaf spot, and bacterial wilt, and to improve the productivity of these crops.

## Claims

1. A method for providing a transgenic plant with resistance to pathogens of anthracnose and bacterial leaf spot, the method **characterized by** comprising:
introducing a combination of
A) an RPS4 gene with the base sequence of SEQ ID NO:1, SEQ ID NO:5, or SEQ ID NO:9; or
i) a DNA encoding a protein having an amino acid sequence in which 1 to 30 amino acids are substituted, deleted, inserted and/or added in the amino acid sequence of SEQ ID NO:2, SEQ ID NO:6, or SEQ ID NO:10; or
ii) a DNA which hybridizes under stringent conditions to a DNA of SEQ ID NO:1, SEQ ID NO:5, or SEQ ID NO:9; or
iii) a DNA encoding a protein having an amino acid sequence having 90% or higher homology to the amino acid sequence of SEQ ID NO:2, SEQ ID NO:6, or SEQ ID NO:10;
and
B) an RCH2 gene with the base sequence of SEQ ID NO:3, SEQ ID NO:7, or SEQ ID NO:11; or
i) a DNA encoding a protein having an amino acid sequence in which 1 to 30 amino acids are substituted, deleted, inserted and/or added in the amino acid sequence of SEQ ID NO:4, SEQ ID NO:8, or SEQ ID NO:12; or
ii) a DNA which hybridizes under stringent conditions to a DNA of SEQ ID NO:3, SEQ ID NO:7, or SEQ ID NO:11; or
iii) a DNA encoding a protein having an amino acid sequence having 90% or higher homology to the amino acid sequence of SEQ ID NO:4, SEQ ID NO:8, or SEQ ID NO:12;
into the plant,
wherein A) and B) do not provide the plant with the resistance to the pathogens when each of A) and B) is introduced alone, but provide the plant with the resistance to the pathogens when introduced in the combination;
provided that the method for providing the transgenic plant does not include crossing.

2. The method according to claim 1, wherein the transgenic plant further has resistance to a bacterial wilt pathogen.

3. The method according to claims 1 or 2, wherein the combination of A) and B) is introduced into the plant by
i) transformation of an expression vector into which the combination of the DNAs are inserted;
ii) using a plant virus as vector; or
iii)using a Ti plasmid of Agrobacterium.

4. A transgenic plant provided with resistance to pathogens of anthracnose and bacterial leaf spot, comprising:
a combination of
A) an RPS4 gene with the base sequence of SEQ ID NO:1, SEQ ID NO:5, or SEQ ID NO:9; or
i) a DNA encoding a protein having an amino acid sequence in which 1 to 30 amino acids are substituted, deleted, inserted and/or added in the amino acid sequence of SEQ ID NO:2, SEQ ID NO:6, or SEQ ID NO:10; or
ii) a DNA which hybridizes under stringent conditions to a DNA of SEQ ID NO:1, SEQ ID NO:5, or SEQ ID NO:9; or
iii) a DNA encoding a protein having an amino acid sequence having 90% or higher homology to the amino acid sequence of SEQ ID NO:2, SEQ ID NO:6, or SEQ ID NO:10;
and
B) an RCH2 gene with the base sequence of SEQ ID NO:3, SEQ ID NO:7, or SEQ ID NO:11; or
i) a DNA encoding a protein having an amino acid sequence in which 1 to 30 amino acids are substituted, deleted, inserted and/or added in the amino acid sequence of SEQ ID NO:4, SEQ ID NO:8, or SEQ ID NO:12; or
ii) a DNA which hybridizes under stringent conditions to a DNA of SEQ ID NO:3, SEQ ID NO:7, or SEQ ID NO:11; or
iii) a DNA encoding a protein having an amino acid sequence having 90% or higher homology to the amino acid sequence of SEQ ID NO:4, SEQ ID NO:8, or SEQ ID NO:12;
introduced therein, wherein
A) and B) do not provide the plant with the resistance to the pathogens when each of A) and B)is introduced alone, but provide the plant with the resistance to the pathogens when introduced in the combination.

5. The transgenic plant according to claim 4, wherein the transgenic plant further has resistance to a bacterial wilt pathogen.

6. A portion or a propagation material of the transgenic plant according to any one of claims 4 or 5, wherein the portion or respectively the propagation material of the transgenic plant comprises the combination of A) and B) as described in claim 4.

7. The portion or propagation material according to claim 6, wherein the portion or propagation material is a plant organ, a plant tissue or a plant cultured cell.

8. The portion or propagation material according to claim 7, wherein the plant organ is selected from the group consisting of seed, leaf, petal, stem and root; the plant tissue is selected from the group consisting of epidermis, phloem, parenchyma, xylem and vascular bundle.

## Patentansprüche

1. Verfahren zur Herstellung einer transgenen Pflanze mit Widerstandsfähigkeit gegenüber Pathogenen der Anthraknose und Blattfleckenkrankheit, **dadurch gekennzeichnet, dass** es das Einschleusen einer Kombination aus:
A) einem RPS4-Gen mit der Basensequenz SEQ ID NR: 1, SEQ ID NR: 5 bzw. SEQ ID NR: 9 oder
I) einer DNA, die ein Protein mit einer Aminosäuresequenz, in welcher 1 bis 30 Aminosäuren der Aminosäuresequenz SEQ ID NR: 2, SEQ ID NR: 6 oder SEQ ID NR: 10 substituiert, deletiert, insertiert und/oder addiert sind, codiert,
II) einer DNA, die unter stringenten Bedingungen mit einer DNA mit der SEQ ID NR: 1, SEQ ID NR: 5 oder SEQ ID NR: 9 hybridisiert, oder
III) einer DNA, die ein Protein mit einer Aminosäuresequenz mit einer Homologie von 90 % oder höher mit der Aminosäuresequenz SEQ ID NR: 2, SEQ ID NR: 6 oder SEQ ID NR: 10 codiert, und
B) einem RCH2-Gen mit der Basensequenz SEQ ID NR: 3, SEQ ID NR: 7 bzw. SEQ ID NR: 11 oder
I) einer DNA, die ein Protein mit einer Aminosäuresequenz, in welcher 1 bis 30 Aminosäuren der Aminosäuresequenz SEQ ID NR: 4, SEQ ID NR: 8 oder SEQ ID NR: 12 substituiert, deletiert, insertiert und/oder addiert sind, codiert,
II) einer DNA, die unter stringenten Bedingungen mit einer DNA mit der SEQ ID NR: 3, SEQ ID NR: 7 oder SEQ ID NR: 11 hybridisiert, oder
III) einer DNA, die ein Protein mit einer Aminosäuresequenz mit einer Homologie von 90 % oder höher mit der Aminosäuresequenz SEQ ID NR: 4, SEQ ID NR: 8 oder SEQ ID NR: 12 codiert,
in die Pflanze umfasst, wobei durch A) und B) die Pflanze mit Widerstandsfähigkeit gegenüber den Pathogenen nicht hergestellt wird, wenn jede/s von A) und B) allein eingeschleust wird, aber die Pflanze mit Widerstandsfähigkeit gegenüber den Pathogenen hergestellt wird, wenn sie in Kombination eingeschleust werden, unter der Voraussetzung, dass das Verfahren zur Herstellung der transgenen Pflanze das Kreuzen nicht einschließt.

2. Verfahren nach Anspruch 1, wobei die transgene Pflanze weiterhin Widerstandsfähigkeit gegenüber einem bakteriellen Welke-Pathogen besitzt.

3. Verfahren nach Anspruch 1 oder 2, wobei die Kombination aus A) und B) in die Pflanze eingeschleust wird durch:
I) Transformieren eines Expressionsvektors, in welchen die Kombination aus den DNAs insertiert worden ist,
II) Verwenden eines Pflanzenvirus als Vektor oder
III) Verwenden eines Agrobacterium-Ti-Plasmids.

4. Transgene Pflanze, der Widerstandsfähigkeit gegenüber Pathogenen der Anthraknose und Blattfleckenkrankheit verliehen worden ist und welche eine Kombination aus:
A) einem RPS4-Gen mit der Basensequenz SEQ ID NR: 1, SEQ ID NR: 5 bzw. SEQ ID NR: 9 oder
I) einer DNA, die ein Protein mit einer Aminosäuresequenz, in welcher 1 bis 30 Aminosäuren der Aminosäuresequenz SEQ ID NR: 2, SEQ ID NR: 6 oder SEQ ID NR: 10 substituiert, deletiert, insertiert und/oder addiert sind, codiert,
II) einer DNA, die unter stringenten Bedingungen mit einer DNA mit der SEQ ID NR: 1, SEQ ID NR: 5 oder SEQ ID NR: 9 hybridisiert, oder
III) einer DNA, die ein Protein mit einer Aminosäuresequenz mit einer Homologie von 90 % oder höher mit der Aminosäuresequenz SEQ ID NR: 2, SEQ ID NR: 6 oder SEQ ID NR: 10 codiert, und
B) einem RCH2-Gen mit der Basensequenz SEQ ID NR: 3, SEQ ID NR: 7 bzw. SEQ ID NR: 11 oder
I) einer DNA, die ein Protein mit einer Aminosäuresequenz, in welcher 1 bis 30 Aminosäuren der Aminosäuresequenz SEQ ID NR: 4, SEQ ID NR: 8 oder SEQ ID NR: 12 substituiert, deletiert, insertiert und/oder addiert sind, codiert,
II) einer DNA, die unter stringenten Bedingungen mit einer DNA mit der SEQ ID NR: 3, SEQ ID NR: 7 oder SEQ ID NR: 11 hybridisiert, oder
III) einer DNA, die ein Protein mit einer Aminosäuresequenz mit einer Homologie von 90 % oder höher mit der Aminosäuresequenz SEQ ID NR: 4, SEQ ID NR: 8 oder SEQ ID NR: 12 codiert,
die in sie eingeschleust worden sind, umfasst, wobei durch A) und B) die Pflanze mit Widerstandsfähigkeit gegenüber den Pathogenen nicht hergestellt wird, wenn jede/s von A) und B) allein eingeschleust wird, aber die Pflanze mit Widerstandsfähigkeit gegenüber den Pathogenen hergestellt wird, wenn sie in Kombination eingeschleust werden.

5. Transgene Pflanze nach Anspruch 4, wobei die transgene Pflanze ferner Widerstandsfähigkeit gegenüber einem bakteriellen Welke-Pathogen besitzt.

6. Teil oder vermehrungsfähiges Material der transgenen Pflanze nach Anspruch 4 oder 5, wobei der Teil oder das vermehrungsfähige Material der transgenen Pflanze die in Anspruch 4 beschriebene Kombination aus A) und B) umfasst.

7. Teil oder vermehrungsfähiges Material nach Anspruch 6, wobei der Teil oder das vermehrungsfähige Material ein pflanzliches Organ, ein Pflanzengewebe oder eine kultivierte Pflanzenzelle ist.

8. Teil oder vermehrungsfähiges Material nach Anspruch 7, wobei das pflanzliche Organ aus der Gruppe ausgewählt ist, die aus Samen, Blättern, Blütenblättern, Stängeln und Wurzeln besteht, und das Pflanzengewebe aus der Gruppe ausgewählt ist, die aus Epidermis, Phloöm, Parenchym, Xylem und Leitbündel besteht.

## Revendications

1. Procédé de préparation d'une plante transgénique présentant une résistance aux pathogènes de l'anthracnose et la moucheture bactérienne, le procédé étant **caractérisé en ce qu'**il comprend :
l'introduction d'une combinaison de :
A) un gène RPS4 avec la séquence de bases de SEQ ID N°1, SEQ ID N°5 ou SEQ ID N°9, ou
i) un ADN codant une protéine ayant une séquence des acides aminés, dans laquelle 1 à 30 acides aminés sont substitués, délétés, insérés et/ou ajoutés dans la séquence des acides aminés de SEQ ID N°2, SEQ ID N°6 ou SEQ ID N°10, ou
ii) un ADN qui s'hybride dans des conditions rigoureuses à un ADN de SEQ ID N°1, SEQ ID N°5 ou SEQ ID N°9, ou
iii) un ADN codant une protéine ayant une séquence des acides aminés ayant 90% d'homologie ou plus avec la séquence des acides aminés de SEQ ID N°2, SEQ ID N°6 ou SEQ ID N°10 ; et
B) un gène RCH2 avec la séquence de bases de SEQ ID N°3, SEQ ID N°7 ou SEQ ID N°11, ou
i) un ADN codant une protéine ayant une séquence des acides aminés, dans laquelle 1 à 30 acides aminés sont substitués, délétés, insérés et/ou ajoutés dans la séquence des acides aminés de SEQ ID N°4, SEQ ID N°8 ou SEQ ID N°12, ou
ii) un ADN qui s'hybride dans des conditions rigoureuses à un ADN de SEQ ID N°3, SEQ ID N°7 ou SEQ ID N°11, ou
iii) un ADN codant une protéine ayant une séquence des acides aminés ayant 90% d'homologie ou plus avec la séquence des acides aminés de SEQ ID N°4, SEQ ID N°8 ou SEQ ID N°12 ;
dans la plante,
où A) et B) ne procurent pas la résistance aux pathogènes à la plante lorsque chacun de A) et B) est introduit seul, mais ils procurent la résistance aux pathogènes à la plante lorsqu'ils sont introduits en combinaison ;
pourvu que le procédé de préparation de la plante transgénique ne comprenne pas de croisement.

2. Procédé selon la revendication 1, où la plante transgénique est également résistante à un pathogène du flétrissement bactérien.

3. Procédé selon la revendication 1 ou 2, où la combinaison de A) et de B) est introduite dans la plante par
i) transformation d'un vecteur d'expression dans lequel la combinaison des ADN est insérée ;
ii) l'utilisation d'un virus végétal comme vecteur, ou
iii) l'utilisation d'un plasmide Ti de *Agrobacterium.*

4. Plante transgénique présentant une résistance aux pathogènes de l'anthracnose et la moucheture bactérienne, comprenant :
une combinaison de :
A) un gène RPS4 avec la séquence de bases de SEQ ID N°1, SEQ ID N°5 ou SEQ ID N°9, ou
i) un ADN codant une protéine ayant une séquence des acides aminés, dans laquelle 1 à 30 acides aminés sont substitués, délétés, insérés et/ou ajoutés dans la séquence des acides aminés de SEQ ID N°2, SEQ ID N°6 ou SEQ ID N°10, ou
ii) un ADN qui s'hybride dans des conditions rigoureuses à un ADN de SEQ ID N°1, SEQ ID N°5 ou SEQ ID N°9, ou
iii) un ADN codant une protéine ayant une séquence des acides aminés ayant 90% d'homologie ou plus avec la séquence des acides aminés de SEQ ID N°2, SEQ ID N°6 ou SEQ ID N°10 ; et
B) un gène RCH2 avec la séquence de bases de SEQ ID N°3, SEQ ID N°7 ou SEQ ID N°11, ou
i) un ADN codant une protéine ayant une séquence des acides aminés, dans laquelle 1 à 30 acides aminés sont substitués, délétés, insérés et/ou ajoutés dans la séquence des acides aminés de SEQ ID N°4, SEQ ID N°8 ou SEQ ID N°12, ou
ii) un ADN qui s'hybride dans des conditions rigoureuses à un ADN de SEQ ID N°3, SEQ ID N°7 ou SEQ ID N°11, ou
iii) un ADN codant une protéine ayant une séquence des acides aminés ayant 90% d'homologie ou plus avec la séquence des acides aminés de SEQ ID N°4, SEQ ID N°8 ou SEQ ID N°12 ;
introduite dans la plante,
où A) et B) ne procurent pas la résistance aux pathogènes à la plante lorsque l'un de A) et B) est introduit seul, mais ils procurent la résistance aux pathogènes à la plante lorsqu'ils sont introduits en combinaison.

5. Plante transgénique selon la revendication 4, où la plante transgénique est également résistante à un pathogène du flétrissement bactérien.

6. Partie ou matériel de propagation de la plante transgénique selon l'une quelconque des revendications 4 ou 5, où la partie ou respectivement, le matériel de propagation de la plante transgénique comprend la combinaison de A) et de B) comme décrit à la revendication 4.

7. Partie ou matériel de propagation selon la revendication 6, où la partie ou le matériel de propagation est un organe de la plante, un tissu végétal ou une cellule végétale en culture.

8. Partie ou matériel de propagation selon la revendication 7, où l'organe de la plante est choisi parmi le groupe consistant en une semence, une feuille, un pétale, la tige et la racine ; le tissu végétal est choisi parmi le groupe consistant en l'épiderme, le ploème, le parenchyme, le xylème et le faisceau vasculaire.
